(19) Europäisches Patentamt
European Patent Office
Office européen des brevets

(11) EP 3 640 350 A1

(12) **EUROPEAN PATENT APPLICATION**

(43) Date of publication:
**22.04.2020 Bulletin 2020/17**

(51) Int Cl.:
***C12Q 1/6886*** *(2018.01)*

(21) Application number: **19162642.3**

(22) Date of filing: **13.03.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.10.2018 GB 201816820**

(71) Applicant: **The University of York**
**Heslington**
**York YO10 5DD (GB)**

(72) Inventors:
- **Pellacani, Davide**
  **Vancouver, V5Z 1L3 (CA)**
- **Maitland, Norman**
  **York, YO10 5DD (GB)**

(74) Representative: **Docherty, Robert Charles**
**Symbiosis IP Limited**
**NAFIC**
**Office 14FA05**
**Sand Hutton**
**York, North Yorkshire YO41 1LZ (GB)**

(54) **DIAGNOSTIC METHOD**

(57) The disclosure relates to a method for diagnosis of prostate cancer (PCa) by determining methylation of selected genomic sites within the human genome; probes useful in determining methylation and kits comprising the probes and components useful in conducting the method.

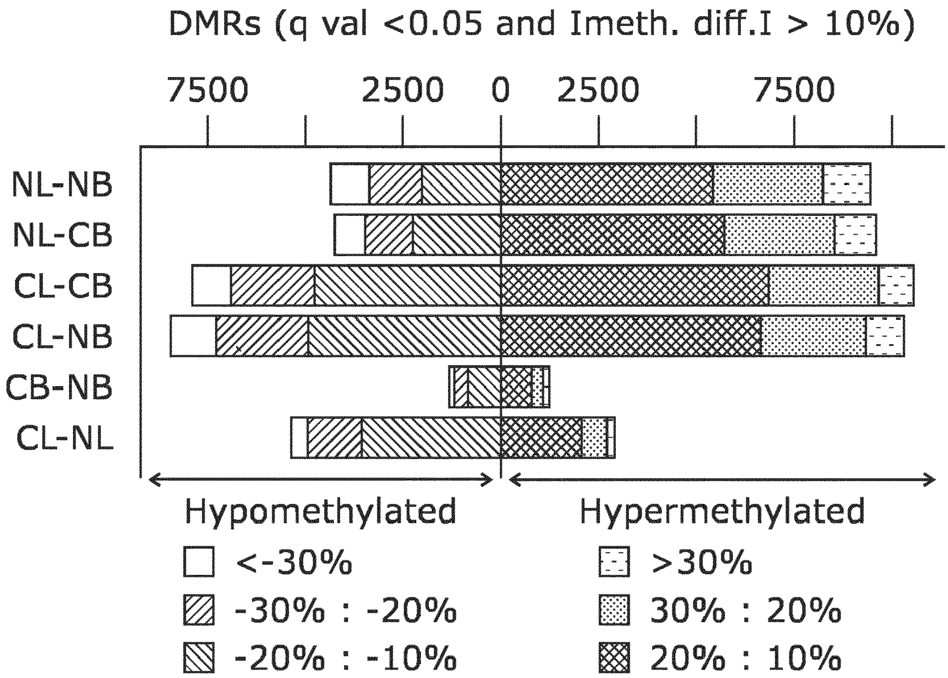


Figure 1D

EP 3 640 350 A1

SEQUENCE LISTING

<110> The University of York

<120> DIAGNOSTIC METHOD

<130> 4722P/EP

<160> 48

<170> PatentIn version 3.5

<210> 1
<211> 99
<212> DNA
<213> homo sapiens

<400> 1
agagctggtg tcggacgcac tgcagagacg tccctgtttt cggcgcgtcc tccgacaccc 60

agcagagagc ttggcgctct ccgctctgcg cgccccgagg 99

<210> 2
<211> 99
<212> DNA
<213> homo sapiens

<400> 2
gggcaattcc ggggcggtcg tgtcatcttt ctgggtgagt acacacgtga caccgagaga 60

agagaaagtg gtttcccagg ttggcggcac cagcacgtt 99

<210> 3
<211> 99
<212> DNA
<213> homo sapiens

<400> 3
tcttcgaaga gggcccacat gcggggctgc agcctcctcc agcggccaga tttgccgtcg 60

gggcccccga gccccgccgc gtcctcgatg cccagcctc 99

<210> 4
<211> 99
<212> DNA
<213> homo sapiens

<400> 4
gctggcggag gacgcggcct tcgtgctggg cctggcgggc gcatccgacc tgagcttccc 60

tgggccgccg cggccccggg gagccgccgc ctcccgcga 99

<210> 5
<211> 99
<212> DNA
<213> homo sapiens

<400> 5
gatggggctt gctggtcttg gccggccgtg cgcggtggag gagggctggc gaggcggcag 60

cagccgtgta ctcgccgtct ccgaggccgt ggaggaagg 99

<210> 6
<211> 99
<212> DNA
<213> homo sapiens

<400> 6
tggtcaacga gttcatcaca cgccagcgcc ggagggaact ctcagaccgc ttgaatctta 60

gtgaccagca ggtcaagatc tggtttcaga accggagaa 99

<210> 7
<211> 99
<212> DNA
<213> homo sapiens

<400> 7
tgtgtgcgtg ggttgagatg attatctctg aggtgtggtg cattccatga cctccgagaa 60

gctctacacg gccacccgcg ggccccaggg cgtcagccg 99

<210> 8
<211> 99
<212> DNA
<213> homo sapiens

<400> 8
cattgtggtg gtggcgatct cccccatcct cttctactca ggtaccgggg tccgcaaaaa 60

caaaaccatc acctgttacg acaccacctc agacgagta 99

<210> 9
<211> 99
<212> DNA
<213> homo sapiens

<400> 9
cctgaggacc caggaaagcg aggagcagaa gcggaaccgg gtgcgcggca tcctgcggat 60

catcaagccc tgcaaccatg tgctgagtct ctccttccc 99

<210> 10
<211> 99
<212> DNA
<213> homo sapiens

<400> 10
tggtcaacga gttcatcaca cgccagcgcc ggagggaact ctcagaccgc ttgaatctta 60

gtgaccagca ggtcaagatc tggtttcaga accggagaa 99

<210> 11
<211> 99
<212> DNA
<213> homo sapiens

<400> 11
ccgctgttta tctgtgtgcc ttaggaacag ccatttaatt acctcatctc tgggctcttg 60

ggaggaacat ggcaggtttt gaagcactta gcgccgggc 99

<210> 12
<211> 99
<212> DNA
<213> homo sapiens

<400> 12
aaagccagtg ccccatgcca cgcacctccc aggattagcc ccaggccggg aggctagagg 60

ccccacaagg agcgagcctg cgactgcgat ccacaggca 99

<210> 13
<211> 99
<212> DNA
<213> homo sapiens

<400> 13
agtgtgggga gaagcagctc ccgggagacg gagggctttg cgaagcggcg gcaatcaggg 60

gcggggcctg gaaaggaggg cagcgcggaa gcttggccc 99

<210> 14
<211> 99
<212> DNA
<213> homo sapiens

<400> 14
ggggaagcgc cccgcgcgcc aaggccccgc gctgctgagc tgtgagcacg gctgccccgt 60

ccgtccgtcc gtccagcacc cgcccggaga gtgaggcca 99

<210> 15
<211> 99
<212> DNA
<213> homo sapiens

<400> 15
gcccccaaca ggcgggtagg agccccgctc cccaggccct gagctgggcc ctgtgcgggt 60

ggggcgaggc ctgagcccca gaaggttcgg gggcggggc 99

<210> 16
<211> 99
<212> DNA
<213> homo sapiens

<400> 16
attccaagac agtattaaac tgatgcatat tttcataagt ccggacccca gggaacgcat 60

cctccagccc tcggcgcgga gcgggtgggc gcggcgagc 99

<210> 17

<211> 99
<212> DNA
<213> homo sapiens

<400> 17
attccaagac agtattaaac tgatgcatat tttcataagt ccggacccca gggaacgcat 60

cctccagccc tcggcgcgga gcgggtgggc gcggcgagc 99

<210> 18
<211> 99
<212> DNA
<213> homo sapiens

<400> 18
ctcccccggt gaccgctaaa cctaccggcc gcaaacatca cgaaatagaa ttaaacactg 60

gcgcctacta aggaccactc cacccagtac aaactaaat 99

<210> 19
<211> 99
<212> DNA
<213> homo sapiens

<400> 19
ggcccaggag agcccgctcc gcgctcacag cctccgttcc cagacacgcc cgggcctgag 60

cccccaggct gcactgtgcc acagaacaga ctccccggc 99

<210> 20
<211> 99
<212> DNA
<213> homo sapiens

<400> 20
cggtggggac agcacaggcg gggcggccga gccgagacct tcccaccccg ccccacgcct 60

ggttggggac ctgacgcgcc gtcgtgagtg gggcgggtg 99

<210> 21
<211> 99
<212> DNA
<213> homo sapiens

<400> 21
cgagcacagc cgaggccatg gaggtgacgg cggaccagcc gcgctgggtg agccaccacc 60

accccgccgt gctcaacggg cagcacccgg acacgcacc 99

<210> 22
<211> 99
<212> DNA
<213> homo sapiens

<400> 22
cgagcacagc cgaggccatg gaggtgacgg cggaccagcc gcgctgggtg agccaccacc 60

accccgccgt gctcaacggg cagcacccgg acacgcacc 99

<210> 23
<211> 99
<212> DNA
<213> homo sapiens

<400> 23
cctttctgtt ccccgccaag ctgtgctcaa tggtttctaa tgattctcac accgcgtttt 60

ctatgtcaaa tacataccgg gagttcattc ttaaatttc 99

<210> 24
<211> 99
<212> DNA
<213> homo sapiens

<400> 24
gccatgaagt tgacctgcgt gttgtacgtg gcctgcacgc tgcgccggat acgcagcatt 60

tccttgatgg tgtcctcatt gccatgccgg acctcaaag 99

<210> 25
<211> 99
<212> DNA
<213> homo sapiens

<400> 25
cgggcccctc tcaaatctag tggcctggtt ttattgagtg ggggaaggca catattttcc 60

aagattaact gccccgcaac agggaatttc gcaggattt 99

<210> 26
<211> 99
<212> DNA
<213> homo sapiens

<400> 26
ctcccccggt gaccgctaaa cctaccggcc gcaaacatca cgaaatagaa ttaaacactg 60

gcgcctacta aggaccactc cacccagtac aaactaaat 99

<210> 27
<211> 99
<212> DNA
<213> homo sapiens

<400> 27
gggtggggaa aggcagcgcc agggtcagag gcgccgaaag aagagcttgg agccgtggtc 60

cagcgtgcac tcgccggggc ccggccgact gggctgcag 99

<210> 28
<211> 99
<212> DNA
<213> homo sapiens

<400> 28

aagcgactga ttctcttttg tcttatcagt ctgggtcccc gacacgctac cttcggtttc 60

aggctgccta ggaccggaac cagggatcgc accgcagcc 99

<210> 29
<211> 99
<212> DNA
<213> homo sapiens

<400> 29
actcactgct tggtgcgcat tcaggccgcg cccacccggc atgggggtca cgtctgcacc 60

cttaatcgcg cgcacctgcg ctgtcccagg acagggaag 99

<210> 30
<211> 99
<212> DNA
<213> homo sapiens

<400> 30
tgttgctggg gaaggaaaac tcagggcgag gtgagagagg acagaacctc taaccaggaa 60

gaggctgtcc ccgccaagct ccggagaggc gcggaagca 99

<210> 31
<211> 99
<212> DNA
<213> homo sapiens

<400> 31
gcggctcctg cgggtgcccg gcctggcctc tccagctgcc tcagataatc gccctcgtaa 60

tcaccttccc tcacgctgct cctttaaaac atatttcca 99

<210> 32
<211> 99
<212> DNA
<213> homo sapiens

<400> 32
gtccccaggc cataccacgt cgctgacgcc ataccgatcc tggcaagaaa ggtgaccttg 60

taccagcccg gtgtccagcc ctccggaaac gtatatttt 99

<210> 33
<211> 99
<212> DNA
<213> homo sapiens

<400> 33
gaccgtttgc cgccctctcg ctgctctaga ccaggtggaa actcaagcct gcactcggga 60

acagaaccgc atctgcacct gcaggcccgg ctggtactg 99

<210> 34
<211> 99
<212> DNA

<213> homo sapiens

<400> 34
agtccccacc ccaggggcgc agctgccagc ccaacgcctg ggacacaccc cctcccgagg 60

cctctagccg ccgccgcgaa aatcccggaa accgtcctg 99

<210> 35
<211> 99
<212> DNA
<213> homo sapiens

<400> 35
aaccacgaag tcctgtggag aacgctggac aggctgccca ggacgcgcct ggggaagctt 60

cgagactgca acacacacga gagcctcctg gaagtgtgc 99

<210> 36
<211> 99
<212> DNA
<213> homo sapiens

<400> 36
cggggggccgc gcggagtttc cgcgggactt cctgctgtgg aggttaaagc acggtttgtc 60

aaaacgcctt agaacttgca atgccggcca ttcgcgtcg 99

<210> 37
<211> 99
<212> DNA
<213> homo sapiens

<400> 37
gaggggctga cggtggcctg ggtggccttg cgccggctct gggctccgcc gctgcggcag 60

ccgagggcct cagctgggaa atccaaaccc gggctggac 99

<210> 38
<211> 99
<212> DNA
<213> homo sapiens

<400> 38
acagagcctg ggggcggtga gagggcgggg ctaaagtgaa atgggcgggg ccggaggtga 60

gttggtggtt taggggcatg agctagggct gtgcctcta 99

<210> 39
<211> 99
<212> DNA
<213> homo sapiens

<400> 39
gccagccacg tccacctcga tcacgtcacc tcgtgggcag aatggcctac gccccatgtc 60

ctcccagccc cacccagaac ccacggtgag cacccggca 99

<210> 40
<211> 99
<212> DNA
<213> homo sapiens

<400> 40
tctgtgacat cacaggggt ttagtggcgc agcctgcggg acagaggccg gggatttgag 60

gtggcctcgt cttgtttgat ctcggggaac ctcggcaga 99

<210> 41
<211> 99
<212> DNA
<213> homo sapiens

<400> 41
tgatttcaag aagctggaca acaacataaa tcatatctaa ggacagcaaa aggcaggtac 60

gtcccggcct ggcctgcatg gggcgggggc cgcggaaga 99

<210> 42
<211> 99
<212> DNA
<213> homo sapiens

<400> 42
aacttcgtcc tgggggaggg agggtgctgg tctgcatcaa cccctccccc gggaccagcc 60

ccagcccgac caggctggcc ccgccatccc ctcagggcc 99

<210> 43
<211> 99
<212> DNA
<213> homo sapiens

<400> 43
caaacctgga ccagggggac ctggggacat ctgtggtttc gactcaatgg actccgccct 60

tgagtggctc cgacgggagc tggtgagtct ggtggggtg 99

<210> 44
<211> 99
<212> DNA
<213> homo sapiens

<400> 44
gcacttccgg actttctgca ggaagtgctg tctctgagtc attctgtttt gtcccgaggc 60

tgctgtgagg acggggacag gtgagcacat tctggaggg 99

<210> 45
<211> 99
<212> DNA
<213> homo sapiens

<400> 45
attgtaccgg ttgtttggct ctgagtggac gccactccca agaagggagc tcgggattcg 60

```
tcgtggcctt agagtgggta tggagccggg ctggccaga                              99


<210>  46
<211>  99
<212>  DNA
<213>  homo sapiens

<400>  46
aaagagtccc cagagccggc cccctggcac ccagactgca cggtgagaca acggagcccg       60

gagtggtggg accagcggcg gaaacagcgc tccatccgg                              99


<210>  47
<211>  99
<212>  DNA
<213>  homo sapiens

<400>  47
ttgaggagac agccgtcgtc cccggggtcc ctgtttgagg agacagccgt cgtccccggg       60

gtccctgttt gaggagacag ccatcgtccc cggggttcc                              99


<210>  48
<211>  99
<212>  DNA
<213>  homo sapiens

<400>  48
atgactcatg ctgtttcctg agccctggtg acacatgctc cctcccggac acgccctcag       60

tgcggcacta acccaccacc ttctagaaca cgaagcctc                              99
```

## Description

### Field of the Disclosure

The disclosure relates to a method for diagnosis of prostate adenocarcinoma (PCa) by determining methylation of selected genomic sites within the human genome; probes useful in determining methylation and kits comprising the probes and components useful in conducting the method are also disclosed.

### Background to the Disclosure

The prostate gland is the major accessory organ of the male reproductive tract and is the most common site of cancer in men. The two main pathologies of the gland are: benign prostatic hyperplasia, which is a non-malignant condition that is common with age; and prostate adenocarcinoma (PCa), which is the second most common cause of death in European men after lung cancer and is increasingly prevalent in our ageing Western Society. Symptoms include, blood in the semen or the urine, frequent pain or stiffness in the lower back, hips or upper thigh. PCa may be primary (i.e. located in the organ of origin) or secondary (i.e. tumours which form in other organs due to the ability of cancerous cells to move and invade other tissues via the circulatory system). The diagnosis of PCa is based on a combination of tests including digital rectal examination, serum PSA and prostate biopsy. This approach can lead to the over diagnosis of indolent cancer and delays in the diagnosis of significant disease. The latter are of most clinical concern.

PCa can vary from relatively harmless to extremely aggressive. Some prostate cancers are slow growing and cause few clinical symptoms. Aggressive PCas spread rapidly to the lymph nodes and other organs, especially bone. It is known that the growth of PCa can be inhibited by blocking the supply of male hormones such as testosterone. However, PCa eventually becomes independent of male sex hormones (i.e. they become androgen-independent prostate cancer cells). These cells are linked with aggressive, malignant prostate cancer. All male mammals have a prostate gland but only humans and dogs are known to naturally develop prostate cancer.

Metastatic prostate cancers predominantly move to the bone and are treated by reducing the production of androgens by blocking androgen production by the adrenal glands and testis. This treatment is only effective for a short period of time as the metastatic lesions become androgen independent and grow uncontrollably. The presence of androgen-independent prostate cancer cells means that this treatment regimen is no longer effective and further intervention is required to control the progress of the disease. A similar response is seen to chemotherapeutic and radiotherapy treatments. As a result, metastatic prostate cancer remains an incurable disease by current treatment strategies and early diagnosis is clearly desirable.

Methylation of cytosine (C) nucleotides in CpG dinucleotide sequences (CpG sites; G= guanosine) of the human DNA is a known phenomenon and is correlated with specific changes gene expression. Moreover, abnormal methylation (hypermethylation and/or hypomethylation) of specific genomic areas is present in virtually all cancer types. Genomic regions with altered methylation states in test samples compares to controls samples are commonly referred to as "differentially methylated regions" (DMRs). For example, WO2016/102674 discloses a sixteen gene methylation signature associated with the risk of developing aggressive prostate cancer and include methylation of regulatory regions of genes such as GSTP1, SFRP2, IGFBP3 and IGFBP7. WO2014/160829 discloses the methylation of eight genes and their association with prostate cancer which include determination of methylation status of one or more genes including CAV1, EVX1, MCF2L and FGF1. WO2013/185779 discloses a six gene methylation signature comprising genes HAPLN3, AOX1 and GAS6. Further examples of methylation signatures allegedly determinant of prostate cancer or the aggressiveness of prostate cancer are disclosed in WO2017/143296, WO2012/138609, WO2013/140161 and WO2008/143896.

This disclosure relates to a comparison of genome wide methylation profiles obtained separately from epithelial cells with luminal and basal phenotypes, isolated with a high purity from patient-matched normal and cancer biopsy samples. From comparative analyses of these profiles a major proportion of the methylation differences between normal basal and luminal cells were conserved in their malignant counterparts. This disclosure also makes it possible to identify, for the first time, regions specifically altered in the luminal fraction of PCa. The hypermethylated DMRs in this group were genes associated to genes involved in metabolic processes.

We disclose a set of CpG sites. These genomic regions were consistently altered in both tumour phenotypes in the PCa samples and can discriminate normal and PCa samples in prostate cancer analysed in bulk and presented in The Cancer Genome Atlas (TCGA) dataset. The new logistic model constructed from these regions makes use of only 17 probes to distinguish normal and PCa samples with similar specificity and sensitivity to previously developed, non-overlapping models[35,36], and will be useful in the context of the low mutagenic burdens seen in most hormone-naive prostate cancers.

We disclose that many DNA methylation changes commonly associated with PCa cells are explained by a predominant luminal phenotype of the treatment-naive PCa population and are not cancer-specific nor are likely to contain driver events. Importantly however, we disclose the identity of a class of PCa-specific DNA methylation changes that are specific to cancer luminal cells that can distinguish normal from cancer samples. The changes common to basal and luminal cancer cells are able to distinguish PCa efficiently from normal samples. This novel set of cancer-specific changes clearly demonstrate the potential of profiling normal and cancer cell subpopulations in identifying signatures that may contain previously unrecognized driver events in the development and progression of PCa.

**Statements of Invention**

We disclose methods of diagnosis of prostate cancer that measures methylation of prostate specific genomic biomarkers associated with upstream or downstream regulatory regions of genes which, when aberrantly methylated may result in gene dysregulation that is either causally related to cancer initiation or the result of cancer initiation; and including kits for testing methylation of said biomarkers comprising said probes.

The disclosure also relates to the analysis of expression of genes associated with the differential methylation of regulatory regions by determining the expression level of one or more genes selected from the group consisting of: FOXI2, IRS2, KCNC2, C3orf22, LOC10192826, HOXC12, VWA5B1, P2RY1, GLUD2, DDOST, BRF1, GABRB3, PLAGL1, L3MBTL1 and SC5D wherein the expression level is a measure of whether the subject has prostate cancer

Alternatively, the disclosure also relates to the analysis of expression of genes associated with the differential methylation of regulatory regions by determining the expression level of one or more genes selected from the group consisting of: TDRP, KCNQ1DN, CLEC18A, GATA3, MIR2467, MIR6792, ADCY1 wherein the expression level is a measure of whether the subject has prostate cancer.

Furthermore, the disclosure relates to the analysis of expression of genes associated with the differential methylation of regulatory regions by determining the expression level of one or more genes selected from the group consisting of: TDRP, VPS28, EFEMP1, LOC10013165, C4orf19, UNCX, CUX2, MIR4632, BARX1, KCNB2, ZNF251, STK3, MAN2B1, RASA3, KLF11, NKX6-2, MAPK11, FAM167B, SLITRK1, PRRX1, MYCNOS, C8orf31 or SH3BGRL3 to determine whether a subject diagnosed with prostate cancer has aggressive or non-aggressive prostate cancer.

According to an aspect of the invention there is provided a diagnostic method to determine the methylation status of one or more genomic regions isolated from a human subject that is suspected of having prostate cancer comprising the steps:

i) obtaining an isolated biological sample from a subject and extracting genomic DNA to provide an isolated sample of genomic DNA; and

ii) determining the methylation status of one or more CpG dinucleotides of said genomic DNA comprising or consisting of one or more a genomic nucleotide sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16 or SEQ ID NO: 17 and wherein said region can be extended 250bp upstream or downstream of said region; or a polymorphic sequence variant that has at least 90% sequence identity over the full length to the recited nucleotide sequences set forth in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15 , SEQ ID NO: 16 or SEQ ID NO: 17, and wherein said region can be extended 250bp upstream or downstream of said region; as a measure of the likelihood said subject has prostate cancer.

To obtain these signatures, first DNA methylation patterns were analysed in four different sub-populations of prostate cells: Normal Basal (NB), Normal Luminal (NL), Cancer Basal (CB), Cancer Luminal (CL), using a technique called Reduced Representation Bisulphite Sequencing (RRBS). DMRs were then calculated for each pair-wise comparison between populations (e.g. CL vs NL). A logistic regression model was then constructed on the probes of the TCGA dataset overlapping the genomic locations of specific sets of DMRs and used to identify statistical relevant probes that could distinguish cancerous (Cancer) vs non-cancerous (Normal) samples, and samples confined within the prostate capsule (Organ Confined - non-aggressive) vs those that already escaped outside or the prostate (Extraprostatic - aggressive).

Methods to determine methylation status of genomic DNA are well known to the skilled person, for example by bisulphite sequencing or related techniques such as: methylation specific PCR, microarray analysis of bisulphite converted DNA, pyrosequencing methylation assay. In a preferred method of the invention the polymorphic sequence variant that has at least 91%, 92%, 93%, 94%, 95%, 96%, 97%, 98% or 99% sequence identity over the full length to said nucleotide sequence.

In a preferred method of the invention said genomic DNA region comprises one or more of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16 or SEQ ID NO: 17.

In a preferred method of the invention the method determines the methylation status of one, two, three, four, five, six, seven, eight, nine, ten, eleven, twelve, thirteen, fourteen, fifteen, sixteen or seventeen genomic regions set forth in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16 or SEQ ID NO: 17.

In a preferred method of the invention the method determines the methylation status of each of the genomic regions set forth in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16 and SEQ ID NO: 17.

In a preferred method of the invention methylation status of genomic DNA is hypermethylation wherein hypermethylation is typically associated with down regulation of the gene associated with the genomic region.

Preferably hypermethylation is of CpGs in one, more or all of the genomic regions as set forth in SEQ ID NO: 3, SEQ ID NO: 4, SEQ ID NO: 5, SEQ ID NO: 8, SEQ ID NO: 9, SEQ ID NO: 11, SEQ ID NO: 14, SEQ ID NO: 16 or SEQ ID NO: 17.

In a preferred method of the invention methylation status of genomic DNA is hypomethylation wherein hypomethylation is typically associated with up-regulation of the gene associated with the genomic region.

Preferably hypomethylation is of CpG in one, more or all of the nucleotide sequence(s) as set forth in SEQ ID NO: 1, SEQ ID NO: 2, SEQ ID NO: 6, SEQ ID NO: 7, SEQ ID NO: 10, SEQ ID NO: 12, SEQ ID NO: 13 or SEQ ID NO: 15.

In a preferred method of the invention the biological sample is analysed for expression of one or more genes selected from the group consisting of FOXI2, IRS2, KCNC2, C3orf22, LOC101928266, HOXC12, VWA5B1, P2RY1, GLUD2, DDOST, BRF1, GABRB3, PLAGL1, L3MBTL and SC5D and analysed for the differential methylation status of one or more genomic regions set forth in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16 or SEQ ID NO: 17.

According to an alternative aspect of the invention there is provided a diagnostic method to determine the methylation status of one or more genomic regions isolated from a human subject that is suspected of having prostate cancer comprising the steps:

i) obtaining an isolated biological sample from a subject and extracting genomic DNA to provide an isolated sample of genomic DNA;
ii) determining the methylation status of one or more CpG dinucleotides of said genomic DNA comprising or consisting of one or more genomic nucleotide sequence selected from the group consisting of SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, and wherein said region can be extended 250bp upstream or downstream of said region, or a polymorphic sequence variant that has at least 90% sequence identity over the full length to the recited nucleotide sequences set forth in SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25 and wherein said region can be extended 250bp upstream or downstream of said region, as a measure of the likelihood said subject has prostate cancer.

In a preferred method of the invention said genomic DNA region comprises or consists of SEQ ID NO: 18, SEQ ID NO: 19, SEQ ID NO: 20, SEQ ID NO: 21, SEQ ID NO: 22, SEQ ID NO: 23, SEQ ID NO: 24 or SEQ ID NO: 25.

In a preferred method of the invention the method determines the methylation status of one, two, three, four, five, six, seven or eight probes corresponding to the genomic regions set forth in SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25.

In a preferred method of the invention the method determines the methylation status of each of the genomic regions set forth in SEQ ID NO: 18, SEQ ID NO: 19, SEQ ID NO: 20, SEQ ID NO: 21, SEQ ID NO: 22, SEQ ID NO: 23, SEQ ID NO: 24 and SEQ ID NO: 25.

In a preferred method of the invention methylation status of genomic DNA is hypermethylation wherein hypermethylation is typically associated with down regulation of the gene associated with the genomic region.

Preferably hypermethylation is of CpG represented in one, more or all of the genomic regions as set forth in SEQ ID NO: 18, SEQ ID NO: 19, SEQ ID NO: 20, SEQ ID NO: 21, SEQ ID NO: 22, SEQ ID NO: 23 or SEQ ID NO: 25.

In a preferred method of the invention methylation status of genomic DNA is hypomethylation wherein hypomethylation is typically associated with up regulation of the gene associated with the genomic region.

Preferably hypomethylation is of CpG represented in the genomic region set forth in SEQ ID NO: 24.

In a preferred method of the invention said biological sample is analysed for expression of one or more genes selected from the group consisting of TDRP, KCNQ1DN, CLEC18A, GATA3, MIR2467, MIR6792, ADCY1 and analysed for the differential methylation status of one or more genomic regions set forth in SEQ ID NO: 18, SEQ ID NO: 19, SEQ ID NO: 20, SEQ ID NO: 21, SEQ ID NO: 22, SEQ ID NO: 23, SEQ ID NO: 24 and SEQ ID NO: 25.

According to an aspect of the invention there is provided a diagnostic method to determine the methylation status of one or more genomic regions isolated from a human subject that has prostate cancer comprising the steps:

i) obtaining an isolated biological sample from a subject and extracting genomic DNA to provide an isolated sample of genomic DNA;
ii) determining the methylation status of one or more CpG dinucleotides of said genomic DNA comprising or consisting of one or more genomic nucleotide sequence selected from the group consisting of SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47 or SEQ ID NO: 48 and wherein said region can be extended 250bp upstream or downstream of said region., or a polymorphic sequence variant that has at least 90% sequence identity over the full length to the recited nucleotide sequences set forth in SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47 or SEQ ID NO: 48 and wherein said region can be extended 250bp upstream or downstream of said region; as a measure of whether the prostate cancer is aggressive or non-aggressive.

In a preferred method of the invention said genomic DNA region comprises or consist of genomic regions set forth in SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47 or SEQ ID NO: 48.

In a preferred method of the invention the method determines the methylation status of one, two, three, four, five, six, seven, eight, nine, ten, eleven, twelve, thirteen, fourteen, fifteen, sixteen, seventeen, eighteen, nineteen, twenty, twenty one, twenty two, twenty three genomic regions set forth in SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47 or SEQ ID NO: 48.

In a preferred method of the invention the method determines the methylation status of one or more CpG dinucleotides of said genomic DNA consisting of SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47 or SEQ ID NO: 48.

In a preferred method of the invention the method determines the methylation status of each of the genomic regions set forth in SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47 and SEQ ID NO: 48.

In a preferred method of the invention methylation status of genomic DNA is hypermethylation wherein hypermethylation is typically associated with down regulation of the gene associated with the genomic region.

Preferably hypermethylation is of CpG represented in one, more or all of the genomic regions as set forth in SEQ ID NO: 26, SEQ ID NO: 28, SEQ ID NO: 29, SEQ ID NO: 31, SEQ ID NO: 33, SEQ ID NO: 34, SEQ ID NO: 36, SEQ ID NO: 37, SEQ ID NO: 38, SEQ ID NO: 40, SEQ ID NO: 42, SEQ ID NO: 46 or SEQ ID NO: 48.

In a preferred method of the invention methylation status of genomic DNA is to hypomethylation wherein hypomethylation is typically associated with up-regulation of the gene associated with the genomic region.

Preferably hypomethylation is of CpG represented in one, more or all of the genomic regions set forth in SEQ ID NO: 27, SEQ ID NO: 30, SEQ ID NO: 32, SEQ ID NO: 35, SEQ ID NO: 39, SEQ ID NO: 41, SEQ ID NO: 43, SEQ ID NO: 44, SEQ ID NO: 45 or SEQ ID NO: 47.

In a preferred method of the invention the biological sample is analysed for expression of one or more genes selected from the group consisting of TDRP, VPS28, EFEMP1, LOC100131655, C4orf19, UNCX, CUX2, MIR4632, BARX1, KCNB2, ZNF251, STK3, MAN2B1, RASA3, KLF11, NKX6-2, MAPK11, FAM167B, SLITRK1, PRRX1, MYCNOS, C8orf31 or SH3BGRL3 and analysed for differential methylation status of one or more genomic regions set forth in SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47 or SEQ ID NO: 48.

In a preferred method of the invention the expression of each gene is correlated with the methylation status of each genomic region.

According to an aspect of the invention there is provided a diagnostic method to determine the methylation status of one or more genomic regions of a human subject that is suspected of having prostate cancer comprising the steps:

i) obtaining a biological sample from a subject and extracting genomic DNA to provide an isolated sample comprising genomic DNA;
ii) determining the methylation status of at least one region of genomic DNA by interrogating the methylation status associated with one or more probes selected from the group consisting of cg02523640, cg02315096, cg06563089, cg04527018, cg10116893, cg06729806, cg22333412, cg03293976, cg03356806, cg13233461, cg24876897, cg01153451, cg14859324, cg10007452, cg09541000, cg15628253 and cg21745537; as a measure of the likelihood that said subject has prostate cancer.

In a preferred method of the invention said extracted genomic DNA is contacted with one, two, three, four, five, six, seven, eight, nine, ten, eleven, twelve, thirteen, fourteen, fifteen, sixteen or seventeen probes selected from the group consisting of cg02523640, cg02315096, cg06563089, cg04527018, cg10116893, cg06729806, cg22333412, cg03293976, cg03356806, cg13233461, cg24876897, cg01153451, cg14859324, cg10007452, cg09541000, cg15628253 and cg21745537.

Typically, probes useful in the analysing genomic DNA use the microarray platform Infinium HumanMethylation450K BeadChip and interrogating any of the probes mentioned above.

In a preferred method of the invention said extracted genomic DNA is contacted with each probe set forth in: cg02523640, cg02315096, cg06563089, cg04527018, cg10116893, cg06729806, cg22333412, cg03293976, cg03356806, cg13233461, cg24876897, cg01153451, cg14859324, cg10007452, cg09541000, cg15628253 and cg21745537.

In a preferred method of the invention the biological sample is analysed for expression of one or more genes selected from the group consisting of FOXI2, IRS2, KCNC2, C3orf22, LOC10192826, HOXC12, VWA5B1, P2RY1, GLUD2, DDOST, BRF1, GABRB3, PLAGL1, L3MBTL1 and SC5D and analysed for the differential methylation status of one or more genomic regions as determined by one or more probes selected from the group consisting of: cg02523640, cg02315096, cg06563089, cg04527018, cg10116893, cg06729806, cg22333412, cg03293976, cg03356806, cg13233461, cg24876897, cg01153451, cg14859324, cg10007452, cg09541000, cg15628253 and cg21745537.

In a preferred method of the invention the expression of each gene is correlated with the methylation status of each genomic region as determined by each of said probes. According to an aspect of the invention there is provided a diagnostic method to determine the methylation status of one or more genomic regions of a human subject that is suspected of having prostate cancer comprising the steps:

i) obtaining a biological sample from a subject and extracting genomic DNA to provide an isolated sample comprising genomic DNA;
ii) determining the methylation status of at least one region of genomic DNA by interrogating the methylation status associated with one or more methylated probes selected from the group consisting of; cg00066748, cg08376310, cg15192750, cg15267232, cg17124583, cg19125791, cg23044391, cg26459372; as a measure of the likelihood said subject has prostate cancer.

In a preferred method of the invention said extracted genomic DNA is contacted with one, two, three, four, five, six, seven or eight probes selected from the group consisting of cg00066748, cg08376310, cg15192750, cg15267232, cg17124583, cg19125791, cg23044391, cg26459372.

In a preferred method of the invention said extracted genomic DNA is contacted with each probe set forth as: cg00066748, cg08376310, cg15192750, cg15267232, cg17124583, cg19125791, cg23044391, cg26459372.

In a preferred method of the invention the biological sample is analysed for expression of one or more genes selected from the group consisting of TDRP, KCNQ1DN, CLEC18A, GATA3, MIR2467, MIR6792, ADCY1 and analysed for the differential methylation status of one or more genomic regions as determined by one or more probes selected from the group consisting of: cg00066748, cg08376310, cg15192750, cg15267232, cg17124583, cg19125791, cg23044391, cg26459372.

According to an aspect of the invention there is provided a diagnostic method to determine the methylation status of one or more genomic regions of a human subject having prostate cancer comprising the steps:

i) obtaining a biological sample from a subject and extracting genomic DNA to provide an isolated sample comprising genomic DNA;
ii) determining the methylation status of at least one region of genomic DNA by interrogating the methylation status associated with one or more probes selected from the group consisting of; cg00066748, cg01285435, cg03122624, cg03731646, cg04301614, cg04804717, cg05977462, cg06740893, cg07076509, cg09433131, cg12010198, cg15830431, cg17737967, cg18020065, cg18825594, cg20595634, cg20811788, cg21155609, cg21265647, cg21914290, cg22755142, cg23986375, cg24348240;
as a measure of whether the prostate cancer is aggressive or non-aggressive

In a preferred method of the invention said extracted genomic DNA is contacted with one, two, three, four, five, six, seven, eight, nine, ten, eleven, twelve, thirteen, fourteen, fifteen, sixteen, seventeen, eighteen, nineteen, twenty, twenty one, twenty two or twenty three probes selected from the group consisting of cg00066748, cg01285435, cg03122624, cg03731646, cg04301614, cg04804717, cg05977462, cg06740893, cg07076509, cg09433131, cg12010198, cg15830431, cg17737967, cg18020065, cg18825594, cg20595634, cg20811788, cg21155609, cg21265647, cg21914290, cg22755142, cg23986375, cg24348240.
In a preferred method of the invention said extracted genomic DNA is contacted with each probe set forth as: cg00066748, cg01285435, cg03122624, cg03731646, cg04301614, cg04804717, cg05977462, cg06740893, cg07076509, cg09433131, cg12010198, cg15830431, cg17737967, cg18020065, cg18825594, cg20595634, cg20811788, cg21155609, cg21265647, cg21914290, cg22755142, cg23986375, cg24348240.
In a preferred method of the invention the biological sample is analysed for expression of one or more genes selected from the group consisting of TDRP, VPS28, EFEMP1, LOC10013165, C4orf19, UNCX, CUX2, MIR4632, BARX1, KCNB2, ZNF251, STK3, MAN2B1, RASA3, KLF11, NKX6-2, MAPK11, FAM167B, SLITRK1, PRRX1, MYCNOS, C8orf31 or SH3BGRL3, and analysed for the differential methylation status of one or more genomic regions as determined by one or more probes selected from the group consisting of: cg00066748, cg01285435, cg03122624, cg03731646, cg04301614, cg04804717, cg05977462, cg06740893, cg07076509, cg09433131, cg12010198, cg15830431, cg17737967, cg18020065, cg18825594, cg20595634, cg20811788, cg21155609, cg21265647, cg21914290, cg22755142, cg23986375, cg24348240.
In a preferred method of the invention said biological sample is selected from the group consisting of: urine, seminal fluid, blood, lymph fluid, or prostate tissue.
Preferably the isolated biological sample is a tissue biopsy. Preferably said biopsy is a prostate tissue biopsy.
According to an aspect of the invention there is provided a diagnostic method for determining if a subject has or is susceptible to prostate cancer comprising:

i) providing an isolated biological sample to be tested and preparing cDNA;
ii) forming a preparation comprising said cDNA and an oligonucleotide primer pairs adapted to anneal to a nucleic acid molecule comprising a nucleotide sequence encoding one or more polypeptides selected from the group consisting of: FOXI2, IRS2, KCNC2, C3orf22, LOC10192826, HOXC12, VWA5B1, P2RY1, GLUD2, DDOST, BRF1, GABRB3, PLAGL1, L3MBTL1 and SC5D, a thermostable DNA polymerase, deoxynucleotide triphosphates and co-factors;
iii) providing polymerase chain reaction conditions sufficient to amplify all or part of said nucleic acid molecule(s);
iv) analyzing the amplified products of said polymerase chain reaction for the presence and/or level of said nucleic acid molecule encoding said polypeptide as a measure of gene expression; and
v) comparing the amplified product with a normal matched control.

According to an aspect of the invention there is provided a diagnostic method for determining if a subject has or is susceptible to prostate cancer comprising:

i) providing an isolated biological sample to be tested and preparing cDNA;
ii) forming a preparation comprising said cDNA and an oligonucleotide primer pairs adapted to anneal to a nucleic acid molecule comprising a nucleotide sequence encoding one or more polypeptides selected from the group consisting of: TDRP, KCNQ1DN, CLEC18A, GATA3, MIR2467, MIR6792, ADCY1, a thermostable DNA polymerase, deoxynucleotide triphosphates and co-factors;
iii) providing polymerase chain reaction conditions sufficient to amplify all or part of said nucleic acid molecule(s);
iv) analyzing the amplified products of said polymerase chain reaction for the presence and/or level of said nucleic acid molecule encoding said polypeptide as a measure of gene expression; and
v) comparing the amplified product with a normal matched control.

According to an aspect of the invention there is provided a method for determining if a subject has aggressive or non-aggressive prostate cancer comprising:

i) providing an isolated biological sample to be tested and preparing cDNA;
ii) forming a preparation comprising said cDNA and an oligonucleotide primer pairs adapted to anneal to a nucleic acid molecule comprising a nucleotide sequence encoding one or more polypeptides selected from the group consisting of: TDRP, VPS28, EFEMP1, LOC10013165, C4orf19, UNCX, CUX2, MIR4632, BARX1, KCNB2, ZNF251, STK3, MAN2B1, RASA3, KLF11, NKX6-2, MAPK11, FAM167B, SLITRK1, PRRX1, MYCNOS, C8orf31 or SH3BGRL3, a thermostable DNA polymerase, deoxynucleotide triphosphates and co-factors;

iii) providing polymerase chain reaction conditions sufficient to amplify all or part of said nucleic acid molecule(s);

iv) analyzing the amplified products of said polymerase chain reaction for the presence and/or level of said nucleic acid molecule encoding said polypeptide as a measure of gene expression; and

v) comparing the amplified product with a normal matched control.

Any primers/probes designed to the nucleic acids encoding polypeptides selected from the group consisting of: TDRP, VPS28, EFEMP1, LOC10013165, C4orf19, UNCX, CUX2, MIR4632, BARX1, KCNB2, ZNF251, STK3, MAN2B1, RASA3, KLF11, NKX6-2, MAPK11, FAM167B, SLITRK1, PRRX1, MYCNOS, C8orf31 and SH3BGRL3; or TDRP, KCNQ1DN, CLEC18A, GATA3, MIR2467, MIR6792 and ADCY1; or FOXI2, IRS2, KCNC2, C3orf22, LOC10192826, HOXC12, VWA5B1, P2RY1, GLUD2, DDOST, BRF1, GABRB3, PLAGL1, L3MBTL1 and SC5D are part of a kit for use in the methods described above. The kit can comprise further DNA polymerase, deoxynucleotide triphosphates and co-factors.

Methods to analyse gene expression are known in the art. For example, quantitative PCR, also referred to as real time PCR. Alternatively, gene expression can be assessed in situ on isolated biological samples. *In situ* detection can also be via PCR amplification of mRNA expressed from target genes, this can also be a real time assay.

Throughout the description and claims of this specification, the words "comprise" and "contain" and variations of the words, for example "comprising" and "comprises", means "including but not limited to", and is not intended to (and does not) exclude other moieties, additives, components, integers or steps. "Consisting essentially" means having the essential integers but including integers which do not materially affect the function of the essential integers.

Throughout the description and claims of this specification, the singular encompasses the plural unless the context otherwise requires. Where the indefinite article is used, the specification is to be understood as contemplating plurality as well as singularity, unless the context requires otherwise.

Features, integers, characteristics, compounds, chemical moieties or groups described in conjunction with a aspect, embodiment or example of the invention are to be understood to be applicable to any other aspect, embodiment or example described herein unless incompatible therewith.

An embodiment of the invention will now be described by example only and with reference to the following figures:

Fig. 1: Identification of DMRs between prostate cancer cell populations. (A) Representative FACS profiles of a cell suspension prepared from core needle biopsies of a radical prostatectomy sample. (**B**) Heatmap showing scaled methylation values of the top 1% most variable regions (100 bp bins) in the samples analysed. Hierarchical clustering is based on Euclidean distance of the unscaled values and complete linkage. (**C**) Diagram showing all pairwise comparisons carried out. (**D**) Number of DMRs found in each comparison. (**E**) Overlap of DMRs with CpG islands, shores (2 kb flanking islands) or shelves (2 kb flanking shores). P-values from hypergeometric test against all regions. E = enriched, D = depleted. (**F**) Distribution of distances of DMRs to the closest TSS. Grey box indicates $\pm$5 kb from a TSS. Purple lines: hypermethylated DMRs, orange lines: hypomethylated DMRs, gry line: all regions. (**G**) Proportion of DMRs proximal or distal to TSSs. P-values from hypergeometric test against all regions. E = enriched, D = depleted;

Fig. 2: Hypermethylated distal DMRs have features of enhancers. (**A**) Average plots of evolutionary conservation scores of the distal DMRs in each set. Purple lines: hypermethylated DMRs; orange lines: hypomethylated DMRs, gray line: all regions. P-values are from bootstrapping analysis. (**B**) Proportion of distal DMRs overlapping with DHSs (identified by ENCODE). P-values from hypergeometric test against all regions. E = enriched, D = depleted. (**C**) Overlap of distal DMRs with ChIP-seq derived TFBSs (identified by ENCODE). P-values are from hypergeometric tests against all regions. E = enriched, D = depleted. (**D**) Overlap of each set of distal DMRs with repetitive elements (UCSC repeatMask), SINEs, LINEs and LTRs. P-values from hypergeometric tests against all regions. E = enriched, D = depleted. (**E**) Number of GO terms enriched by each set of DMRs. GO terms identified using GREAT (FDR<0.05 and at least 3 genes in the set);

Fig. 3: Shared phenotype-specific DMRs. (A) Overlap between the DMRs identified in the NL-NB and CL-CB comparisons. P-values derived from Fisher's exact test. (**B**) Heatmap showing scaled methylation values of the DMRs identified in the NL-NB (left) or CL-CB (right) comparisons. Hierarchical clustering is based on Euclidean distances of the unscaled values and complete linkage. (**C**) TFBSs enriched in the hypermethylated (purple) or hypomethylated (orange) DMRs common between the NL-NB and CL-CB comparisons. Left panel: analysis performed using HOMER findMotifs, p-values from binomial test. Right panel: enrichment of ENCODE defined TFBSs, p-values from hypergeometric test against all regions. (**D**) Frequently hyper- or hypomethylated genes in PCa[1] that were also hypermethylated (purple) or hypomethylated (orange) in the NL-NB and CL-CB comparisons. (**E-F**) Genome browser plots of the promoter regions of GSTP1 (**E**) and CCDC8 (**F**). Grey squares are the bins analysed. Lines and shaded areas represent mean ±SEM of each category (NB=light blue, NL=light red, CB=dark blue, CL=dark red). DMRs are shown on top: hypermethylated=purple, hypomethylated=orange;

Fig. 4: Aberrant methylation in CL. (A) Frequently hyper- or hypomethylated genes in PCa[1] that are also hypermethylated (purple) or hypomethylated (orange) in the CL-CB and CL-NL comparisons. (B) Overlap between the DMRs identified in the CL-CB and CL-NL comparisons. P-values derived from Fisher's exact test. (C) Clustering of the gene ontologies (biological process) enriched in DMRs common between the CL-CB and CL-NL comparisons based on information similarity. Each circle shows an individual GO term enriched in regions hypermethylated (purple), hypomethylated (orange) or both (green), the size of the circles is proportional to the enrichment p-value. The 2 main clusters of GO terms determined by k-means are highlighted (light blue and pink), and named after the most frequent terms. (D) Heatmap showing scaled methylation values (β-values) of probes overlapping the DMRs common to the CL-CB and CL-NL comparisons in the PCa samples (magenta) and matched normal samples (green) within the TCGA dataset. Hierarchical clustering based on Euclidean distances of the unscaled values and complete linkage. The dark green and gray clusters were generated by cutting the tree at the first bifurcation. (E) Heatmap showing scaled methylation values (β-values) of probes overlapping the DMRs common to the CL-CB and CL-NL comparisons in the PCa samples (matched normal samples not included) of the TCGA dataset. Hierarchical clustering based on Euclidean distance of the unscaled values and complete linkage. The dark green and gray clusters are generated by cutting the tree at the first bifurcation;

Fig. 5: PCa-specific DMRs shared between CB and CL. (A) Overlap between the DMRs identified in the CL-NL and CB-NB comparisons. P-values derived from Fisher's exact test. (**B**) Genome browser views of KCNC2 promoter (top) and RHCG exon 2 (bottom). Grey squares are the bins analysed. Lines and shaded areas represent mean ±SEM of each category (NB=light blue, NL=light red, CB=dark blue, CL=dark red). DMRs are shown on top: hypermethylated=purple, hypomethylated=orange. (**C**) Heatmap showing scaled methylation values of probes overlapping the DMRs common between CL-CB and CB-NB in the matched normal and cancer samples within the TCGA dataset. Hierarchical clustering based on Euclidean distances of the unscaled values and complete linkage. The dark green and gray clusters were generated by cutting the tree at the first 2 bifurcations. (**D**) Selection of a 17-probe signature distinguishing normal and PCa samples applying LASSO regression on a logistic model of the training dataset (70% of the TCGA samples). Lines show the changes in coefficients in relation to different lambdas. The vertical dashed line shows the optimal lambda identified using cross-validation. (**E**) Receiver-operating characteristic curve generated by applying the optimal logistic model to the test dataset (30% of the TCGA samples). (**F**) Heatmap showing scaled methylation values of the 17-probe signature in the test dataset (30% of the TCGA samples). The bar plot on the left side shows the final coefficients for each probe in the model, and the bar plot on top shows the logistic probability generated by for each sample (Green: normal samples, magenta: cancer samples);

Figure 6: Cancer and extraprostatic specific signatures. (A) Selection of an 8-probe signature distinguishing normal and PCa samples applying LASSO regression on a logistic model of the training dataset (70% of the TCGA samples) composed of the probes presented in figure 4D. Lines show the changes in coefficients in relation to different lambdas. The vertical dashed line shows the optimal lambda identified using cross-validation. (B) Receiver-operating characteristic curve generated by applying the optimal logistic model generated in A to the test dataset (30% of the TCGA samples). (C) Heatmap showing scaled methylation values of the 8-probe signature generated in A in the test dataset (30% of the TCGA samples). The bar plot on the left side shows the final coefficients for each probe in the model, and the bar plot on top shows the logistic probability generated by for each sample (Green: normal samples, magenta: cancer samples). (D) Selection of a 23-probe signature distinguishing organ confined and extraprostatic PCa samples applying LASSO regression on a logistic model of the training dataset (70% of the TCGA samples) composed of the probes presented in figure 4E. Lines show the changes in coefficients in relation to different lambdas. The vertical dashed line shows the optimal lambda identified using cross-validation. (B) Receiver-operating characteristic curve generated by applying the optimal logistic model generated in D to the test dataset (30% of the TCGA samples). (C) Heatmap showing scaled methylation values of the 23-probe signature generated in D in the test dataset (30% of the TCGA samples). The bar plot on the left side shows the final coefficients for each probe in the model, and the bar plot on top shows the logistic probability generated by for each sample (Light blue: organ confined, orange: extraprostatic).

Supplementary Figure 1: Validation of the FACS sorting strategy for basal and luminal cells. (**A**) Immunofluorescence for pan-cytokeratin (PanCK), cytokeratin 5 (KRT5), cytokeratin 8 (KRT8) and androgen receptor (AR) on cytospin preparations of sorted EpCAM+CD49f+CD24- and EpCAM+CD49f-CD24+ cells. The pie charts show the proportion of negative (white) dim (grey) and bright (black) cells for each marker. At least 100 cells per population per marker were counted. For AR, the fraction of dim or positive cells with cytoplasmic (yellow) or nuclear (blue) localization is also shown. One representative image is shown for each marker in the green channel, and DAPI counterstain in blue. Scale bars = 10 μm. (**B**) qRT-PCR analysis performed on FACS sorted EpCAM+CD49f+CD24- and EpCAM+CD49f-CD24+ cells from one matched tumor directed and contralateral biopsy. Bar show mean ±SEM of the log10(ΔΔCt values) using GAPDH as housekeeping gene as NB population as a reference sample. (**C**) Table of the pre-operation clinical features of the donors for the samples used for the DNA methylation analysis (PSA measured in ng/ml);

Supplementary Fig. 2: Identification of differentially methylated CpGs between prostate cancer cell populations. (A) Heatmap showing scaled methylation values of the top 1% most variable CpGs in the samples analysed. Hierarchical clustering is based on Euclidean distance of the unscaled values and complete linkage. (B) Number of differentially methylated CpGs found in each comparison. (C) Distribution of distances of differentially methylated CpGs to the closest TSS. Grey box indicates ±5 kb from a TSS. Purple lines: hypermethylated, orange lines: hypomethylated, gray line: all CpGs. (D) Overlap of differentially methylated CpGs with CpG islands, shores (2 kb flanking islands) or shelves (2 kb flanking shores). P-values from hypergeometric test against all CpGs analysed. E = enriched, D = depleted. (E) Heatmap showing scaled methylation values of the differentially methylated CpGs identified in the NL-NB (left) or CL-CB (right) comparisons. Hierarchical clustering is based on Euclidean distances of the unscaled values and complete linkage.

Supplementary Fig. 3: Proximal DMRs are associated with differential expression. (A) Dot-plot showing for each differentially expressed gene associated with a proximal (<5 kb from TSS) DMR in NL-NB differential methylation (x axis) and differential expression (y axis) in similarly selected cell populations. Each dot represents one gene/DMR association; purple dots: hypermethylated and downregulated genes; orange dots: hypomethylated and upregulated genes; blue line: least squares linear fit. (B) Top: Venn diagrams showing the overlap of the DMRs obtained in the NL-NB and NL-CB comparisons (p-values from Fisher's exact test). Bottom: dot-plot showing the methylation difference of the DMRs identified in the NL-NB (green), NL-CB (red) comparisons, or both (Black). (C) Top: Venn diagrams showing the overlap of the DMRs obtained in the CL-CB and CL-NB comparisons. Bottom: dot-plot showing the methylation difference of the DMRs identified in the CL-CB (green), CL-NB (red) comparisons, or both (Black).

Supplementary Fig. 4: Gene ontology enrichment analysis. Clustering of the gene ontologies (biological process) enriched in DMRs identified in the NL-NB (left) CL-CB (middle) and CL-NL (right) comparisons based on information similarity. Each circle shows an individual GO term, the size of the circles is proportional to the enrichment p-value. The 3 main clusters of GO terms determined by k-means are highlighted.

Supplementary Fig. 5: Phenotype-specific distal DMRs are highly enriched in enhancer features. (A) Distribution of distances of DMRs common between the NL-NB and CL-CB comparisons to the closest TSS. Grey box indicates ±5 kb from TSS. Purple line: hypermethylated DMRs, orange line: hypomethylated DMRs, gray line: all regions. (B) Average plots of evolutionary conservation scores of the distal DMRs common between the NL-NB and CL-CB comparisons. Purple line: hypermethylated DMRs, orange line: hypomethylated DMRs, gray line: all regions. P-values from bootstrapping analysis. (C) Proportion of distal DMRs common between the NL-NB and CL-CB comparisons that overlapped with DHSs (identified by ENCODE). P-values from hypergeometric test against all regions. E = enriched, D = depleted. (D) Overlap of distal DMRs common between the NL-NB and CL-CB comparisons with ChIP-seq-derived TFBSs (identified by ENCODE). P-values from hypergeometric test against all regions. E = enriched, D = depleted. (E) Top 15 gene ontologies enriched in hypermethylated and hypomethylated DMRs common between NL-NB and CL-CB. P-values from hypergeometric test (FDR<0.05 and at least 3 genes in the set). (F) Clustering of the gene ontologies (biological process) enriched in DMRs common between the NL-NB and CL-CB comparisons based on information similarity. Each circle shows an individual GO term, the size of the circles is proportional to the enrichment p-value. The 3 main clusters of GO terms determined by k-means are highlighted.

Supplementary Fig. 6: Aberrant methylation in luminal cells from PCa samples. (A) Distribution of distances of the DMRs common between the CL-CB and CL-NL comparisons to the closest TSS. Grey box indicates ±5 kb from TSS. Purple line: hypermethylated DMRs, orange lines hypomethylated DMRs, gray line: all regions. (B) Average plots of evolutionary conservation scores of the distal DMRs common between the CL-CB and CL-NL comparisons. Purple line: hypermethylated DMRs, orange lines hypomethylated DMRs, gray line: all regions. P-values from bootstrapping analysis. (C) Proportion of distal DMRs common between the CL-CB and CL-NL comparisons that overlapped with DHSs (identified by ENCODE). P-values from hypergeometric test against all regions. E = enriched, D = depleted. (D) Overlap of distal DMRs common between the CL-CB and CL-NL comparisons with ChIP-seq-derived TFBSs (identified by ENCODE). P-values from hypergeometric test against all regions. E = enriched, D = depleted. (E) Overlap of each set of distal DMRs common between the CL-CB and CL-NL comparisons with repetitive elements (UCSC repeatMask), SINEs, LINEs and LTRs. P-values from hypergeometric test against all regions. E = enriched, D = depleted. (F) TFBSs enriched in the DMRs common between the CL-CB and CL-NL comparisons. Top panel: enrichment of ENCODE defined TFBSs, p-values from hypergeometric tests against all regions. Bottom panel: analysis performed using HOMER findMotifs, p-values from binomial tests. (G) Heatmap showing scaled methylation values of probes in the TCGA dataset (all samples) overlapping the DMRs common between the CL-CB and CL-NL comparisons. Hierarchical clustering based on Euclidean distance of the unscaled values and complete linkage. The dark green and gray clusters are generated by cutting the tree at the first 2 bifurcations.

Supplementary Fig. 7: PCa-specific DMRs shared by both basal and luminal subsets. (A) Heatmap showing scaled methylation values of the DMRs common between the CL-CB and CB-NB comparisons. Hierarchical clustering is based on Euclidean distance of the unscaled values and complete linkage. (B) Heatmap showing scaled methylation values of probes in the TCGA dataset (all samples) overlapping the DMRs common between the CL-CB and CB-NB comparisons. Hierarchical clustering based on Euclidean distance of the unscaled values and complete linkage. The dark green and gray clusters are generated by cutting the tree at the first 3 bifurcations.

**Table S1:** Quality metrics for all RRBS libraries generated.

Table 1: Methylation signature SEQ ID N0 1-17

| SEQ ID NO | chromosome | start | end | probe_ID | LASSO_ logistic_ model_ coefficient | distance _to_ nearest_ TSS | nearest_ TSS_gene_ symbol | nearest_ TSS_ gene-accession_ number | RRBS_ hyper_ start | RRBS_ hyper_ end | RRBS_ hypo_ start | RRBS_ hypo_end | DMR_sequence |
|---|---|---|---|---|---|---|---|---|---|---|---|---|---|
| 1 | chr10 | 129,534,612 | 129,534,613 | cg02523640 | 3.135376798 | -925 | FOXI2 | NM_ 207426 | | | 129,534,601 | 129,534,700 | AGAGCTGGTGTCGGACGCACTGCAGAGACGTCCCTGTTTTCGGCGCGTCCTCCGACACCCAGCAGAGAGCTTGGCGCTCTCCGCTCTGCGCGCCCGAGG |
| 2 | chr13 | 110,522,019 | 110,522,020 | cg02315096 | 2.87279041 | -83,105 | IRS2 | NM_ 003749 | | | 110,522,001 | 110,522,100 | GGGCAATTCCGGGGCGGTCGTGTCATCTTTCTGGGTGAGTACACACGTGACACCGAGAGAAGAGAAAGTGGTTTCCCAGGTTGGCGGCACCAGCACGTT |
| 3 | chr12 | 75,601,156 | 75,601,157 | cg06563089 | 2.246033642 | 2,372 | KCNC2 | NM_ 001260497 | 75,601,101 | 75,601,200 | | | TCTTCGAAGAGGGCCCACATGCGGGGCTGCAGCCTCCTCCAGCGGCCAGATTTGCCGTCGGGGCCCCCGAGCCCCGCCGCGTCCTCGATGCCCAGCCTC |

(continued)

| SEQ ID NO | chromo-some | start | end | probe_ID | LASSO_logistic_model_coefficient | distance_to_nearest_TSS | nearest_TSS_gene_symbol | nearest_TSS_gene-accession_number | RRBS_hyper_start | RRBS_hyper_end | RRBS_hypo_start | RRBS_hypo_end | DMR_sequence |
|---|---|---|---|---|---|---|---|---|---|---|---|---|---|
| 4 | chr3 | 126,261,297 | 126,261,298 | cg04527018 | 2.141570426 | 16,461 | C3orf22 | NM_152533 | 126,261,201 | 126,261,300 | | | GCTGGCGGAGGACGCGGCCTTCGTGCTGGGCCTGGCGGGCGCATCCGACCTGAGCTTCCCTGGGCCGCCGCGGCCCCGGGGAGCCGCCGCCTCCCGCGA |
| 5 | chr17 | 9,019,123 | 9,019,124 | cg10116893 | 2.009681394 | 63,312 | LOC101928266 | NR_110828 | 9,019,101 | 9,019,200 | | | GATGGGGCTTGCTGGTCTTGGCCGGCCGTGCGCGGTGGAGGAGGGCTGGCGAGGCGGCAGCAGCCGTGTACTCGCCGTCTCCGAGGCCGTGGAGGAAGG |
| 6 | chr12 | 54,350,293 | 54,350,294 | cg06729806 | 1.828922367 | 1,580 | HOXC12 | NM_173860 | | | 54,350,201 | 54,350,300 | TGGTCAACGAGTTCATCACACGCCAGCGCCGGAGGGAACTCTCAGACCGCTTGAATCTTAGTGACCAGCAGGTCAAGATCTGGTTTCAGAACCGGAGAA |

(continued)

| SEQ ID NO | chromo-some | start | end | probe_ID | LASSO_logistic_model_coefficient | distance_to_nearest_TSS | nearest_TSS_gene_symbol | nearest_TSS_gene-accession_number | RRBS_hyper_start | RRBS_hyper_end | RRBS_hypo_start | RRBS_hypo_end | DMR_sequence |
|---|---|---|---|---|---|---|---|---|---|---|---|---|---|
| 7 | chr1 | 20,569,455 | 20,569,456 | cg22333412 | 1.54537724 | -47,956 | VWA5B1 | NM_001039500 | | | 20,569,401 | 20,569,500 | TGTGTGCGTGGGTTGAGATGATTATCTCTGAGGTGTGGTGCATTCCATGACCTCCGAGAAGCTCTACACGGCCACCCGCGGGCCCCAGGGCGTCAGCCG |
| 8 | chr3 | 152,554,194 | 152,554,195 | cg03293976 | 0.4385642 | 1,459 | P2RY1 | NM_002563 | 152,554,101 | 152,554,200 | | | CATTGTGGTGGTGGCGATCTCCCCCATCCTCTTCTACTCAGGTACCGGGGTCCGCAAAAACAAAACCATCACCTGTTACGACACCACCTCAGACGAGTA |
| 9 | chrX | 120,181,819 | 120,181,820 | cg03356806 | 0.079070901 | 358 | GLUD2 | NM_012084 | 120,181,801 | 120,181,900 | | | CCTGAGGACCCAGGAAAGCGAGGAGCAGAAGCGGAACCGGGTGCGCGGCATCCTGCGGATCATCAAGCCCTGCAACCATGTGCTGAGTCTCTCCTTCCC |

(continued)

| SEQ ID NO | chromo-some | start | end | probe_ID | LASSO_logistic_model_coefficient | distance_to_nearest_TSS | nearest_TSS_gene_symbol | nearest_TSS_gene-accession_number | RRBS_hyper_start | RRBS_hyper_end | RRBS_hypo_start | RRBS_hypo_end | DMR_sequence |
|---|---|---|---|---|---|---|---|---|---|---|---|---|---|
| 10 | chr12 | 54,350,208 | 54,350,209 | cg13233461 | 0.007024662 | 1,495 | HOXC12 | NM_173860 | | | 54,350,201 | 54,350,300 | TGGTCAACGAGTTCATCACACGCCAGCGCCGGAGGGAACTCTCAGACCGCTTGAATCTTAGTGACCAGCAGGTCAAGATCTGGTTTCAGAACCGGAGAA |
| 11 | chr1 | 20,988,602 | 20,988,603 | cg24876897 | 0.151677159 | -565 | DDOST | NM_005216 | 20,988,601 | 20,988,700 | | | CCGCTGTTTATCTGTGTGCCTTAGGAACAGCCATTTAATTACCTCATCTCTGGGCTCTTGGGAGGAACATGGCAGGTTTTGAAGCACTTAGCGCCGGGC |
| 12 | chr14 | 105,714,273 | 105,714,274 | cg01153451 | -1.125337639 | -51 | BRF1 | NM_145685 | | | 105,714,201 | 105,714,300 | AAAGCCAGTGCCCCATGCCACGCACCTCCCAGGATTAGCCCCAGGCCGGGAGGCTAGAGGCCCCACAAGGAGCGAGCCTGCGACTGCGATCCACAGGCA |

(continued)

| SEQ ID NO | chromo-some | start | end | probe_ID | LASSO_logistic_model_coefficient | distance_to_nearest_TSS | nearest_TSS_gene_symbol | nearest_TSS_gene-accession_number | RRBS_hyper_start | RRBS_hyper_end | RRBS_hypo_start | RRBS_hypo_end | DMR_sequence |
|---|---|---|---|---|---|---|---|---|---|---|---|---|---|
| 13 | chr15 | 26,874,362 | 26,874,363 | cg14859324 | -2.575559744 | -37 | GABRB3 | NM_001191321 | | | 26,874,301 | 26,874,400 | AGTGTGGGGAGAAGCAGCTCCCGGGAGACGGAGGGCTTTGCGAAGCGGCGGCAATCAGGGGCGGGGCCTGGAAAGGAGGGCAGCGCGGAAGCTTGGCCC |
| 14 | chr6 | 144,329,330 | 144,329,331 | cg10007452 | -4.093208345 | 211 | PLAGL1 | NM_001080952 | 144,329,301 | 144,329,400 | | | GGGGAAGCGCCCCGCGCGCCAAGGCCCCGCGCTGCTGAGCTGTGAGCACGGCTGCCCCGTCCGTCCGTCCGTCCAGCACCCGCCCGGAGAGTGAGGCCA |
| 15 | chr20 | 42,143,488 | 42,143,489 | cg09541000 | -4.649570108 | 413 | L3MBTL1 | NM_015478 | | | 42,143,401 | 42,143,500 | GCCCCCAACAGGCGGGTAGGAGCCCCGCTCCCCAGGCCCTGAGCTGGGCCCTGTGCGGGTGGGGCGAGGCCTGAGCCCCAGAAGGTTCGGGGGCGGGGC |

(continued)

| SEQ ID NO | chromo-some | start | end | probe_ID | LASSO_logistic_model_coefficient | distance_to_nearest_TSS | nearest_TSS_gene_symbol | nearest_TSS_gene-accession_number | RRBS_hyper_start | RRBS_hyper_end | RRBS_hypo_start | RRBS_hypo_end | DMR_sequence |
|---|---|---|---|---|---|---|---|---|---|---|---|---|---|
| 16 | chr11 | 121,233,756 | 121,233,757 | cg15628253 | -7.818536303 | 70,180 | SC5D | NM_001024956 | 121,233,701 | 121,233,800 | | | ATTCCAAGACAGTATTAAACTGATGCATATTTTCATAAGTCCGGACCCCAGGGAACGCATCCTCCAGCCCTCGGCGCGGAGCGGGTGGGCGCGGCGAGC |
| 17 | chr11 | 121,233,742 | 121,233,743 | cg21745537 | -16.13056529 | 70,166 | SC5D | NM_001024956 | 121,233,701 | 121,233,800 | | | ATTCCAAGACAGTATTAAACTGATGCATATTTTCATAAGTCCGGACCCCAGGGAACGCATCCTCCAGCCCTCGGCGCGGAGCGGGTGGGCGCGGCGAGC |

Table 2: Methylation signature SEQ ID NO 18-25

| SEQ ID NO | chromosome | start | end | probe_ID | LASSO_ logistic_ model _coefficient | distance to_near- est _TSS | nearest_ TSS_gen e_symbol | nearest_ TSS_gene_ accession_ number | RRBS_ hyper_ start | RRBS_ hyper_ end | RRBS_ hypo_ start | RRBS_ hypo_ end | DMR_sequence |
|---|---|---|---|---|---|---|---|---|---|---|---|---|---|
| 18 | chr8 | 496,526 | 496,527 | cg00066748 | - 0.217688671 | -745 | TDRP | NM_ 175075 | 496 ,50 1 | 496 ,60 0 | | | CTCCCCCGGT GACCGCTAAA CCTACCGGCC GCAAACATCA CGAAATAGAA TTAAACACTG GCGCCTACTA AGGACCACTC CACCCAGTAC AAACTAAAT |
| 19 | chr11 | 2,858,620 | 2,858,62 1 | cg08376310 | 1.438919448 | -32,642 | KCNQ1D N | NR_ 024627 | 2,8 58, 601 | 2,8 58, 700 | | | GGCCCAGGA GAGCCCGCTC CGCGCTCACA GCCTCCGTTC CCAGACACGC CCGGGCCTGA GCCCCCAGGC TGCACTGTGC CACAGAACAG ACTCCCCGGC |
| 20 | chr16 | 69,999,424 | 69,999,4 25 | cg15192750 | 0.786175106 | 14,536 | CLEC18 A | NM_ 001271 197 | 69, 999 ,40 1 | 69, 999 ,50 0 | | | CGGTGGGGA CAGCACAGGC GGGGCGGCC GAGCCGAGAC CTTCCCACCC CGCCCCACGC CTGGTTGGGG ACCTGACGCG CCGTCGTGAG TGGGGCGGG TG |

(continued)

| SEQ ID NO | chromosome | start | end | probe_ID | LASSO_ logistic_ model _coefficient | distance to_near- est _TSS | nearest_ TSS_gen e_symbol | nearest_ TSS_gene_ accession_ number | RRBS_ hyper_ start | RRBS_ hyper_ end | RRBS_ hypo_ start | RRBS_ hypo_ end | DMR_sequence |
|---|---|---|---|---|---|---|---|---|---|---|---|---|---|
| 21 | chr10 | 8,097,688 | 8,097,68 9 | cg15267232 | 6.384944501 | 1,022 | GATA3 | NM_ 001002 295 | 8,0 97, 601 | 8,0 97, 700 | | | CGAGCACAGC CGAGGCCATG GAGGTGACGG CGGACCAGCC GCGCTGGGTG AGCCACCACC ACCCCGCCGT GCTCAACGGG CAGCACCCGG ACACGCACC |
| 22 | chr10 | 8,097,640 | 8,097,64 1 | cg17124583 | 0.870598079 | 974 | GATA3 | NM_ 001002 295 | 8,0 97, 601 | 8,0 97, 700 | | | CGAGCACAGC CGAGGCCATG GAGGTGACGG CGGACCAGCC GCGCTGGGTG AGCCACCACC ACCCCGCCGT GCTCAACGGG CAGCACCCGG ACACGCACC |
| 23 | chr2 | 240,265,914 | 240,265, 915 | cg19125791 | -0.23483559 | 7,585 | MIR2467 | NR_ 039948 | 240 ,26 5,9 01 | 240 ,26 6,0 00 | | | CCTTTCTGTTC CCCGCCAAGC TGTGCTCAAT GGTTTCTAAT GATTCTCACA CCGCGTTTTC TATGTCAAATA CATACCGGGA GTTCATTCTTA AATTTC |

(continued)

| SEQ ID NO | chromosome | start | end | probe_ID | LASSO_ logistic_ model _coefficient | distance to_near- est _TSS | nearest_ TSS_gen e_symbol | nearest_ TSS_gene_ accession_ number | RRBS_ hyper_ start | RRBS_ hyper_ end | RRBS_ hypo_ start | RRBS_ hypo_ end | DMR_sequence |
|---|---|---|---|---|---|---|---|---|---|---|---|---|---|
| 24 | chr19 | 7,685,238 | 7,685,23 9 | cg23044391 | - 0.202072272 | 2,914 | MIR6792 | NR_ 106850 | | | 7,685 , 201 | 7,685 , 300 | GCCATGAAGT TGACCTGCGT GTTGTACGTG GCCTGCACGC TGCGCCGGAT ACGCAGCATT TCCTTGATGG TGTCCTCATT GCCATGCCGG ACCTCAAAG |
| 25 | chr7 | 45,613,675 | 45,613,6 76 | cg26459372 | 1.664355442 | -63 | ADCY1 | NM_ 001281 768 | 45, 613 ,60 1 | 45, 613 ,70 0 | | | CGGGCCCCTC TCAAATCTAGT GGCCTGGTTT TATTGAGTGG GGGAAGGCAC ATATTTTCCAA GATTAACTGC CCCGCAACAG GGAATTTCGC AGGATTT |

Table 3: Methylation signature SEQ ID NO 26-48

| SEQ ID NO | chromosome | start | end | probe_ID | LASSO_logistic_model_coefficient | distance_to_nearest_TSS | nearest_TSS_gene_symbol | nearest_TSS_gene_accession_number | RRBS_hyper_start | RRBS_hyper_end | RRBS_hypo_start | RRBS_hypo_end | DMR_sequence |
|---|---|---|---|---|---|---|---|---|---|---|---|---|---|
| 26 | chr8 | 496,52 6 | 496,52 7 | cg00066748 | -1.407778584 | -745 | TDRP | NM_175075 | 496,501 | 496,60 0 | | | CTCCCCCGGTGACCGCTAAACCTACCGGCCGCAAACATCACGAAATAGAATTAAACACTGGCGCCTACTAAGGACCACTCCACCCAGTACAAACTAAAT |
| 27 | chr8 | 145,654,564 | 145,654,565 | cg01285435 | -0.960912814 | -618 | VPS28 | NM_016208 | | | 145 , 65 4,501 | 145 , 65 4,600 | GGGTGGGGAAAGGCAGCGCCAGGGTCAGAGGCGCCGAAAGAAGAGCTTGGAGCCGTGGTCCAGCGTGCACTCGCCGGGGCCCGGCCGACTGGGCTGCAG |
| 28 | chr2 | 56,150,340 | 56,150,341 | cg03122624 | 0.276292383 | 958 | EFEMP1 | NM_001039348 | 56,150,301 | 56,150,400 | | | AAGCGACTGATTCTCTT TTGTCTTATCAGTCTGGGTCCCCGACACGCTACCTTCGGTTTCAGGCTGCCTAGGACCGGAACCAGGGATCGCACCGCAGCC |
| 29 | chr18 | 74,499, 371 | 74,499, 372 | cg03731646 | 1.221037593 | 34,880 | LOC100 1 31655 | NR_040024 | 74,499, 301 | 74,499, 400 | | | ACTCACTGCTTGGTGCGCATTCAGGCCGCGCCCACCCGGCATGGGGGTCACGTCTGCACCCTTAATCGCGCGCACCTGCGCTGTCCCAGGACAGGGAAG |

(continued)

| SEQ ID NO | chromo-some | start | end | probe_ID | LASSO_logistic_model_coefficient | distance_to_nearest_TSS | nearest_TSS_gene_symbol | nearest_TSS_gene_accession_number | RRBS_hyper_start | RRBS_hyper_end | RRBS_hypo_start | RRBS_hypo_end | DMR_sequence |
|---|---|---|---|---|---|---|---|---|---|---|---|---|---|
| 30 | chr4 | 37,585,992 | 37,585,993 | cg04301614 | -0.894555357 | 138 | C4orf19 | NM_018302 | | | 37,585,901 | 37,586,000 | TGTTGCTGGGGAAGGAAAACTCAGGGCGAGGTGAGAGAGGACAGAACCTCTAACCAGGAAGAGGCTGTCCCCGCCAAGCTCCGGAGAGGCGCGGAAGCA |
| 31 | chr7 | 1,300,319 | 1,300,320 | cg04804717 | -0.203997011 | 27,666 | UNCX | NM_001080461 | 1,300,301 | 1,300,400 | | | GCGGCTCCTGCGGGTGCCCGGCCTGGCCTCTCCAGCTGCCTCAGATAATCGCCCTCGTAATCACCTTCCCTCACGCTGCTCCTTTAAAACATATTTCCA |
| 32 | chr12 | 111,475,135 | 111,475,136 | cg05977462 | -0.147423766 | 3,308 | CUX2 | NM_015267 | | | 111,475,101 | 111,475,200 | GTCCCCAGGCCATACCACGTCGCTGACGCCATACCGATCCTGGCAAGAAAGGTGACCTTGTACCAGCCCGGTGTCCAGCCCTCCGGAAACGTATATTTT |
| 33 | chr1 | 12,251,868 | 12,251,869 | cg06740893 | -0.566384155 | 99 | MIR4632 | NR_039775 | 12,251,801 | 12,251,900 | | | GACCGTTTGCCGCCCTCTCGCTGCTCTAGACCAGGTGGAAACTCAAGCCTGCACTCGGGAACAGAACCGCATCTGCACCTGCAGGCCCGGCTGGTACTG |

(continued)

| SEQ ID NO | chromo-some | start | end | probe_ID | LASSO_ logistic_ model_coefficient | distance _to_ nearest_ TSS | nearest_ TSS_ gene_ symbol | nearest_ TSS_ gene_ accession _number | RRBS_ hyper_ start | RRBS_ hyper_ end | RRBS_ hypo_ start | RRBS_ hypo_ end | DMR_sequence |
|---|---|---|---|---|---|---|---|---|---|---|---|---|---|
| 34 | chr9 | 96,720, 818 | 96,720, 819 | cg0707650 9 | 0.10890761 8 | -3,210 | BARX1 | NM_ 021570 | 96,720, 801 | 96,720, 900 | | | AGTCCCCACCCCAGGG GCGCAGCTGCCAGCCC AACGCCTGGGACACAC CCCCTCCCGAGGCCTC TAGCCGCCGCCGCGAA AATCCCGGAAACCGTC CTG |
| 35 | chr8 | 73,480, 178 | 73,480, 179 | cg0943313 1 | - 0.00944197 | 30,553 | KCNB2 | NM_ 004770 | | | 73, 480 , 10 1 | 73, 480 , 20 0 | AACCACGAAGTCCTGT GGAGAACGCTGGACAG GCTGCCCAGGACGCG CCTGGGGAAGCTTCGA GACTGCAACACACACG AGAGCCTCCTGGAAGT GTGC |
| 36 | chr8 | 145,93 9,498 | 145,93 9,499 | cg1201019 8 | 0.28059533 3 | 41,472 | ZNF251 | NM_ 138367 | 145,93 9,401 | 145,93 9,500 | | | CGGGGGCCGCGCGGA GTTTCCGCGGGACTTC CTGCTGTGGAGGTTAA AGCACGGTTTGTCAAA ACGCCTTAGAACTTGC AATGCCGGCCATTCGC GTCG |
| 37 | chr8 | 99,952, 590 | 99,952, 591 | cg1583043 1 | 1.59053601 4 | 2,209 | STK3 | NM_ 001256 312 | 99,952, 501 | 99,952, 600 | | | GAGGGGCTGACGGTG GCCTGGGTGGCCTTGC GCCGGCTCTGGGCTCC GCCGCTGCGGCAGCC GAGGGCCTCAGCTGG GAAATCCAAACCCGGG CTGGAC |

(continued)

| SEQ ID NO | chromo-some | start | end | probe_ID | LASSO_logistic_model_coefficient | distance_to_nearest_TSS | nearest_TSS_gene_symbol | nearest_TSS_gene_accession_number | RRBS_hyper_start | RRBS_hyper_end | RRBS_hypo_start | RRBS_hypo_end | DMR_sequence |
|---|---|---|---|---|---|---|---|---|---|---|---|---|---|
| 38 | chr19 | 12,758,715 | 12,758,716 | cg17737967 | 0.431963731 | 18,876 | MAN2B1 | NM_000528 | 12,758,701 | 12,758,800 | | | ACAGAGCCTGGGGGCGGTGAGAGGGCGGGGCTAAAGTGAAATGGGCGGGGCCGGAGGTGAGTTGGTGGTTTAGGGGCATGAGCTAGGGCTGTGCCTCTA |
| 39 | chr13 | 114,829,732 | 114,829,733 | cg18020065 | -1.093004886 | 68,363 | RASA3 | NM_007368 | | | 114 , 82 9,7 01 | 114 , 82 9,8 00 | GCCAGCCACGTCCACCTCGATCACGTCACCTCGTGGGCAGAATGGCCTACGCCCCATGTCCTCCCAGCCCCACCCAGAACCCACGGTGAGCACCCGGCA |
| 40 | chr2 | 10,184,649 | 10,184,650 | cg18825594 | -0.210860902 | 278 | KLF11 | NM_001177718 | 10,184,601 | 10,184,700 | | | TCTGTGACATCACAGGGGGTTTAGTGGCGCAGCCTGCGGGACAGAGGCCGGGGATTTGAGGTGGCCTCGTCTTGTTTGATCTCGGGGAACCTCGGCAGA |
| 41 | chr10 | 134,662,560 | 134,662,561 | cg20595634 | 2.946490029 | -63,023 | NKX6-2 | NM_177400 | | | 134 , 66 2,5 01 | 134 , 66 2,6 00 | TGATTTCAAGAAGCTGGACAACAACATAAATCATATCTAAGGACAGCAAAAGGCAGGTACGTCCCGGCCTGGCCTGCATGGGGCGGGGGCCGCGGAAGA |

(continued)

| SEQ ID NO | chromo-some | start | end | probe_ID | LASSO_logistic_model_coefficient | distance_to_nearest_TSS | nearest_TSS_gene_symbol | nearest_TSS_gene_accession_number | RRBS_hyper_start | RRBS_hyper_end | RRBS_hypo_start | RRBS_hypo_end | DMR_sequence |
|---|---|---|---|---|---|---|---|---|---|---|---|---|---|
| 42 | chr22 | 50,721,367 | 50,721,368 | cg20811788 | 0.317796092 | -12,545 | MAPK11 | NM_002751 | 50,721,301 | 50,721,400 | | | AACTTCGTCCTGGGGGAGGGAGGGTGCTGGTCTGCATCAACCCCTCCCCCGGGACCAGCCCCAGCCCGACCAGGCTGGCCCCGCCATCCCCTCAGGGCC |
| 43 | chr1 | 32,713,274 | 32,713,275 | cg21155609 | -0.17501249 | 457 | FAM167B | NM_032648 | | | 32,713,201 | 32,713,300 | CAAACCTGGACCAGGGGGACCTGGGGACATCTGTGGTTTCGACTCAATGGACTCCGCCCTTGAGTGGCTCCGACGGGAGCTGGTGAGTCTGGTGGGGTG |
| 44 | chr13 | 84,480,708 | 84,480,709 | cg21265647 | 0.098730046 | -24,180 | SLITRK1 | NM_001281503 | | | 84,480,701 | 84,480,800 | GCACTTCCGGACTTTCTGCAGGAAGTGCTGTCTCTGAGTCATTCTGTTTTGTCCCGAGGCTGCTGTGAGGACGGGGACAGGTGAGCACATTCTGGAGGG |
| 45 | chr1 | 170,635,959 | 170,635,960 | cg21914290 | -0.33427762 | 2,647 | PRRX1 | NM_006902 | | | 170,635,901 | 170,636,000 | ATTGTACCGGTTGTTTGGCTCTGAGTGGACGCCACTCCCAAGAAGGGAGCTCGGGATTCGTCGTGGCCTTAGAGTGGGTATGGAGCCGGGCTGGCCAGA |

(continued)

| SEQ ID NO | chromo-some | start | end | probe_ID | LASSO_logistic_model_coefficient | distance_to_nearest_TSS | nearest_TSS_gene_symbol | nearest_TSS_gene_accession_number | RRBS_hyper_start | RRBS_hyper_end | RRBS_hypo_start | RRBS_hypo_end | DMR_sequence |
|---|---|---|---|---|---|---|---|---|---|---|---|---|---|
| 46 | chr2 | 16,243, 376 | 16,243, 377 | cg2275514 2 | - 0.87433136 | - 161,53 1 | MYCNO S | NR_ 026766 | 16,243, 301 | 16,243, 400 | | | AAAGAGTCCCCAGAGC CGGCCCCCTGGCACCC AGACTGCACGGTGAGA CAACGGAGCCCGGAGT GGTGGGACCAGCGGC GGAAACAGCGCTCCAT CCGG |
| 47 | chr8 | 144,15 5,291 | 144,15 5,292 | cg2398637 5 | - 2.73029881 7 | 34,613 | C8orf31 | NM_ 173687 | | | 144 , 15 5,2 01 | 144 , 15 5,3 00 | TTGAGGAGACAGCCGT CGTCCCCGGGGTCCCT GTTTGAGGAGACAGCC GTCGTCCCCGGGGTCC CTGTTTGAGGAGACAG CCATCGTCCCCGGGGT TCC |
| 48 | chr1 | 26,611, 452 | 26,611, 453 | cg2434824 0 | - 0.00349261 8 | 5,240 | SH3BGR L3 | NM_ 031286 | 26,611, 401 | 26,611, 500 | | | ATGACTCATGCTGTTTC CTGAGCCCTGGTGACA CATGCTCCCTCCCGGA CACGCCCTCAGTGCGG CACTAACCCACCACCTT CTAGAACACGAAGCCT C |

Key Table 1-3

| | |
|---|---|
| Chromosome | Location of methylated C interrogated by the probe (hg19 genome) |
| Start | |
| End | |
| probe_ID | Official probe ID of Illumina Array |
| LASSO_logistic_model_coefficient | Coefficient used in the logistic model to calculate the probability of a sample being prostate cancer vs normal prostate |
| CpG_context | Is the probe in a CpG island, CpG shore or CpG shelf? |
| distance_to_nearest_TSS | Distance to the closest Transcription Start Site |
| nearest_TSS_gene_symbol | official gene symbol of the closest Transcription Start Site |
| nearest_TSS_gene_accession_number | official accession number of the closest Transcription Start Site |
| | Genomic location (hg19 genome) of the differentially methylated region (hypermethylated) identified in the RRBS dataset and overlaping with the probe |
| RRBS_hyper_start | |
| RRBS_hyper_end | |
| | Genomic location (hg19 genome) of the differentially methylated region (hypomethylated) identified in the RRBS dataset and overlaping with the probe |
| RRBS_hypo_start | |
| RRBS_hypo_end | |
| DMR_sequence | Sequence of the DMR (hg19 genome) |

Table S1

| library | patient code | population | total read pairs | aligned pairs | % aligned | unique pairs | % unique | covered Cs in CpGs | mean coverage |
|---|---|---|---|---|---|---|---|---|---|
| zr546_3 | H309 | CB | 27,152,869 | 19,270,327 | 71 | 15,731,688 | 57.9 | 8,752,994 | 7.24 |
| zr546_8 | H323 | CB | 27,234,823 | 19,822,591 | 72.8 | 15,845,172 | 58.2 | 8,771,236 | 7.05 |
| zr530_3 | H324 | CB | 28,307,024 | 20,957,541 | 74 | 16,044,500 | 56.7 | 9,107,598 | 7.83 |
| zr546_11 | H329 | CB | 24,067,754 | 14,876,511 | 61.8 | 12,073,590 | 50.2 | 7,953,823 | 5.62 |
| zr546_4 | H309 | CL | 29,197,085 | 20,737,947 | 71 | 16,778,451 | 57.5 | 8,903,552 | 7.58 |
| zr546_9 | H323 | CL | 33,480,373 | 24,271,977 | 72.5 | 18,886,669 | 56.4 | 9,189,984 | 8.20 |
| zr530_4 | H324 | CL | 30,804,021 | 21,860,796 | 71 | 16,731,816 | 54.3 | 9,228,160 | 7.19 |
| zr546_12 | H329 | CL | 26,902,300 | 19,253,888 | 71.6 | 15,564,034 | 57.9 | 8,614,119 | 6.95 |
| zr546_1 | H309 | NB | 27,792,651 | 20,000,596 | 72 | 16,241,098 | 58.4 | 8,770,562 | 7.51 |
| zr530_1 | H324 | NB | 31,258,528 | 23,037,775 | 73.7 | 17,977,073 | 57.5 | 9,276,371 | 8.27 |
| zr546_10 | H329 | NB | 29,004,417 | 20,087,364 | 69.3 | 16,164,281 | 55.7 | 8,626,487 | 7.10 |
| zr546_2 | H309 | NL | 27,842,679 | 20,034,519 | 72 | 16,024,003 | 57.6 | 9,009,515 | 7.36 |
| zr530_2 | H324 | NL | 36,524,536 | 26,508,686 | 72.6 | 20,457,980 | 56 | 9,631,029 | 9.00 |
| zr546_5 | H329 | NL | 30,902,206 | 21,827,712 | 70.6 | 17,317,759 | 56 | 8,816,603 | 7.87 |
| MEAN: | | | 29,319,376 | 20,896,302 | 71.1 | 16,559,865 | 56.5 | 8,903,717 | 7.48 |
| MIN: | | | 24,067,754 | 14,876,511 | 61.8 | 12,073,590 | 50.2 | 7,953,823 | 5.62 |
| MAX: | | | 36,524,536 | 26,508,686 | 74 | 20,457,980 | 58.4 | 9,631,029 | 9.00 |

## Materials and Methods

### Tissue processing

Prostate tissues were obtained from patients undergoing radical prostatectomy at Castle Hill Hospital (Cottingham, UK) with informed patient consent and approval from the NRES Committee Yorkshire & The Humber (LREC Number 07/H1304/121). Tissues were sampled immediately after surgery. For radical prostatectomies, three core needle biopsies were taken from four different sites (left base, left apex, right base, right apex) and were directed by previous pathology, imaging and palpation. Tissues were transported in RPMI-1640 with 5% FCS and 100U/ml antibiotic/antimitotic solution at 4°C and processed immediately upon arrival. PCa diagnosis was confirmed by histological examination of the whole prostate. Tissues were disaggregated as previously described[13], and all reagents were supplemented with 10 nM R1881 to better preserve the viability of luminal cells.

### Fluorescence activated cell sorting (FACS) and characterization of cell populations:

Single-cell suspensions were labelled with Lineage Cell Depletion Kit (human) and CD31 MicroBead Kit (Miltenyi Biotec) and $Lin^+/CD31^+$ cells depleted twice using MACS LS Columns (Miltenyi Biotec). $Lin^-/CD31^-$ cells were then labelled with EpCAM-APC, CD49f-FITC and CD24-PE (Miltenyi Biotec) and DAPI and $EpCAM^+/CD49f^+/CD24^-$ and $EpCAM^+/CD49f^-/CD24^+$ sorted at >95% purity using a MoFlo (Beckman Coulter) cell sorter. Sorted populations were characterized by immunofluorescence and qRT-PCR as previously described[18].

### Reduced Representation Bisulphite Sequencing (RRBS):

DNA was extracted from FACS-sorted populations using phenol/chloroform extraction and ethanol precipitation. DNA was quantified using a NanoDrop 1000 Spectrophotometer (Thermo Fisher Scientific) and shipped to Zymo Research for RRBS analysis. Bisulphite conversion, library preparation, sequencing, and initial bioinformatics analyses were performed by Zymo Research following the Methyl-MiniSeq pipeline.

### Sequence data processing and methylation calls

Fastq files were trimmed using Trim Galore! v0.4.1 (http://www.bioinformatics.babraham.ac.uk/projects/trim_galore/) with the following parameters: --fastqc --illumina --paired --rrbs --non_directional. Trimmed sequences were aligned to the human genome (hg19 downloaded from UCSC, 08-Mar-2009 version) using bsmap v2.90[14] and the following parameters: -m 0 -x 1000 -n 1 -p 8 -S 1. The resulting bam files were sorted and indexed using samtools v0.1.19[15], and methylation and coverage calls for each CpG site calculated using the methratio.py script in the bsmap software (Supplementary Table 1). Methylation calls were then filtered for low (<3) and high (>99.95%) read coverage and merged in non-overlapping genomic bins of 100 bp using the methylKit package v0.99.2[16] within R v3.3.1 to increase comparability between samples. All subsequent analyses were carried out using only those genomic bins covered in all samples, with the exception of the results presented in Supplementary Fig. 2 which were generated using single CpG information.

### Identification of differentially methylated regions (DMRs)

DMRs were calculated using methylKit[4]; with all pairwise comparisons between the four cell populations carried out and similar populations from different donors defined as biological replicates. The patient of origin was used as a categorical covariate to account for the strong inter-donor variability seen. All p-values were generated using a logistic regression model and corrected for multiple testing using the SLIM method[16].

DMRs were defined as those genomic bins with q-values <0.05 and absolute methylation difference >10% in each pairwise comparison.

### Characterization of DMRs

All genomic features were downloaded from the UCSC Table browser (genome.ucsc.edu) for the hg19 genome. Gene models: "refGene" (RefSeq Genes), CpG Islands: "cpgIslandExt", Evolutionary conservation: "phastCons100way", DNase hypersensitivity sites (DHSs): "wgEncodeRegDnaseClusteredV3", transcription factor binding sites (TFBSs): "wgEncodeRegTfbsClusteredV3", repeats: "rmsk" (RepeatMasker). Overlaps and distances of DMRs to other genomic features were calculated using BEDtools v2.26.0[17], and significance of enrichments or depletions was calculated using custom R scripts. All p-values $<10^{-300}$ were approximated to $10^{-300}$ to avoid reaching the minimum value for a floating-point number ($2.2*10^{-308}$). Average conservation signals around DMRs were calculated using bwtool v1.0[5]. P-values were calculated using a bootstrapping approach comparing the average conservation of the distal DMRs with the average of an equal number of randomly selected, non-overlapping, distal genomic bins, 1000 times. Gene ontology (GO) analysis was performed using GREAT v3.0[6], using all covered genomic bins as background and the default "Basal plus extension" association rules. Results were filtered to include only GO categories, with a Benjamini-Hochberg corrected (FDR) hypergeometric test p-value <0.05 and ≥3 genes with associated regions. K-means clustering of GO categories (biological processes only) was based on information similarity values calculated using the GOSim package within R v3.3.1. Promoters frequently altered in PCa were downloaded from the review by Massie et al., 2017[1]. Only promoters reported by ≥3 studies were considered frequently altered. Genome browser plots were generated using the package Sushi within R v3.3.1 and custom scripts.

**TCGA data analysis**

Illumina Infinium HumanMethylation450 data generated within the The Cancer Genome Atlas (TCGA) consortium[7] was downloaded (pre-processed Level 3 data only) from the NCI Genomic Data Commons website using the provided GDC Data Transfer Tool (data downloaded on 7th Dec 2016). Clinical data was downloaded from firebrowse.org (8th Dec 2016). The presence of evident batch effects was excluded by visualizing the data on TCGA Batch Effects (http://bioinformatics.mdanderson.org/tcgambatch/). A data matrix containing the beta values for each sample was generated using custom scripts.

Probes were mapped to hg19 using the positions officially reported by Illumina. Overlap of array probes with DMRs was carried out using BEDtools v2.26.0. Hierarchical clustering was based on Euclidean distances of unscaled beta-values. Logistic model training using least absolute shrinkage and selection operator (LASSO) regression was performed using the glmnet package within R v3.3.1 on a random selection of 70% of the samples. 200 lambda values ranging from $e^{-7}$ to $e^{-2}$ were tested and 10-fold cross validation performed. The lambda with the minimum mean cross-validated error was selected and resulted in 17 probes with non-zero coefficients. The optimal model was then tested on the remaining 30% of samples and receiver operator curve and area under the curve (AUC) calculated using the ROCR package.

**Calculation of probability of a sample containing prostate cancer (Model 1 - related to Table 1).**

$$p_{prostate\ cancer} = \frac{1}{1+\exp\left(-\left(sum\left(coefficients_{probe} * \beta val_{probe}\right)+23.4781567769938\right)\right)}$$

wherein

pprostate cancer = calculated probability of a single sample of being a prostate cancer as opposed to a normal prostate sample

*coefficients*$_{probe}$ = coefficients of the logistic models for each probe

$\beta val_{probe}$ OR $\beta val_{cgXXXXXXX}$ = beta values of each individual probe as determined by the Illumina Infinium HumanMethylation450K Bead Chip

**Calculation of probability of a sample containing prostate cancer (Model 2 - related to Table 2).**
wherein

pprostate cancer = calculated probability of a single sample of being a prostate cancer as opposed to a normal prostate sample

*coefficients*$_{probe}$ = coefficients of the logistic models for each probe

$\beta val_{probe}$ OR $\beta val_{cgXXXXXXX}$ = beta values of each individual probe as determined by the Illumina Infinium HumanMethylation450K Bead Chip

**Calculation of probability of a sample containing aggressive (extraprostatic) prostate cancer(Model 3 - related to Table 3).**
wherein

paggressive prostate cancer = calculated probability of a single sample of being a prostate cancer that has escaped the prostate (extraprostatic) as opposed to being still contained within the prostate capsule (organ confined)

$coefficients_{probe}$ = coefficients of the logistic models for each probe

$\beta val_{probe}$ OR $\beta val_{cgXXXXXXXX}$ = beta values of each individual probe as determined by the Illumina Infinium HumanMethylation450K Bead Chip

## Example 1

### Phenotypically defined prostate cells from patient-matched normal and PCa samples show donor-specific DNA methylation profiles

Matched tumour-directed (cancer) and contralateral (normal) core needle biopsies (1 or 2 per site) were obtained from 4 treatment-naive prostate cancer patients undergoing radical prostatectomies. These samples were then enzymatically dissociated and labeled with antibodies against EpCAM, CD49f and CD24 to enable the prospective isolation of luminal (EpCAM+CD49f-CD24+) and basal (EpCAM+CD49f+CD24-) cells at >95% purity (Fig. 1A). EpCAM+CD49f+CD24- cells expressed higher levels of molecular markers associated with basal cells and lower levels of luminal markers compared to EpCAM+CD49f-CD24+ cells from the same biopsy, both at the mRNA and protein level (Supplementary Fig. 1A-B). For convenience, we named the paired subsets as follows: Cancer Luminal (CL) EpCAM+CD49f-CD24+ cells purified from tumour-directed biopsies; Cancer Basal (CB) EpCAM+CD49f+CD24- cells purified from tumour-directed biopsies; Normal Luminal (NL) EpCAM+CD49f-CD24+ cells from contralateral biopsies; Normal Basal (NB) EpCAM+CD49f+CD24- cells purified from contralateral biopsies. This yielded 4 CL and CB populations, and 3 matched NL and NB populations, as in one prostate the palpable tumour was extended to most of the prostate and it was not possible to obtain a contralateral "normal" tissue biopsy (Supplementary Fig. 1C). DNA obtained from each of these isolates was then subjected to Reduced Representation Bisulphite Sequencing (RRBS). On average, this generated information on the DNA methylation status of >$8.9x10^6$ cytosines within CpG sites per sample (range $8x10^6$ - $9.6x10^6$, with an average coverage of 7.5 reads, Supplementary Table 1). The data was processed as described in Method and binned into 100 bp genomic regions to maximize the comparability between samples (932,905 bins covering $4.1x10^6$ CpGs in all samples). Unsupervised hierarchical clustering of the top 1% most variable regions (bins) across all samples showed clustering primarily according to the patient of origin, rather than the subset analyzed (Fig. 1B). This indicates a high donor-determined variation in CpG methylation, consistent with previous reports of similarly accrued data[8].

## Example 2

### Distinct DNA methylation profiles in basal and luminal cells

We then calculated DMRs for all pairwise comparisons between the 4 sorted populations (Fig. 1C). Among these, the comparison between CB and NB cells (CB-NB comparison) produced the smallest number of DMRs. In contrast, a large number of DMRs were seen when either normal or cancer luminal cells were compared with either source of basal cells (i.e., NL-NB, NL-CB, CL-NB and CL-CB, Fig. 1D). Of the DMRs revealed in these latter comparisons, -2/3 were hypermethylated in luminal cells, which correlates with the higher levels of DNMT3a seen in these cells[3]. We also calculated differential methylation on single CpGs (prior the 100bp binning) with very similar results Moreover, integration of the DMRs identified in NL-NB proximal (±5 kb) to annotated transcriptional start sites (TSSs) with RNA-seq data of similarly purified cells[2] showed the expected inverse correlation (Supplementary Fig. 3A).

We also found an extensive overlap in the DMRs obtained from both the NL-NB and NL-CB comparisons, and also from the CL-NB and CL-CB comparisons (Supplementary Fig. 3B-C). Accordingly, we focussed our subsequent analyses on comparisons of NL-NB and CL-CB, where cells from the same biopsy could be compared directly.

Characterization of the genomic features of the DMRs thus identified showed that >50% of them fell outside of CpG islands, shores or shelves (Fig. 1E), and >70% were >5 kb away from any annotated TSSs (Fig. 1F-G). These features were particularly pronounced (highly significant hypergeometric test) for the hypomethylated DMRs identified in the comparisons of NL-NB, CL-CB and CL-NL. Because hypermethylated and hypomethylated DMRs might be anticipated to differ in their genomic context, their impact on the biological properties of basal and luminal cells could also be different.

## Example 3

### Distal hypermethylated DMRs are enriched in enhancer features

Given that most of the DMRs identified were outside CpG islands and far from TSSs, we asked whether they might affect distal regulatory elements (enhancers). We therefore examined three genomic characteristics of such elements: evolutionary conservation[9], open chromatin shown by hypersensitivity to DNase I[18], and presence of TFBSs[10]. Distal hypermethylated DMRs in each comparison were enriched for evolutionarily conserved sequences (Fig. 2A, bootstrapped p-value) and overlapped significantly with both DHSs and ChIP-seq-defined TFBSs (identified within the ENCODE project, Fig. 2B-C, hypergeometric test). Distal hypomethylated DMRs generally scored lower than the hypermethylated counterparts for each metric measured. DMRs hypomethylated in the CL-CB and CL-NL comparisons showed the weakest enrichments. However, all distal hypomethylated DMRs had high overlaps with genomic repetitive elements (Fig. 2D). Specifically, LINE and LTR elements, but not SINE elements, were significantly enriched in the distal CL hypomethylated regions.

GO enrichment analysis (Fig. 2E, Supplementary Fig. 4) showed that hypermethylated DMRs in NL-NB were enriched for more than 500 terms, many of which were linked to prostate development or epithelial stem cell regulation; while hypomethylated DMRs in the same comparison were enriched for terms related to androgen receptor signalling and response to cytokines. In the CL-CB comparison, hypermethylated DMRs were also enriched for more than 500 terms, 311 of which were also identified in the NL-NB comparison, suggesting a high functional overlap in hypermethylated regions in luminal cells from both normal and cancer samples. In the CL-NL comparison, hypermethylated DMRs were enriched in terms related to cell adhesion, while hypomethylated DMRs were enriched in terms related to epithelial morphogenesis. These results indicate that several pathways fundamental to the establishment and maintenance of the normal prostate epithelium are altered in cancer cells with a luminal phenotype.

## Example 4

### Phenotype-specific DMRs are shared in normal and cancerous prostate tissues

As suggested by the enriched GO analyses, we found a 28% overlap in all the DMRs identified from the NL-NB and the CL-CB comparisons (3852/13816, Fisher's exact test p-value < $10^{-300}$, Fig. 3A). Hierarchical clustering of all samples based on both sets of DMRs separated them by phenotype (Fig. 3B), reinforcing the presence of a strong phenotypic signature independent of disease state. These shared DMRs were enriched in features characteristic of enhancers (Supplementary Fig. 5A-D) and linked to GO terms related to prostate development, regulation of epithelial stem cells and androgen receptor signalling (Supplementary Fig. 5E-F). Moreover, hypermethylated DMRs were highly enriched for TFBSs of *TP63, TP53* and *NF1,* and hypomethylated DMRs for *FOXA1, p65-NFkB* and *GATA3* (Fig. 3C), all well-known regulators of basal and luminal epithelial cells, respectively. Interestingly, 26 of the 168 genes described as frequently differentially methylated in PCa[1], showed hyper- or hypomethylated DMRs within 5 kb of their TSSs in both the NL-NB and CL-CB comparisons (Fig. 3D). These included the frequently hypermethylated genes, *GSTP1* and *CCDC8* (Fig. 3E-F).

In summary, these analyses identified a large set of phenotype-specific and disease-independent DMRs, both of which contained many binding sites for TFs with known regulatory roles in the normal prostate.

## Example 5

### CL hypermethylate PRC2 target sites and hypomethylate repetitive elements

A second group of genes frequently hypermethylated in PCa were found hypermethylated in both the CL-CB and CL-NL comparisons (Fig. 4a), but not in the NL-NB comparison. These might be expected to reflect a PCa-specific methylation signature. DMRs identified in the CL-CB and CL-NL comparisons showed that many were shared (1472 DMRs, Fisher's exact test p-value < $10^{-300}$, Fig. 4B) with very few also different between NL and NB cells (106 DMRs). 65% of these CL-specific hypermethylated DMRs were distal to TSSs and were again highly enriched for enhancer features, but significantly depleted in repetitive elements (Supplementary Fig. 6A-E). These regions were associated with GO terms related to metabolic processes, cell proliferation and epithelial development (Fig. 4C) and showed a high enrichment of DNA sequences potentially bound by EZH2 and SUZ12, two main members of the PRC2 complex (Supplementary Fig. 6F). On the other hand, distal hypomethylated DMRs were not enriched for any feature of putative regulatory regions, but significantly overlapped with LINE and LTR elements.

Since the CL subset represents the majority of the cells in untreated PCa samples, we hypothesized that aberrant methylation of these DMRs would be measurable even when whole tissue homogenates are analysed. We therefore interrogated the DNA methylation array dataset for PCa made available by the TCGA consortium, which consists of 50 PCa samples with matched normal counterparts, 452 additional PCa samples without normal counterparts, and 1 metastatic PCa sample[7]. 255 array probes overlap these 1472 DMRs. Hierarchical clustering of the 50 matched normal and PCa samples showed an almost perfect subdivision based on the malignancy status of the samples (TPR = 0.92, TNR = 0.92, Chi-squared test p-value = $2.4 \times 10^{-16}$, Fig. 4D). The same analysis carried out on all 553 samples produced similar results, with one cluster highly enriched in normal samples (Chi-squared test p-value = $1.7 \times 10^{-39}$, Supplementary Fig. 6G). This clustering also appeared to divide the PCa samples into two main groups, according to their differences from the normal samples. Exclusive analysis of the cancer samples confirmed this clustering pattern (Fig. 4E) and showed one cluster to be significantly enriched for samples with extra-prostatic extensions (pT3 or pT4 in TNM classification, Chi-squared test p-value < 0.005) in the absence of significant differences in Gleason score (Chi-squared test p-value >0.1).

Overall, these results indicate that phenotypic luminal PCa cells possess an aberrant methylation signature characterized by hypermethylation of putative regulatory sequences involved in tissue development, and hypomethylation of LINEs and LTRs repetitive elements. This signature was also able to distinguish cancer samples from normal, and organ-confined from extra-prostatic disease.

### Example 6

### Identification of PCa-specific, phenotype-independent DMRs

Comparisons of the DMRs in the CL-NL and CB-NB pairs showed a small but significant overlap of both hyper- and hypomethylated DMRs in each (189 DMRs in total, Fig. 5A). These common DMRs were able to cluster all samples according to their disease state in a phenotype-independent manner (Supplementary Fig. 7A). Notably, they included DMRs close to many genes previously implicated in prostate cancer (e.g., *NEAT1, MTOR, RHCG, KCNC2, WT1, HOXC12, KMT2B,* Fig. 5B). To determine whether these DMRs would be altered in an independent dataset, we applied the same analysis to the TCGA dataset, where 66 array probes overlapped these 189 DMRs. Hierarchical clustering of the 50 matched normal and PCa samples produced a single cluster containing 46/50 normal samples and 10/50 PCa samples (TPR = 0.8, TNR = 0.92, Chi-squared test p-value = $1.8 \times 10^{-12}$, Fig. 5C). Application of the same analysis to all samples in the TCGA database produced similar results: one cluster was highly enriched in normal samples (TPR = 0.87, TNR = 0.74, Chi-squared test p-value = $8.3 \times 10^{-26}$, Supplementary Fig. 7B), indicating that at least some of these DMRs are frequently altered in PCa.

To select the probes most strongly associated with disease state (i.e., PCa vs normal), we trained a logistic model using LASSO regression on 70% of the TCGA samples and selected a 17-probe signature (Fig. 5D). We then tested this model on the remaining 30% of the dataset. This resulted in an AUC of 0.92 (TPR = 0.9, TNR = 0.94, Fisher's exact test p-value = $2.82 \times 10^{-12}$ at the selected cut-off of 0.8, Fig. 5E-F, Table 1. The 17-probe signature also included sequences proximal to several genes with recognized importance in PCa (e.g., *PLAGL1/HYMAI, HOXC12, KCNC2),* but was completely non-overlapping with other similar signatures recently developed for PCa[19-21 11-12].

### Example 7

To identify another two relevant signatures indicative of prostate cancer or aggressiveness of cancer respectively, we again trained logistic models using LASSO regression on the 255 array probes of the TCGA dataset overlappping the 1472 DMRs found in the comparisons CL-NL and CL-CB (Figure 4).

We trained a first model using a random selection of 70% of all samples categorized as normal or PCa, and selected the 8 probes most associated with disease state. We then tested this model on the remaining 30% of the samples. This resulted in an AUC of 0.96 (TPR = 0.93, TNR = 1, Fisher's exact test p-value = $1.06 \times 10^{-12}$ at the selected cut-off of 0.78, Fig. 6A-C).

We trained a second model using a random selection of 70% of only the PCa samples categorized into organ confined or extraprostatic. This classification was derived from the official TNM classification given by TCGA: Extraprostatic = pT3 or pT4 / Organ Confined = pT2. The LASSO regression returned an optimal model based on 23 probes most associated with disease aggressiveness. We then tested this model on the remaining 30% of the samples. This resulted in an AUC of 0.74 (TPR = 0.64, TNR = 0.78, Fisher's exact test p-value = $4.23 \times 10^{-7}$ at the selected cut-off of 0.63, Fig. 6D-F).

### References

1. Massie CE, Mills IG, Lynch AG. The importance of DNA methylation in prostate cancer development. J Steroid Biochem Mol Biol. 2017 Feb;166:1-15.
2. Zhang D, Park D, Zhong Y, Lu Y, Rycaj K, Gong S, et al. Stem cell and neurogenic gene-expression profiles link prostate basal cells to aggressive prostate cancer. Nat Commun. 2016 Feb 29;7:10798.
3. Pellacani D, Kestoras D, Droop A, Frame FM, Berry PA, Lawrence MG, et al. DNA hypermethylation in prostate cancer is a consequence of aberrant epithelial differentiation and hyperproliferation. Cell Death Differ. 2014 May;21(5):761-73.
13. Frame FM, Pellacani D, Collins AT, Maitland NJ. Harvesting Human Prostate Tissue Material and Culturing Primary Prostate Epithelial Cells. Methods Mol Biol. 2016;1443:181-201.4. Akalin A, Kormaksson M, Li S, Garrett-Bakelman FE, Figueroa ME, Melnick A, et al. methylKit: a comprehensive R package for the analysis of genome-wide DNA methylation profiles. Genome Biol. 2012 Oct 3;13(10):R87.
5. Pohl A, Beato M. bwtool: a tool for bigWig files. Bioinformatics. 2014 Jun 1;30(11):1618-9.
6. McLean CY, Bristor D, Hiller M, Clarke SL, Schaar BT, Lowe CB, et al. GREAT improves functional interpretation of cis-regulatory regions. Nat Biotechnol. 2010 May;28(5):495-501.
7. Cancer Genome Atlas Research Network. The Molecular Taxonomy of Primary Prostate Cancer. 2015 Nov 5;163(4):1011-25.
8. Yu YP, Ding Y, Chen R, Liao SG, Ren B-G, Michalopoulos A, et al. Whole-genome methylation sequencing reveals distinct impact of differential methylations on gene transcription in prostate cancer. Am J Pathol. 2013 Dec;183(6):1960-70.
9. Heintzman ND, Hon GC, Hawkins RD, Kheradpour P, Stark A, Harp LF, et al. Histone modifications at human enhancers reflect global cell-type-specific gene expression. Nature. 2009 May 7;459(7243):108-12.
10. Heinz S, Romanoski CE, Benner C, Glass CK. The selection and function of cell type-specific enhancers. Nat Rev Mol Cell Biol. 2015 Mar;16(3):144-54.
11. Mundbjerg K, Chopra S, Alemozaffar M, Duymich C, Lakshminarasimhan R, Nichols PW, et al. Identifying aggressive prostate cancer foci using a DNA methylation classifier. Genome Biol. BioMed Central; 2017 Jan 12;18(1):3.
12. Tang Y, Jiang S, Gu Y, Li W, Mo Z, Huang Y, et al. Promoter DNA methylation analysis reveals a combined diagnosis of CpG-based biomarker for prostate cancer. Oncotarget. Impact Journals; 2017 Aug 29;8(35):58199-209.
13. Frame FM, Pellacani D, Collins AT, Maitland NJ. Harvesting Human Prostate Tissue Material and Culturing Primary Prostate Epithelial Cells. Methods Mol Biol. 2016;1443:181-20
14. Xi Y, Li W. BSMAP: whole genome bisulfite sequence MAPping program. BMC Bioinformatics. 2009;10:232.
15. Li H, Handsaker B, Wysoker A, Fennell T, Ruan J, Homer N, et al. The Sequence Alignment/Map format and SAMtools. Bioinformatics. 2009 Aug 15;25(16):2078-9.
16. Wang H-Q, Tuominen LK, Tsai C-J. SLIM: a sliding linear model for estimating the proportion of true null hypotheses in datasets with dependence structures. Bioinformatics. 2011 Jan 15;27(2):225-31.
17. Quinlan AR, Hall IM. BEDTools: a flexible suite of utilities for comparing genomic features. Bioinformatics. 2010 Mar 15;26(6):841-2.
18. Thurman RE, Rynes E, Humbert R, Vierstra J, Maurano MT, Haugen E, et al. The accessible chromatin landscape of the human genome. Nature. 2012 Aug 29;489(7414):75-82
19. Zhao S, Geybels MS, Leonardson A, Rubicz R, Kolb S, Yan Q, et al. Epigenome-Wide Tumor DNA Methylation Profiling Identifies Novel Prognostic Biomarkers of Metastatic-Lethal Progression in Men Diagnosed with Clinically Localized Prostate Cancer. Clin Cancer Res. American Association for Cancer Research; 2017 Jan 1;23(1):311-9.
20. Geybels MS, Zhao S, Wong C-J, Bibikova M, Klotzle B, Wu M, et al. Epigenomic profiling of DNA methylation in paired prostate cancer versus adjacent benign tissue. Prostate. 2015 Dec;75(16):1941-50.
21. Geybels MS, Wright JL, Bibikova M, Klotzle B, Fan J-B, Zhao S, et al. Epigenetic signature of Gleason score and prostate cancer recurrence after radical prostatectomy. Clin Epigenetics. BioMed Central; 2016;8(1):97.

## Claims

**1.** A diagnostic method to determine the methylation status of genomic regions isolated from a human subject that has prostate cancer comprising the steps:

i) obtaining an isolated biological sample from a subject and extracting genomic DNA to provide an isolated sample of genomic DNA;
ii) determining the methylation status of one or more CpG dinucleotides of said genomic DNA consisting of (a) the nucleotide sequence set forth in SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47 and SEQ ID NO: 48 and wherein said region can be extended 250bp upstream or downstream of said region, or (b) a polymorphic sequence variant that has at least 90% sequence identity over the full length nucleotide sequences set forth in SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47 or SEQ ID NO: 48; wherein said region can be extended 250bp upstream or downstream of said region,

as a measure of whether the prostate cancer is aggressive or non-aggressive.

**2.** The method of claim 1 wherein said genomic DNA region is hypermethylated in one or more CpG dinucleotides in genomic regions set forth in SEQ ID NO: 26, SEQ ID NO: 28, SEQ ID NO: 29, SEQ ID NO: 31, SEQ ID NO: 33, SEQ ID NO: 34, SEQ ID NO: 36, SEQ ID NO: 37, SEQ ID NO: 38, SEQ ID NO: 40, SEQ ID NO: 42, SEQ ID NO: 46 and SEQ ID NO: 48.

**3.** The method of claim 1 wherein said genomic DNA region is hypomethylated in one or more CpG dinucleotides in genomic regions set forth in SEQ ID NO: 27, SEQ ID NO: 30, SEQ ID NO: 32, SEQ ID NO: 35, SEQ ID NO: 39, SEQ ID NO: 41, SEQ ID NO: 43, SEQ ID NO: 44, SEQ ID NO: 45 and SEQ ID NO: 47.

**4.** A diagnostic method to determine the methylation status of genomic DNA regions isolated from a human subject that is suspected of having prostate cancer comprising the steps:

i) obtaining an isolated biological sample from a subject and extracting genomic DNA to provide an isolated sample of genomic DNA;
ii) determining the methylation status of one or more CpG dinucleotides of said genomic DNA comprising or consisting of (a) a genomic nucleotide sequence set forth SEQ ID NO: 18, SEQ ID NO: 19, SEQ ID NO: 20, SEQ ID NO: 21, SEQ ID NO: 22, SEQ ID NO: 23, SEQ ID NO: 24 and SEQ ID NO: 25, and wherein said region can be extended 250bp upstream or downstream of said region or (b) a polymorphic sequence variant that has at least 90% sequence identity over the full length nucleotide sequences set forth in SEQ ID NO: 18, SEQ ID NO: 19, SEQ ID NO: 20, SEQ ID NO: 21, SEQ ID NO: 22, SEQ ID NO: 23, SEQ ID NO: 24 and SEQ ID NO: 25 and, wherein said region can be extended 250bp upstream or downstream of said region as a measure of the likelihood said subject has prostate cancer.

**5.** The method of claim 4 wherein said genomic DNA region is hypermethylated in one or more CpG dinucleotides in genomic regions set forth in SEQ ID NO: 18, SEQ ID NO: 19, SEQ ID NO: 20, SEQ ID NO: 21, SEQ ID NO: 22, SEQ ID NO: 23 and SEQ ID NO: 25.

**6.** The method of claim 4 wherein said genomic DNA region is hypomethylated in one or more CpG dinucleotides in a genomic region as set forth in SEQ ID NO: 24.

**7.** A diagnostic method to determine the methylation status of genomic DNA regions isolated from a human subject that is suspected of having prostate cancer comprising the steps:

i) obtaining an isolated biological sample from a subject and extracting genomic DNA to provide an isolated sample of genomic DNA;
ii) determining the methylation status of one or more CpG dinucleotides of said genomic DNA comprising or consisting of a (a) genomic nucleotide sequence set forth in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16 and SEQ ID NO: 17 and wherein said region can be extended 250bp upstream or downstream of said region, or (b) a polymorphic sequence variant that has at least 90% sequence identity over the full length nucleotide sequences set forth in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16 and SEQ ID NO: 17 and wherein said region can be extended 250bp upstream or downstream of said region, as a measure of the likelihood said subject has prostate cancer.

**8.** The method of claim 7 wherein said genomic DNA region is hypermethylated in one or more CpG dinucleotides in genomic regions as set forth in SEQ ID NO: 3, SEQ ID NO: 4, SEQ ID NO: 5, SEQ ID NO: 8, SEQ ID NO: 9, SEQ ID NO: 11, SEQ ID NO: 14, SEQ ID NO: 16 and SEQ ID NO: 17, or alternatively wherein said genomic DNA region is hypomethylated in one or more CpG dinucleotides in genomic regions as set forth in SEQ ID NO: 1, SEQ ID NO: 2, SEQ ID NO: 6, SEQ ID NO: 7, SEQ ID NO: 10, SEQ ID NO: 12, SEQ ID NO: 13 and SEQ ID NO: 15.

**9.** A diagnostic method to determine the methylation status of genomic regions of a human subject having prostate cancer comprising the steps:

i) obtaining a biological sample from a subject and extracting genomic DNA to provide an isolated sample comprising genomic DNA;
ii) determining the methylation status of genomic DNA by interrogating the methylation status associated with the probes cg00066748, cg01285435, cg03122624, cg03731646, cg04301614, cg04804717, cg05977462, cg06740893, cg07076509, cg09433131, cg12010198, cg15830431, cg17737967, cg18020065, cg18825594, cg20595634, cg20811788, cg21155609, cg21265647, cg21914290, cg22755142, cg23986375, cg24348240;

as a measure of whether the prostate cancer is aggressive or non-aggressive.

**10.** A diagnostic method to determine the methylation status of one or more genomic regions of a human subject that is suspected of having prostate cancer comprising the steps:

i) obtaining a biological sample from a subject and extracting genomic DNA to provide an isolated sample comprising genomic DNA;
ii) determining the methylation status of genomic DNA by interrogating the methylation status associated with the probes cg00066748, cg08376310, cg15192750, cg15267232, cg17124583, cg19125791, cg23044391, cg26459372, as a measure of the likelihood said subject has prostate cancer.

**11.** A diagnostic method to determine the methylation status of genomic regions of a human subject that is suspected of having prostate cancer comprising the steps:

iii) obtaining a biological sample from a subject and extracting genomic DNA to provide an isolated sample comprising genomic DNA;
iv) determining the methylation status of genomic DNA by interrogating the methylation status associated with the probes cg02523640, cg02315096, cg06563089, cg04527018, cg10116893, cg06729806, cg22333412, cg03293976, cg03356806, cg13233461, cg24876897, cg01153451, cg14859324, cg10007452, cg09541000, cg15628253 and cg21745537, as a measure of the likelihood that said subject has prostate cancer.

**12.** A diagnostic method for determining if a subject has aggressive or non-aggressive prostate cancer comprising:

i) providing an isolated biological sample to be tested and preparing cDNA;
ii) forming a preparation comprising said cDNA and an oligonucleotide primer pairs adapted to anneal to a nucleic acid molecule comprising a nucleotide sequence encoding: TDRP, VPS28, EFEMP1, LOC10013165, C4orf19, UNCX, CUX2, MIR4632, BARX1, KCNB2, ZNF251, STK3, MAN2B1, RASA3, KLF11, NKX6-2, MAPK11, FAM167B, SLITRK1, PRRX1, MYCNOS, C8orf31 and SH3BGRL3, a thermostable DNA polymerase, deoxynucleotide triphosphates and co-factors;
v) providing polymerase chain reaction conditions sufficient to amplify all or part of said nucleic acid molecule(s);
vi) analyzing the amplified products of said polymerase chain reaction for the presence and/or level of said nucleic acid molecule encoding said polypeptide as a measure of gene expression; and
vii) comparing the amplified product with a normal matched control.

**13.** A diagnostic method for determining if a subject has or is susceptible to prostate cancer comprising:

i) providing an isolated biological sample to be tested and preparing cDNA;
ii) forming a preparation comprising said cDNA and an oligonucleotide primer pairs adapted to anneal to a nucleic acid molecule comprising a nucleotide sequence encoding TDRP, KCNQ1DN, CLEC18A, GATA3, MIR2467, MIR6792 and ADCY1, a thermostable DNA polymerase, deoxynucleotide triphosphates and co-factors;
iii) providing polymerase chain reaction conditions sufficient to amplify all or part of said nucleic acid molecule(s);
iv) analyzing the amplified products of said polymerase chain reaction for the presence and/or level of said nucleic acid molecule encoding said polypeptide as a measure of gene expression; and
v) comparing the amplified product with a normal matched control.

**14.** A diagnostic method for determining if a subject has or is susceptible to prostate cancer comprising:

i) providing an isolated biological sample to be tested and preparing cDNA;
ii) forming a preparation comprising said cDNA and an oligonucleotide primer pairs adapted to anneal to a nucleic acid molecule comprising a nucleotide sequence encoding FOXI2, IRS2, KCNC2, C3orf22, LOC10192826, HOXC12, VWA5B1, P2RY1, GLUD2, DDOST, BRF1, GABRB3, PLAGL1, L3MBTL1 and SC5D, a thermostable DNA polymerase, deoxynucleotide triphosphates and cofactors;
iii) providing polymerase chain reaction conditions sufficient to amplify all or part of said nucleic acid molecule(s);
iv) analyzing the amplified products of said polymerase chain reaction for the presence and/or level of said nucleic acid molecule encoding said polypeptide as a measure of gene expression; and
v) comparing the amplified product with a normal matched control.

**15.** The diagnostic method according to any one of claims 1 to 14 wherein said biological sample is selected from the group consisting of: urine, seminal fluid, blood, lymph fluid, or prostate tissue., optionally wherein the isolated biological sample is a tissue biopsy, optionally wherein said biopsy is a prostate tissue biopsy.

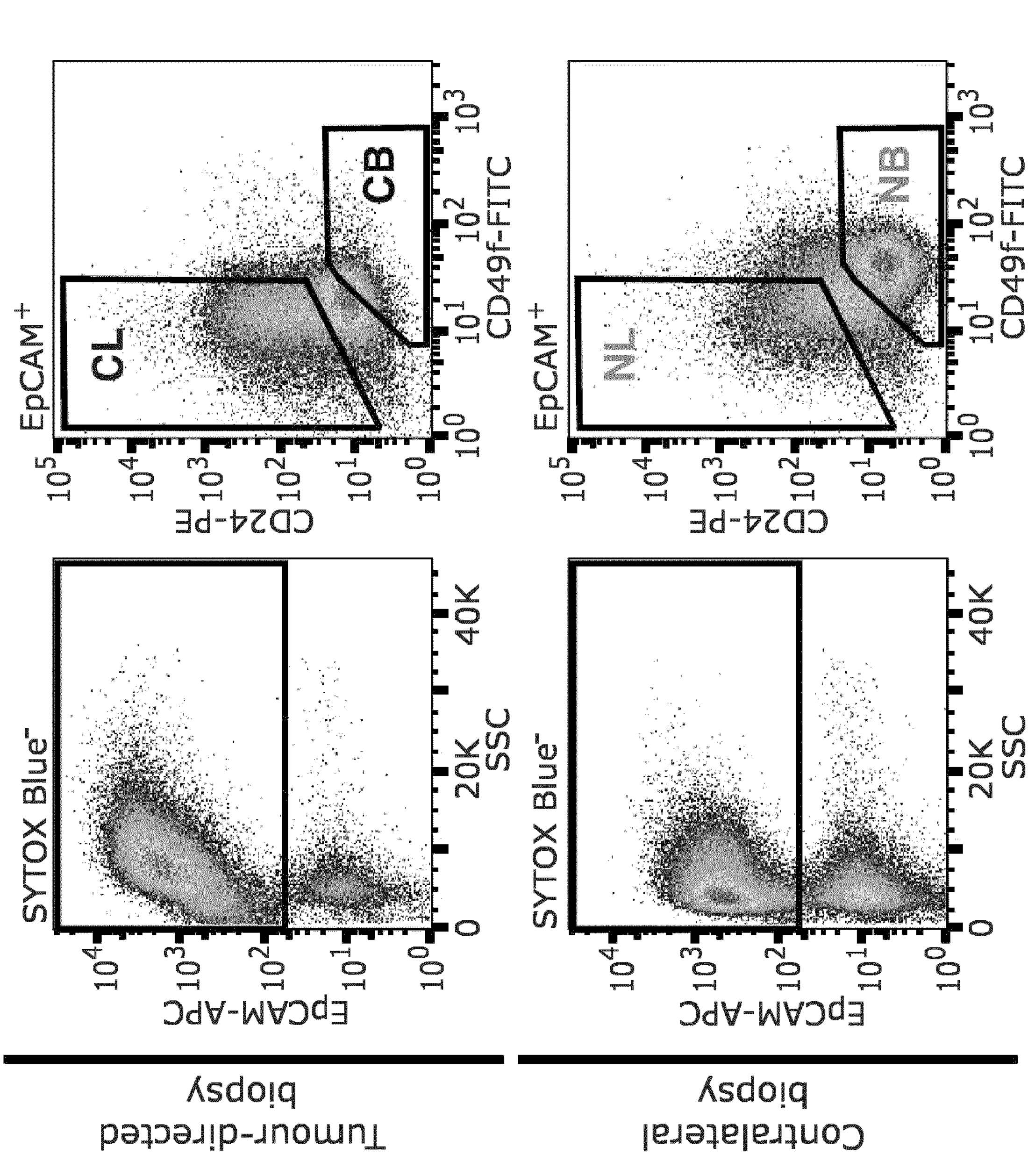
Tumour-directed biopsy
Contralateral biopsy
SYTOX Blue⁻
EpCAM-APC
SSC
EpCAM⁺
CD24-PE
CD49f-FITC
CL
CB
NL
NB

Figure 1A

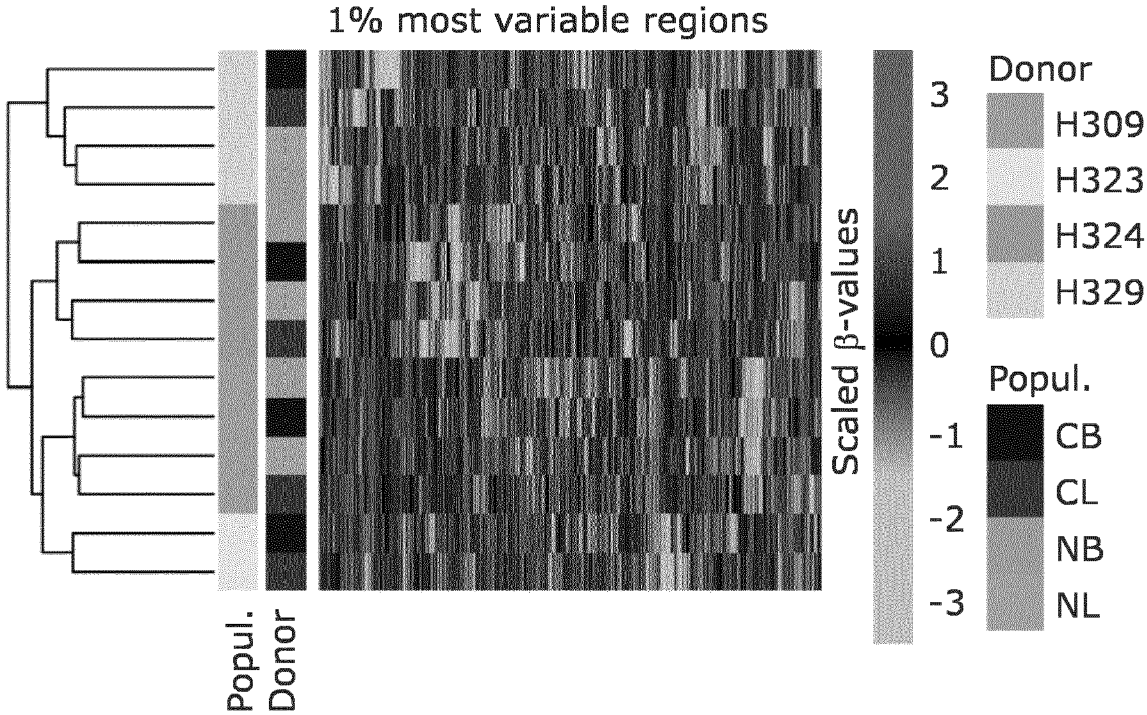


Figure 1B

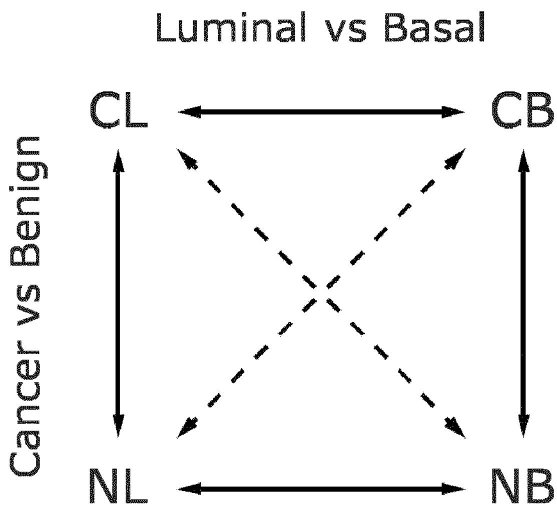


Figure 1C

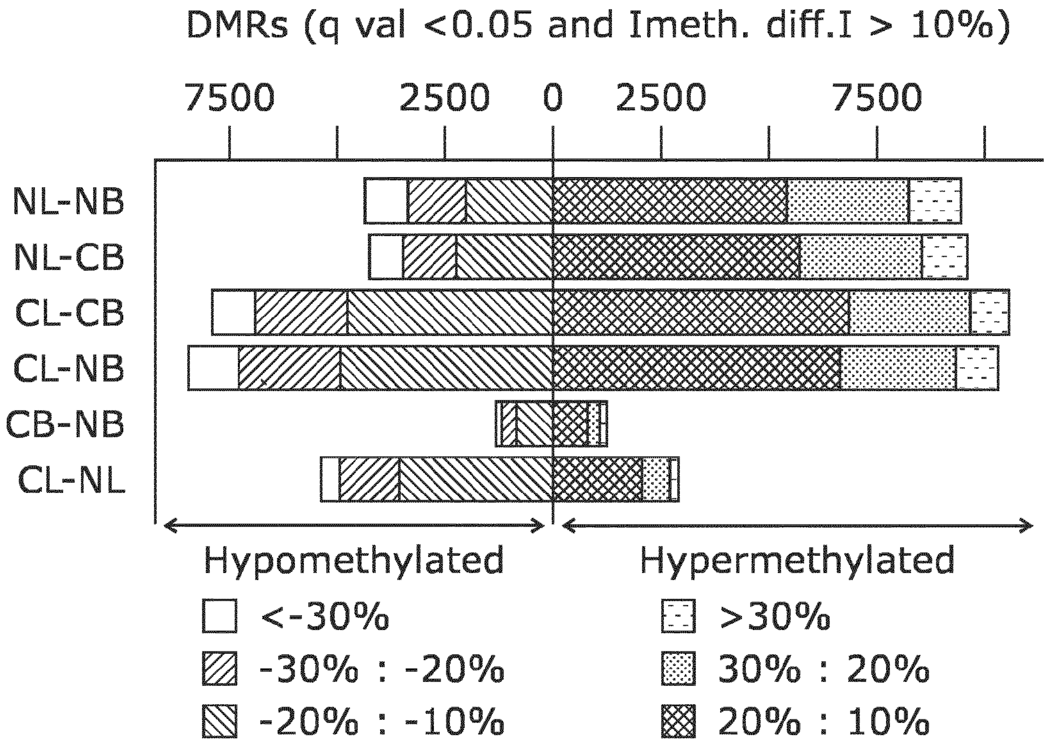


Figure 1D

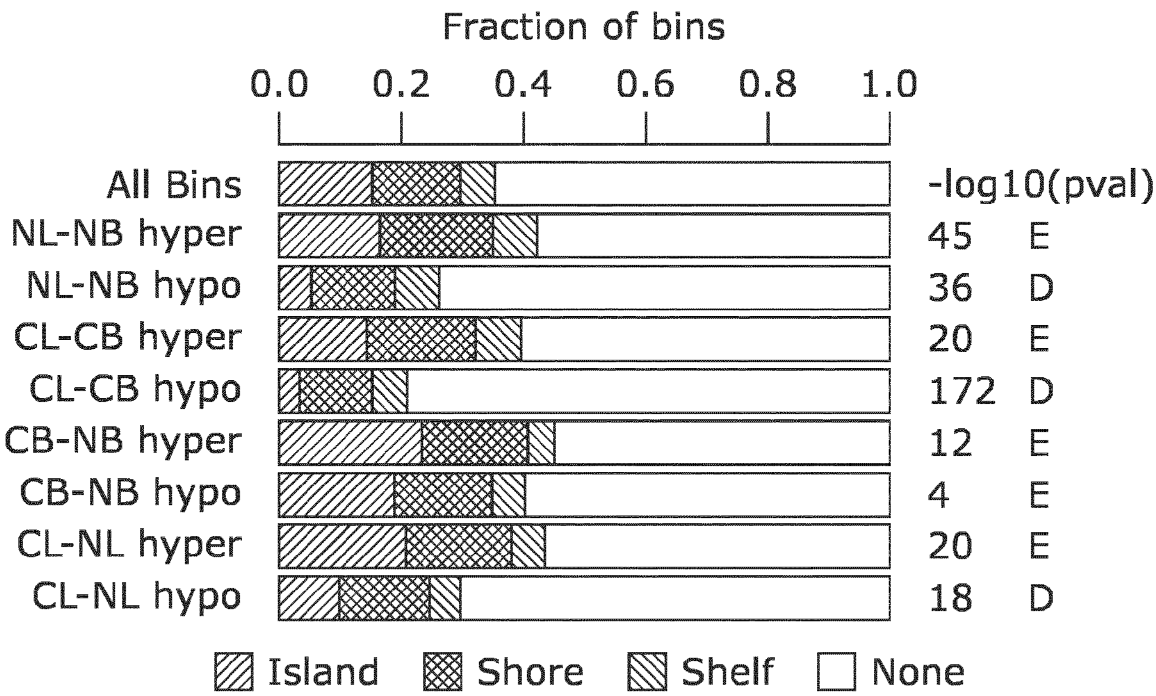


Figure 1E

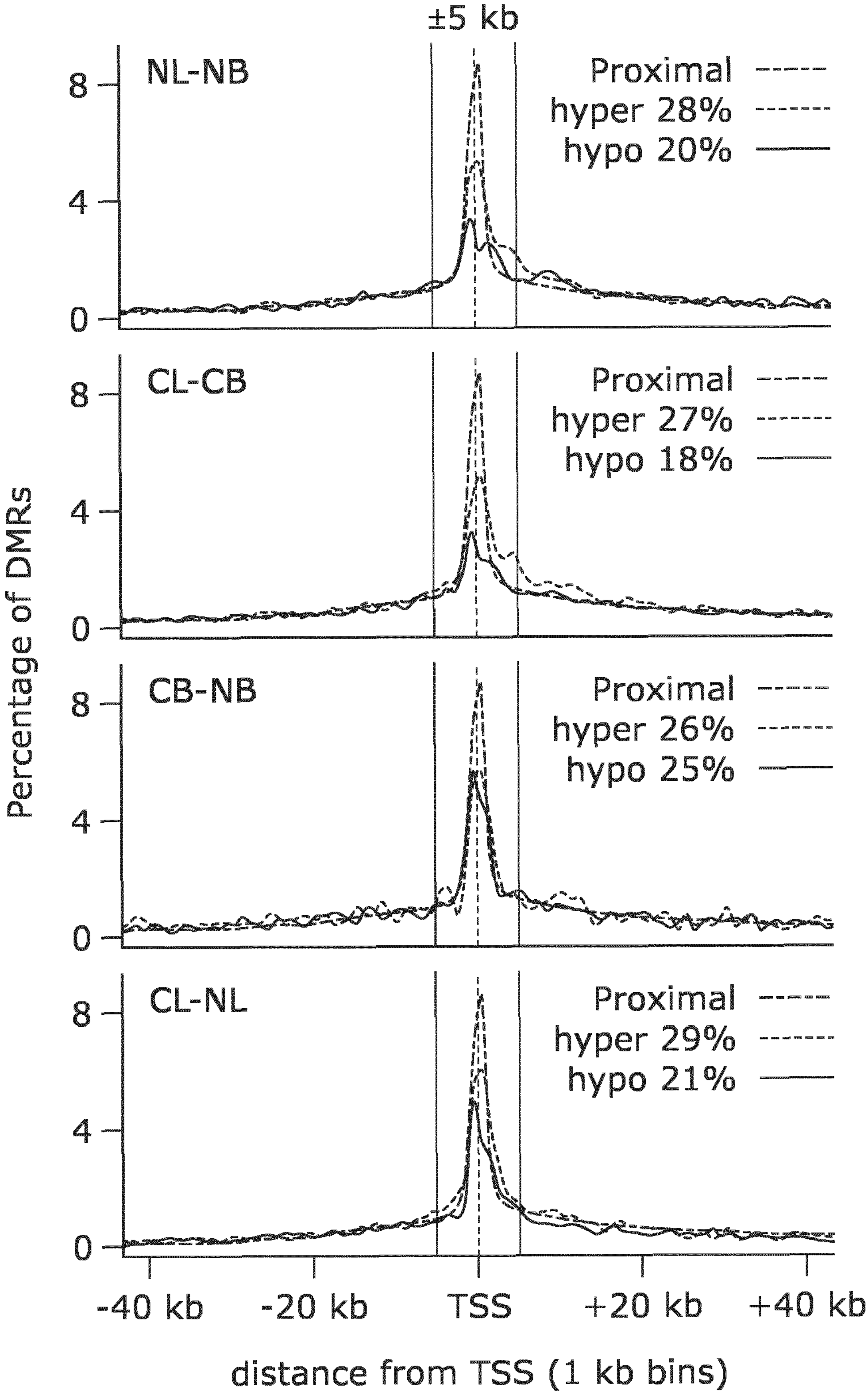
±5 kb
NL-NB
Proximal
hyper 28%
hypo 20%
CL-CB
Proximal
hyper 27%
hypo 18%
CB-NB
Proximal
hyper 26%
hypo 25%
CL-NL
Proximal
hyper 29%
hypo 21%
Percentage of DMRs
8
4
0
-40 kb
-20 kb
TSS
+20 kb
+40 kb
distance from TSS (1 kb bins)

Figure 1F

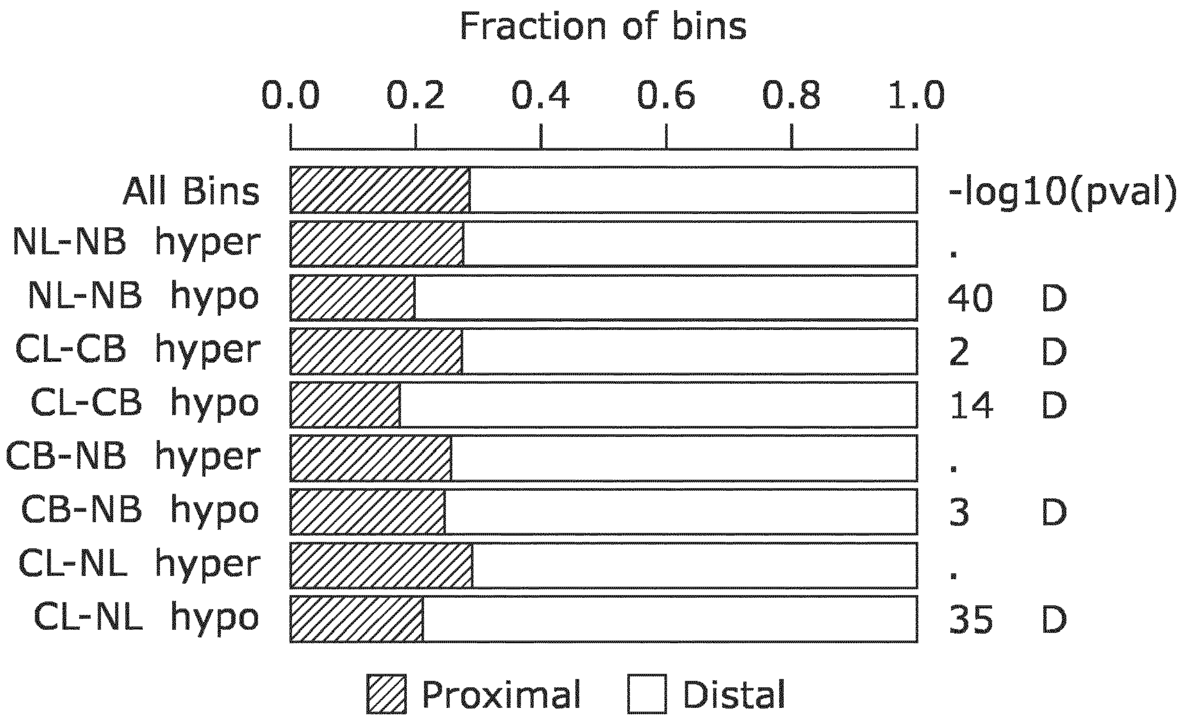
Fraction of bins
0.0
0.2
0.4
0.6
0.8
1.0
All Bins
NL-NB hyper
NL-NB hypo
CL-CB hyper
CL-CB hypo
CB-NB hyper
CB-NB hypo
CL-NL hyper
CL-NL hypo
-log10(pval)
.
40 D
2 D
14 D
.
3 D
.
35 D
Proximal
Distal

Figure 1G

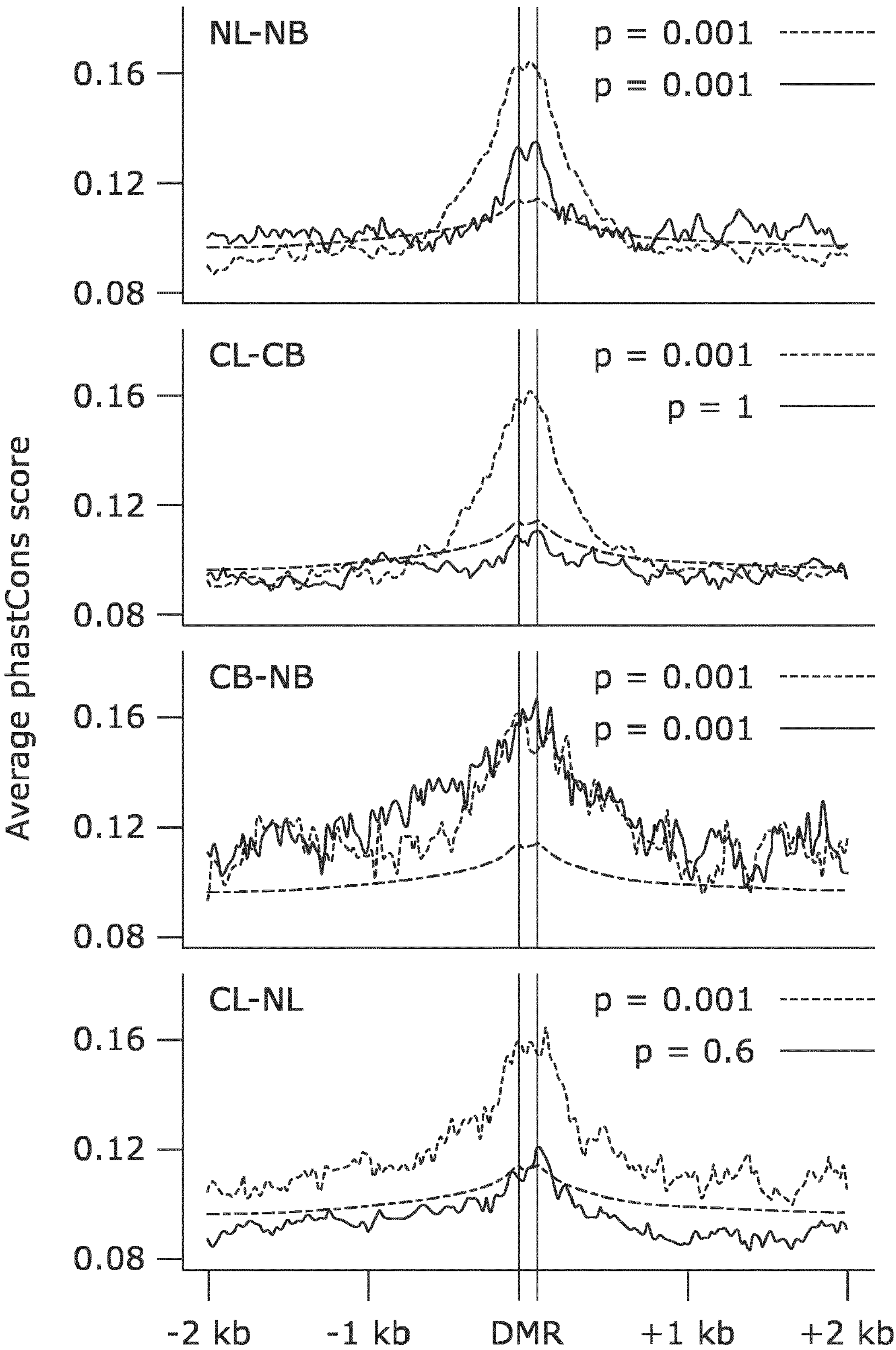
NL-NB
p = 0.001
p = 0.001
CL-CB
p = 0.001
p = 1
CB-NB
p = 0.001
p = 0.001
CL-NL
p = 0.001
p = 0.6
0.16
0.12
0.08
Average phastCons score
-2 kb
-1 kb
DMR
+1 kb
+2 kb

Figure 2A

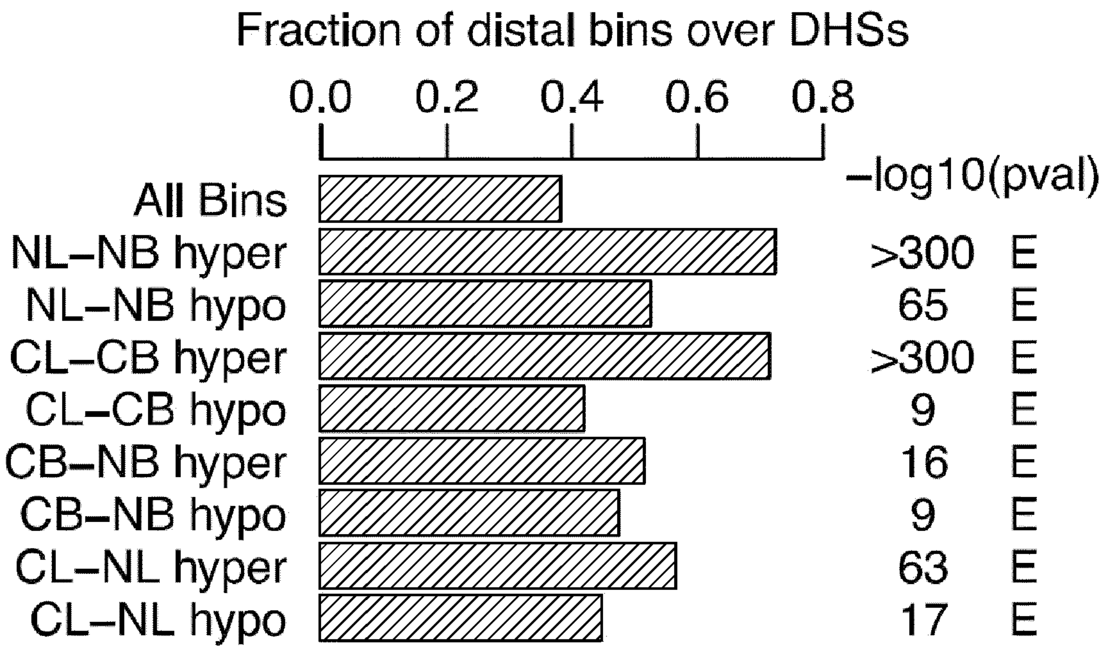


Figure 2B

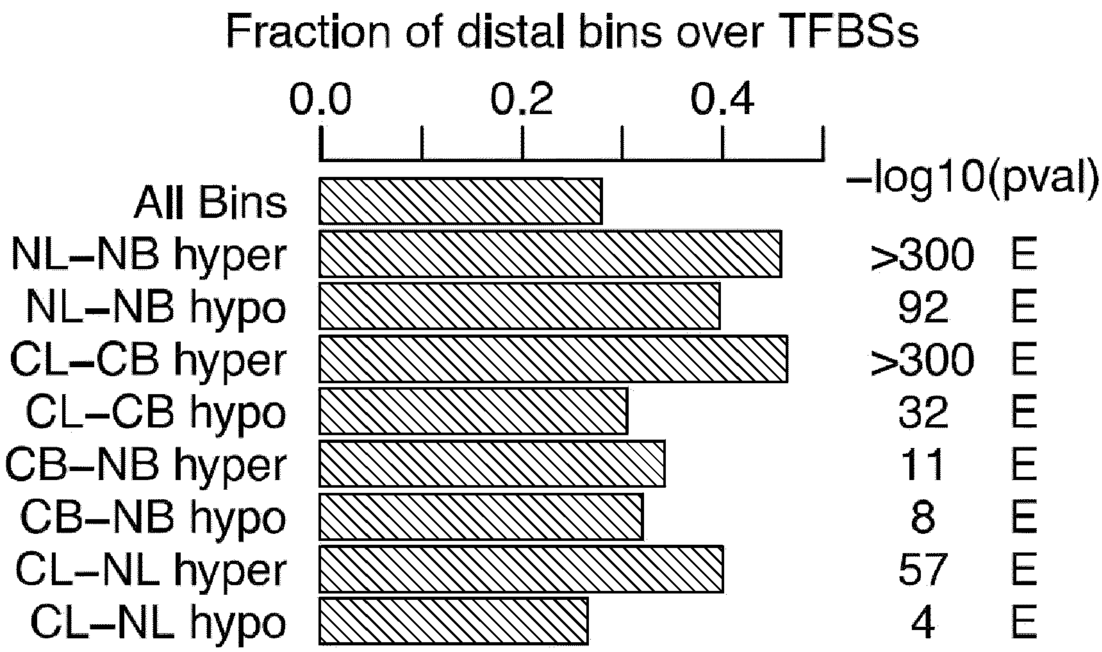


Figure 2C

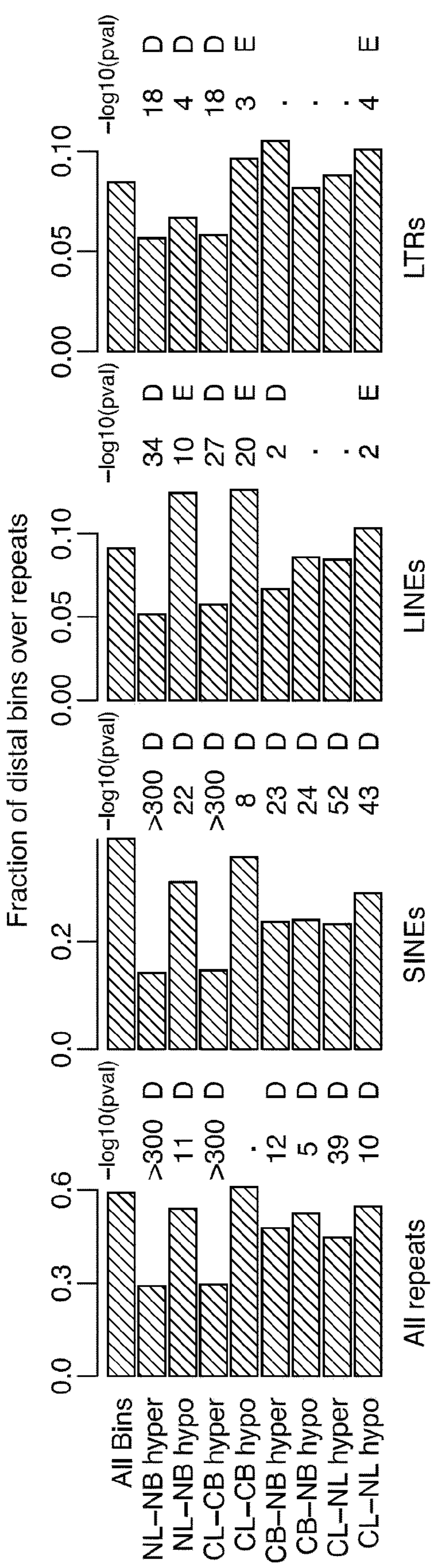


Figure 2D

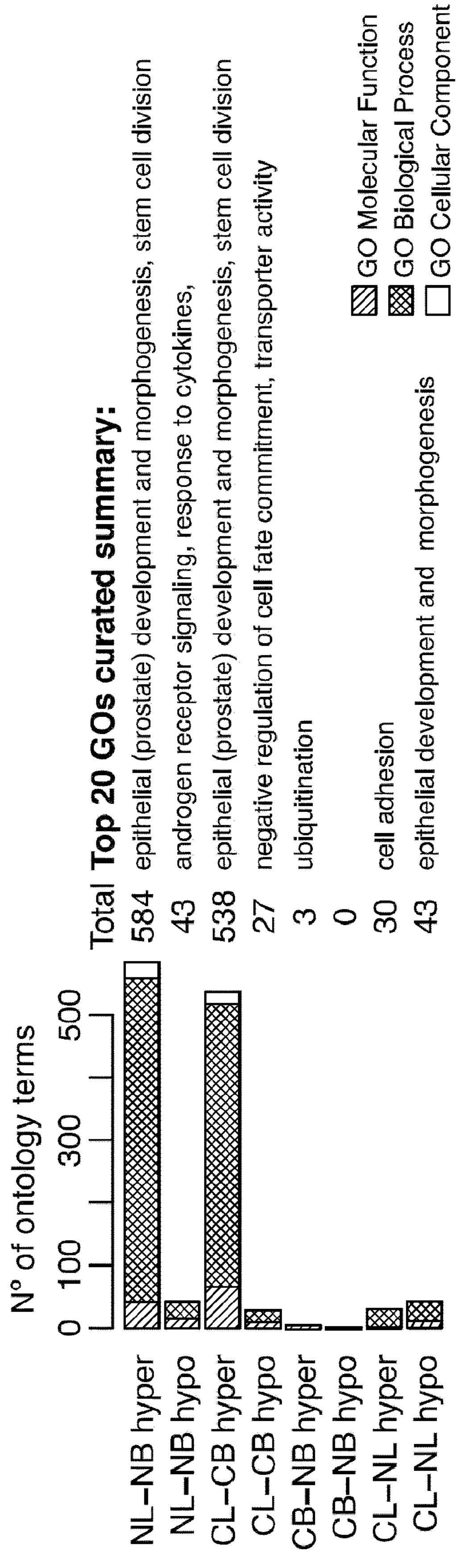


Figure 2E

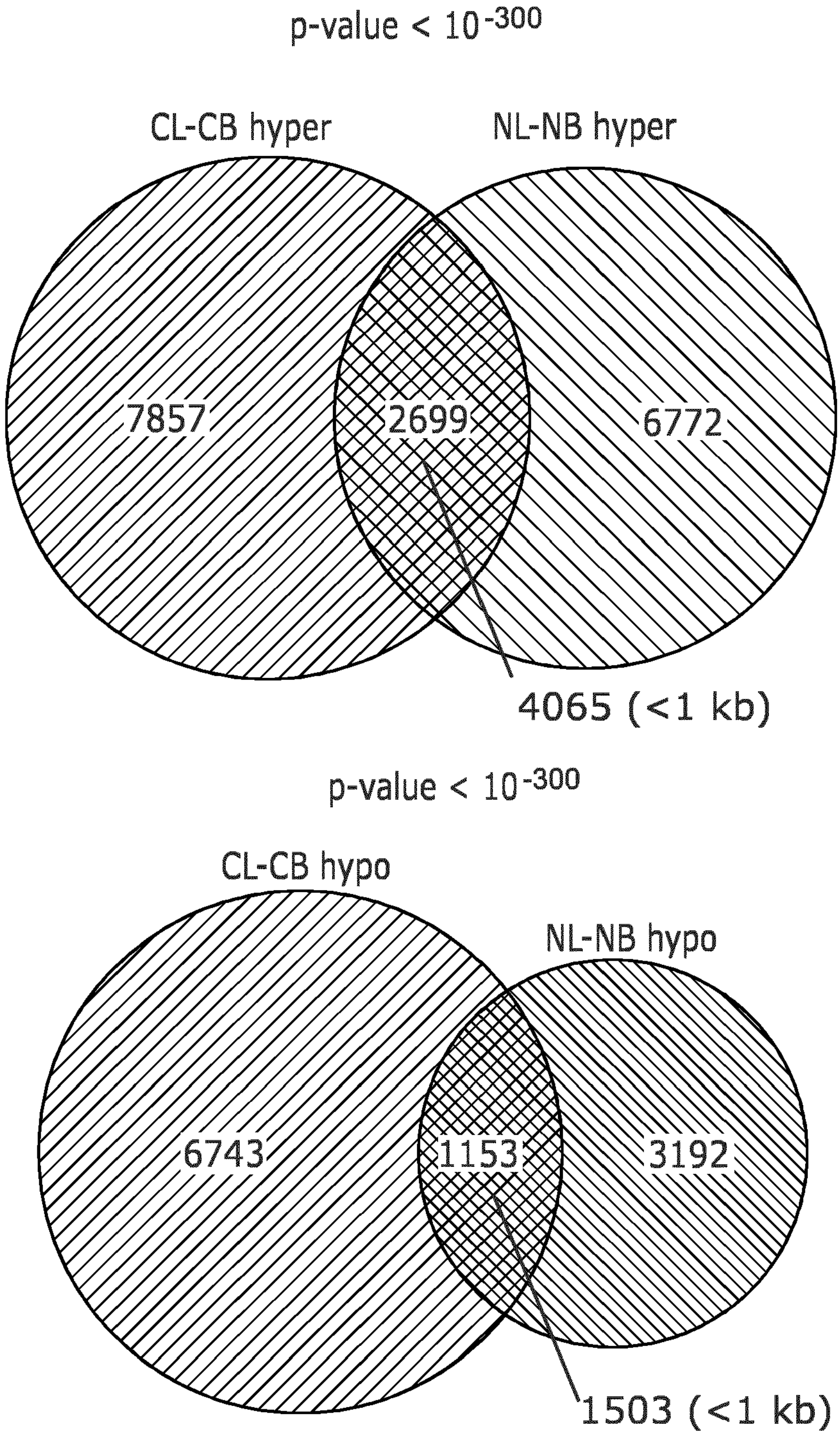
p-value < 10-300
CL-CB hyper
NL-NB hyper
7857
2699
6772
4065 (<1 kb)
p-value < 10-300
CL-CB hypo
NL-NB hypo
6743
1153
3192
1503 (<1 kb)

Figure 3A

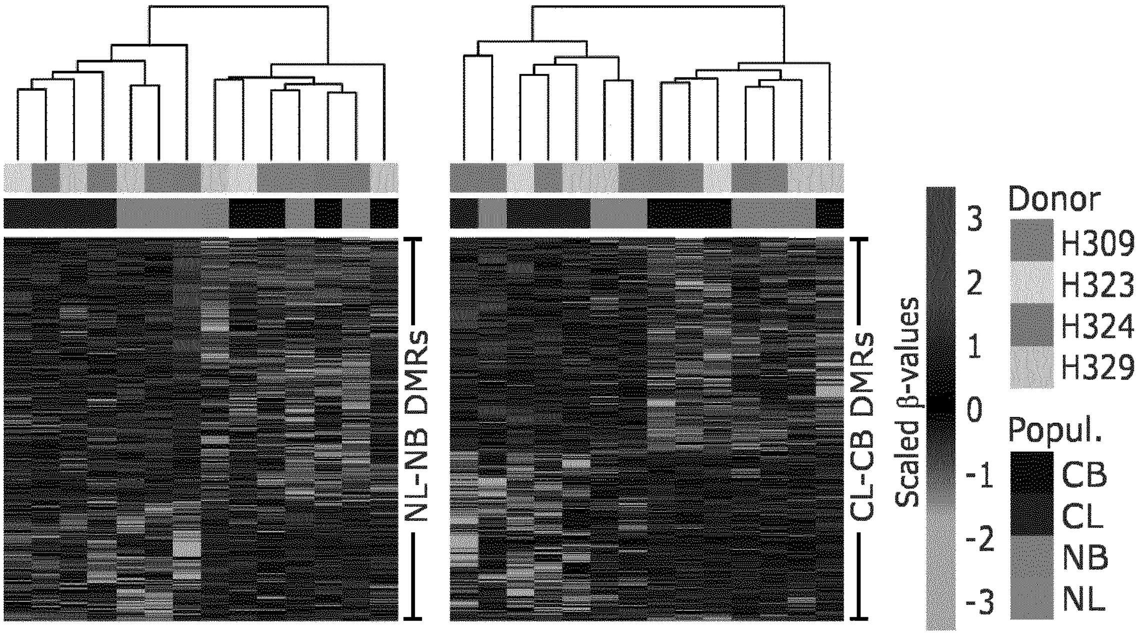


Figure 3B

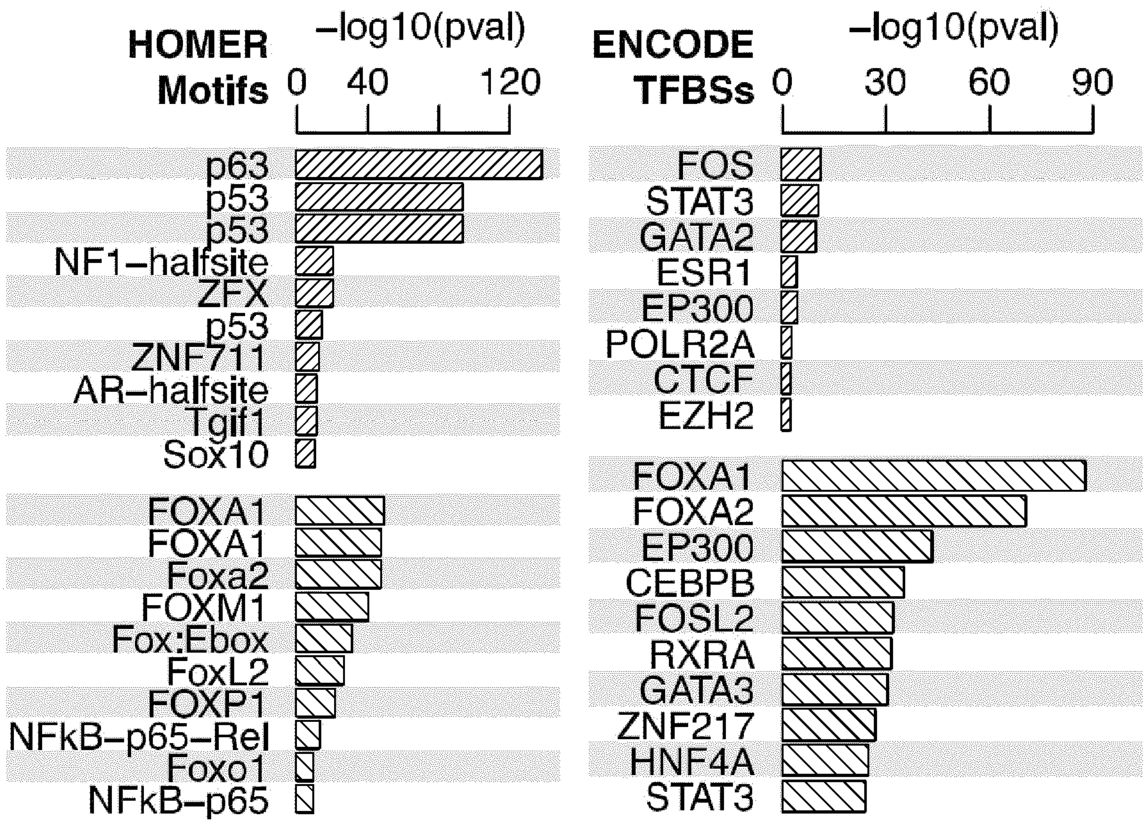


Figure 3C

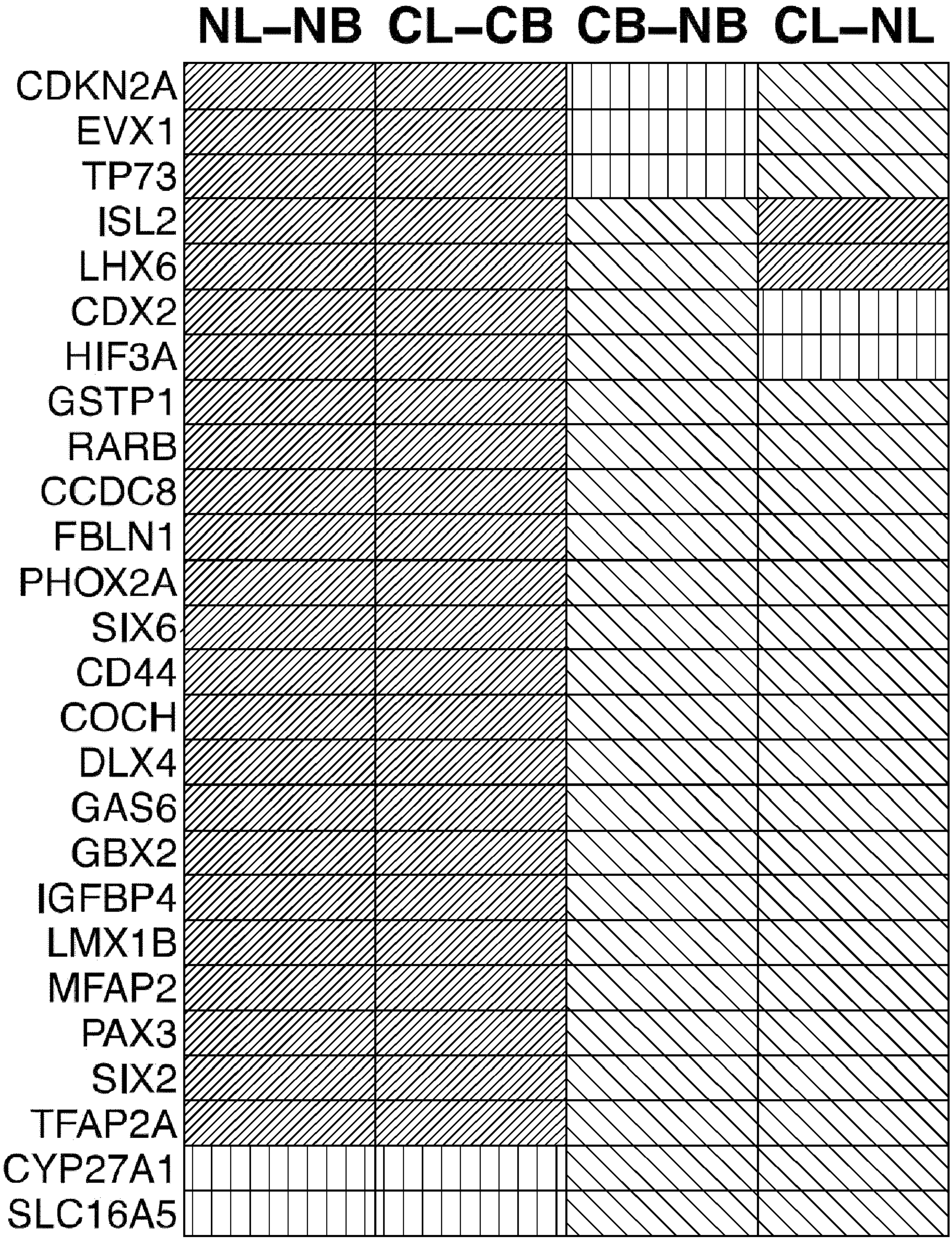
NL–NB
CL–CB
CB–NB
CL–NL
CDKN2A
EVX1
TP73
ISL2
LHX6
CDX2
HIF3A
GSTP1
RARB
CCDC8
FBLN1
PHOX2A
SIX6
CD44
COCH
DLX4
GAS6
GBX2
IGFBP4
LMX1B
MFAP2
PAX3
SIX2
TFAP2A
CYP27A1
SLC16A5

Figure 3D

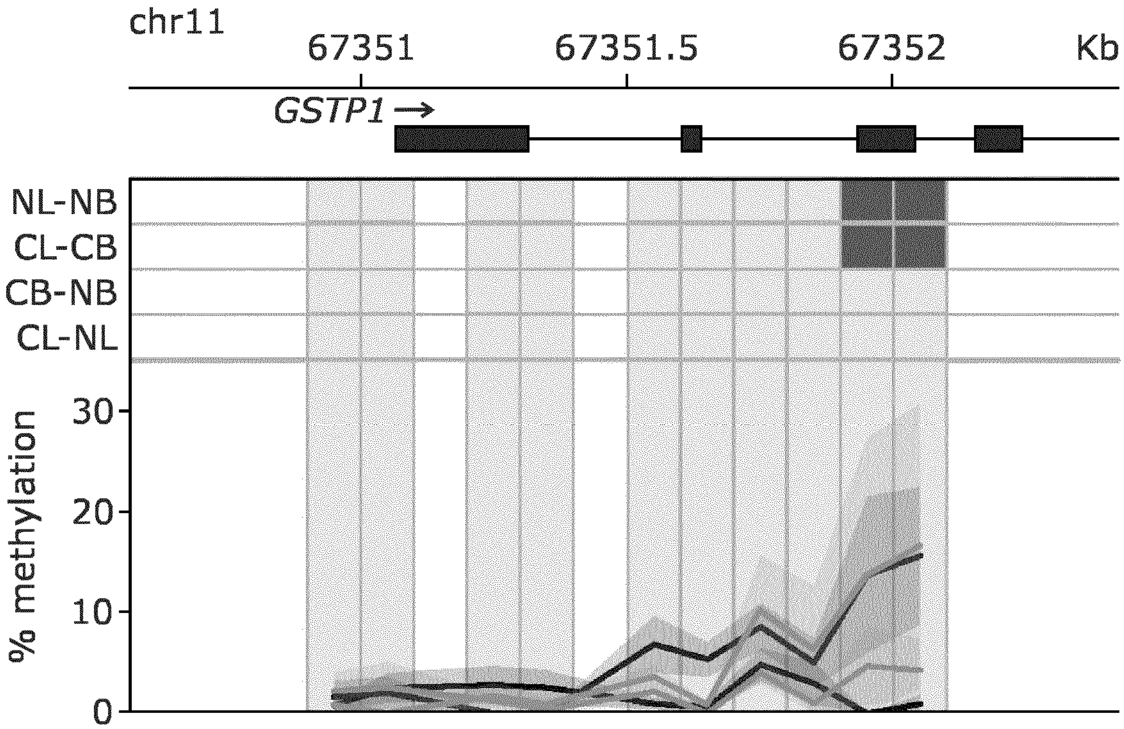


Figure 3E

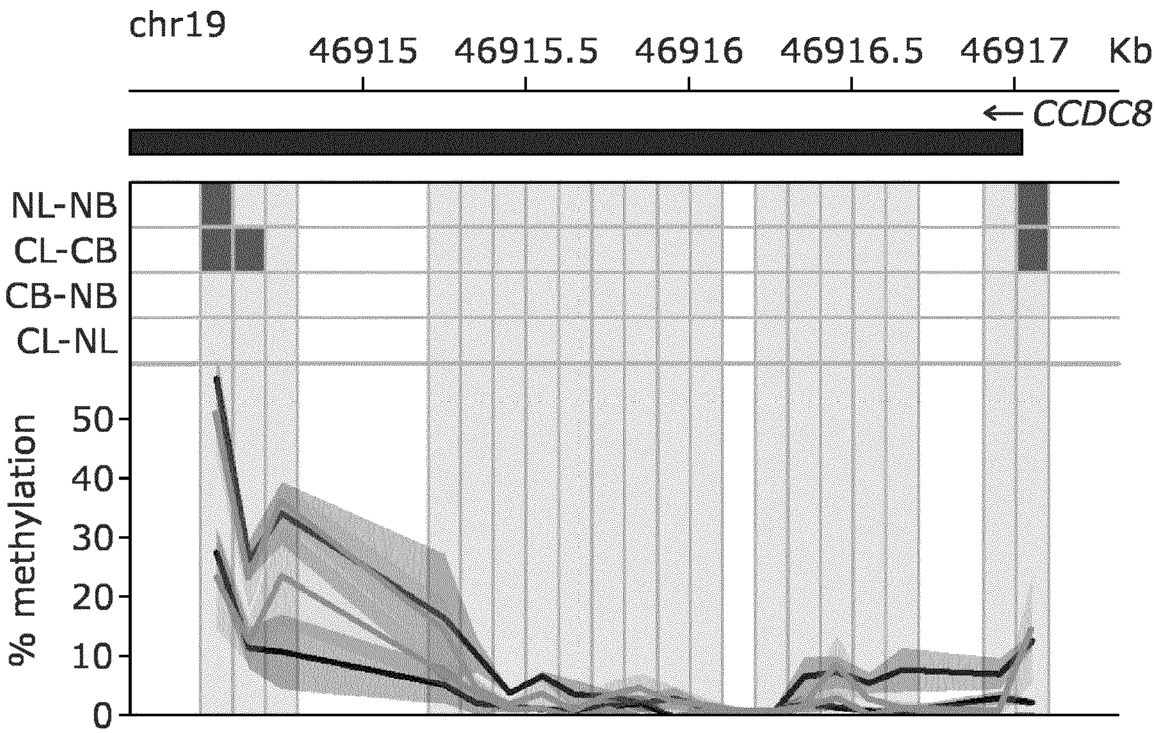


Figure 3F

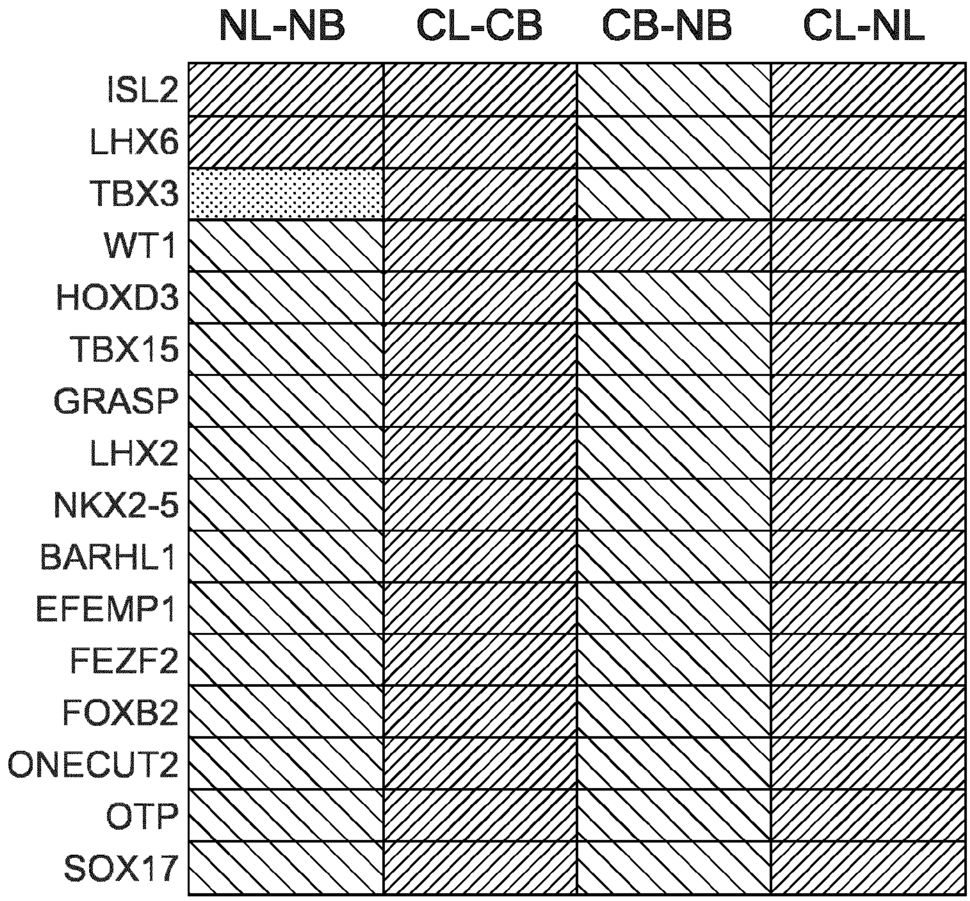


Figure 4A

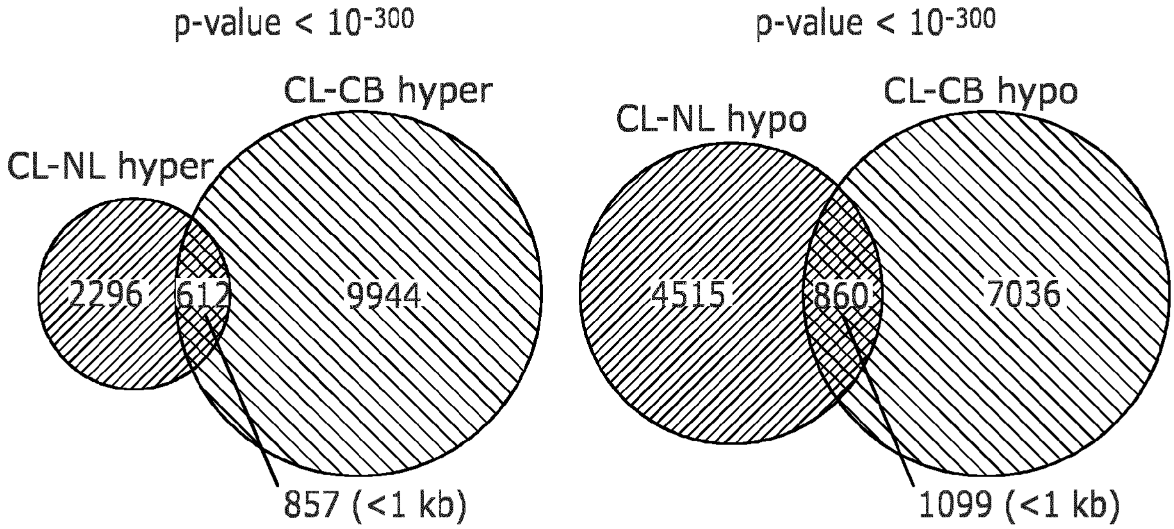


Figure 4B

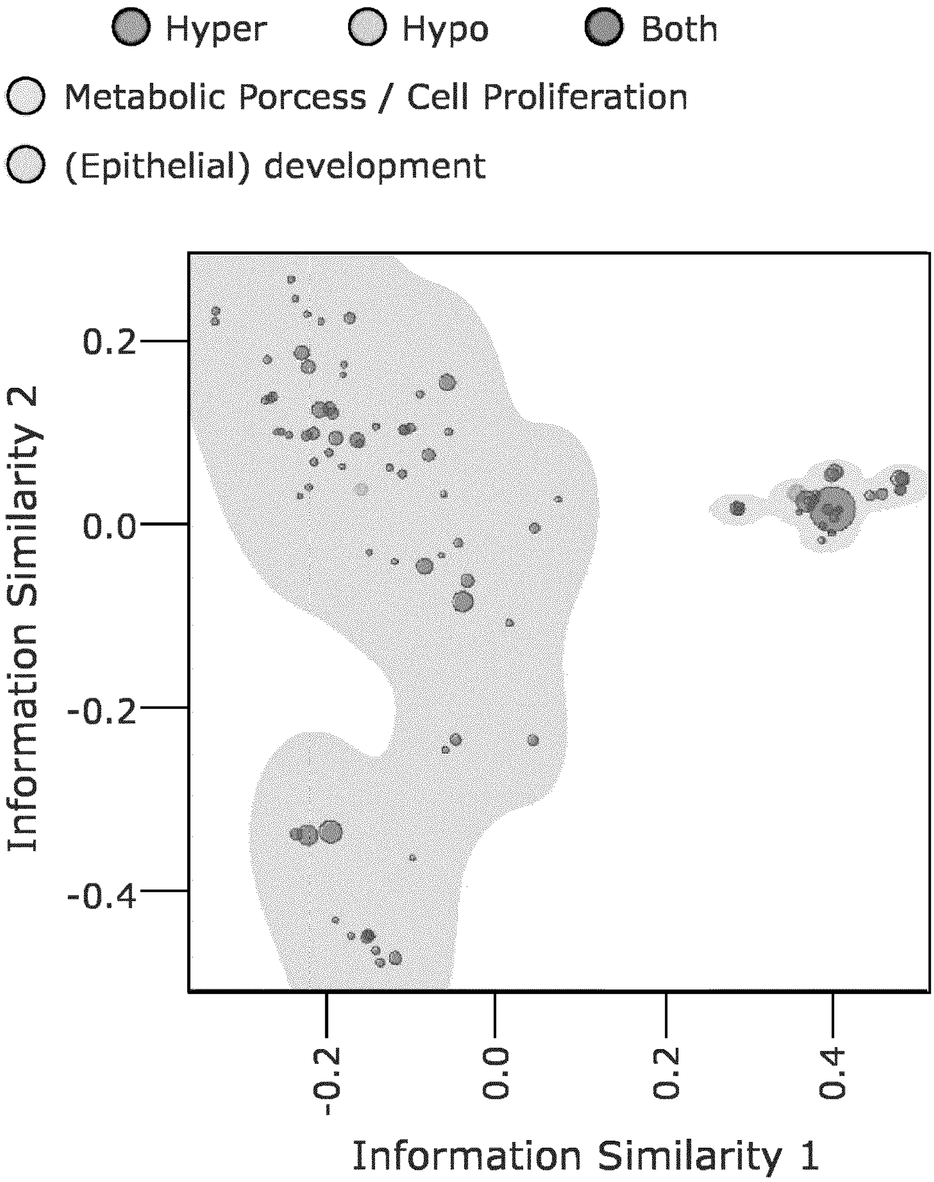
Hyper
Hypo
Both
Metabolic Porcess / Cell Proliferation
(Epithelial) development
0.2
0.0
-0.2
-0.4
Information Similarity 2
-0.2
0.0
0.2
0.4
Information Similarity 1

Figure 4C

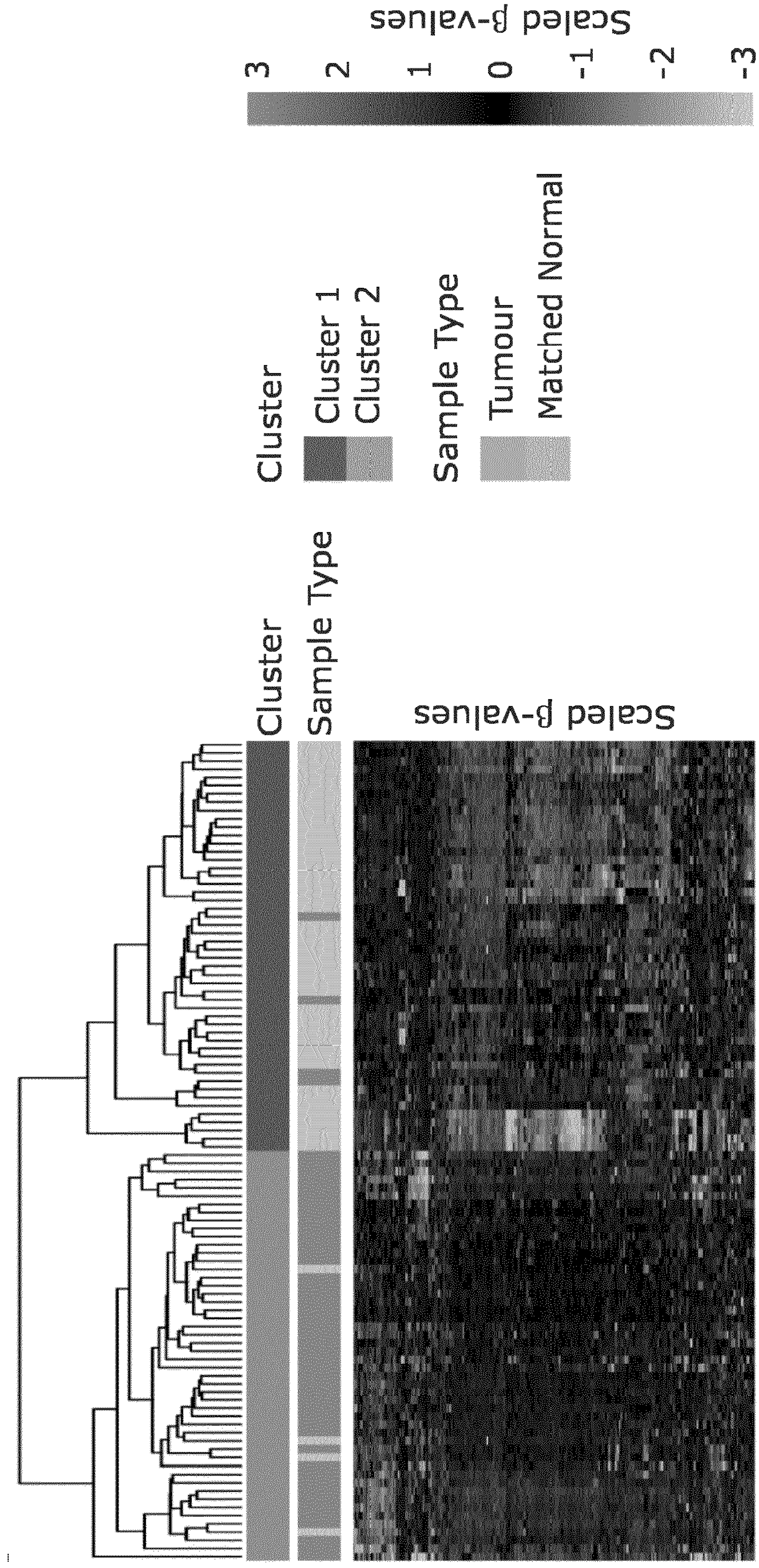
Scaled β-values
3
2
1
0
-1
-2
-3
Cluster
Cluster 1
Cluster 2
Sample Type
Tumour
Matched Normal
Cluster
Sample Type
Scaled β-values

Figure 4D

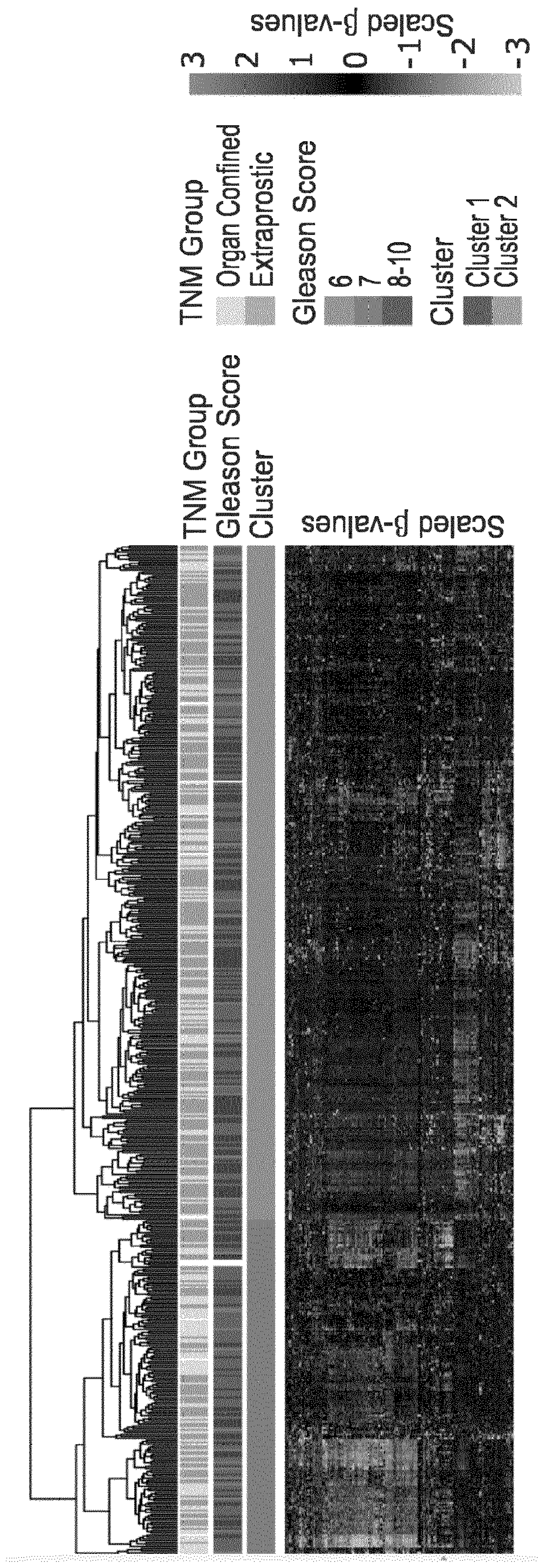
TNM Group
Gleason Score
Cluster
Scaled β-values
TNM Group
Organ Confined
Extraprostic
Gleason Score
6
7
8-10
Cluster
Cluster 1
Cluster 2
Scaled β-values
3
2
1
0
-1
-2
-3

Figure 4E

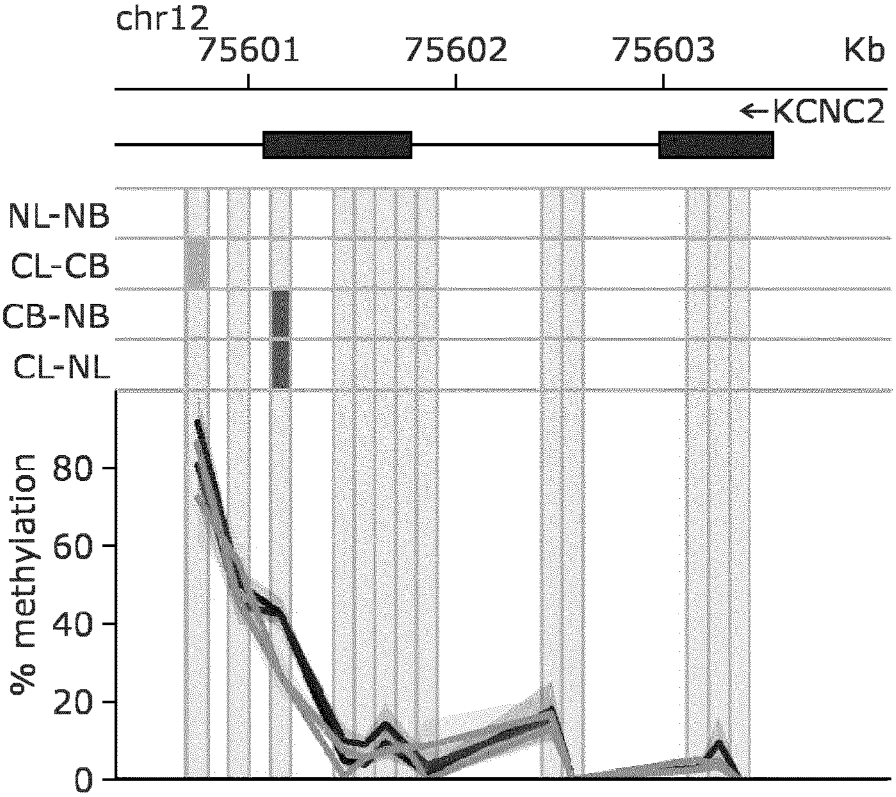
chr12
75601
75602
75603
Kb
←KCNC2
NL-NB
CL-CB
CB-NB
CL-NL
% methylation
80
60
40
20
0

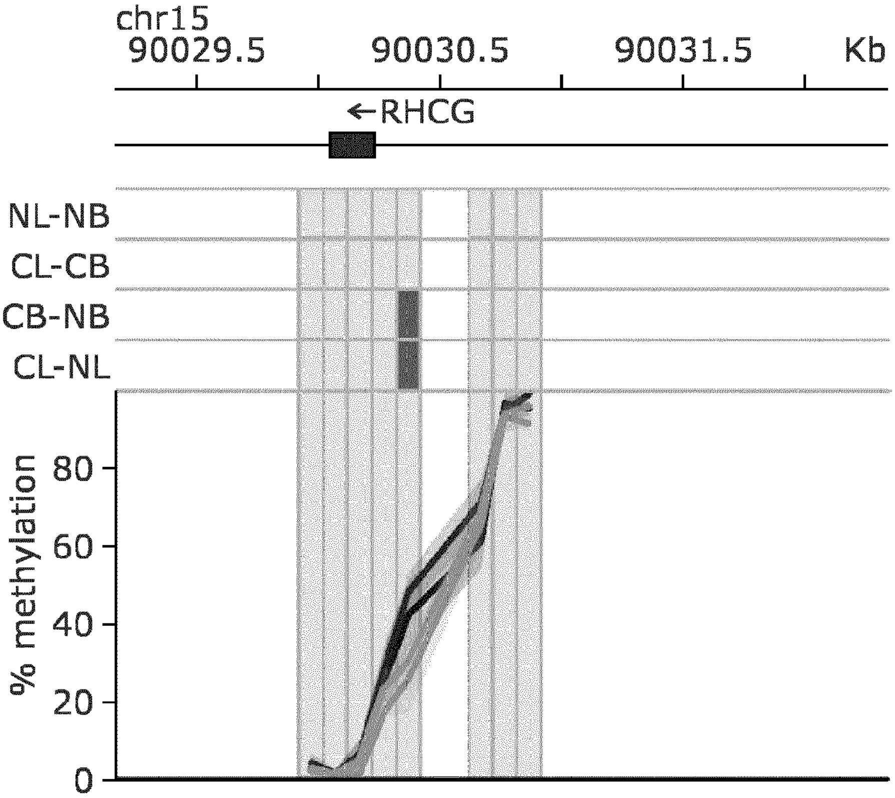
chr15
90029.5
90030.5
90031.5
Kb
←RHCG
NL-NB
CL-CB
CB-NB
CL-NL
% methylation
80
60
40
20
0

Figure 5B

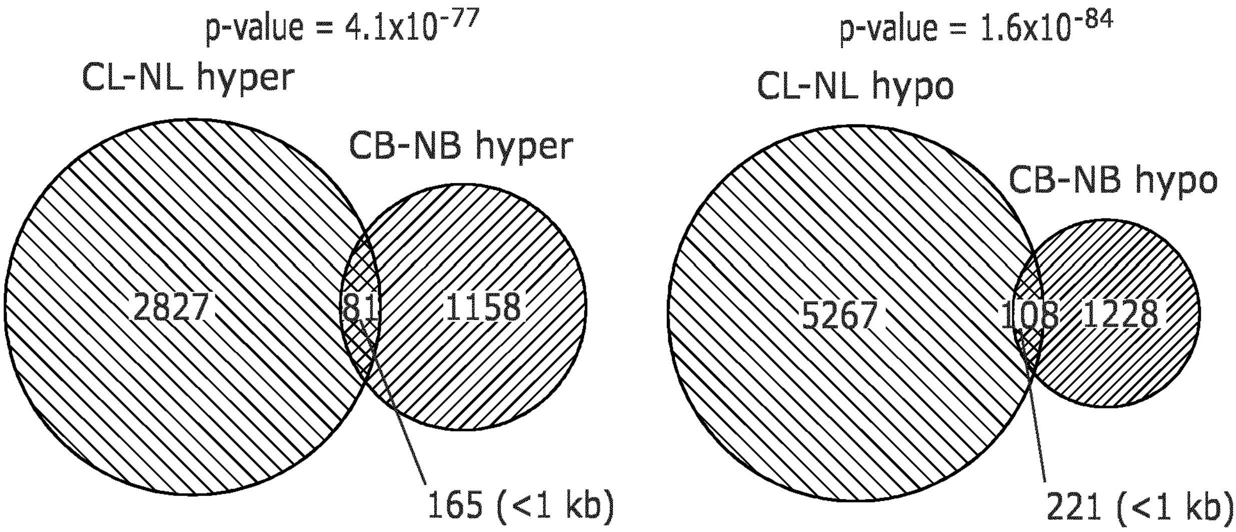
p-value = 4.1x10-77
CL-NL hyper
CB-NB hyper
2827
81
1158
165 (<1 kb)
p-value = 1.6x10-84
CL-NL hypo
CB-NB hypo
5267
108
1228
221 (<1 kb)

Figure 5A

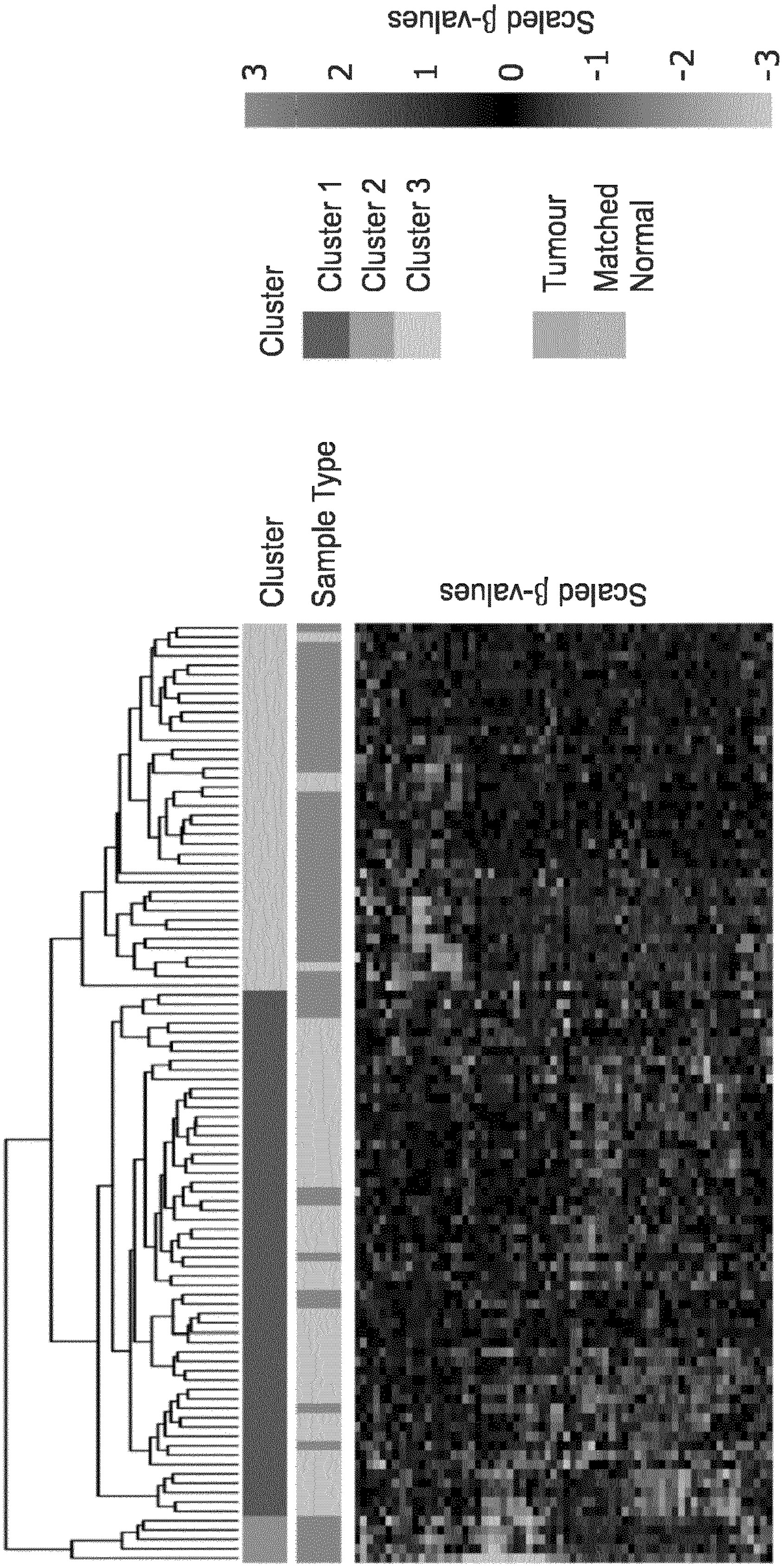
Cluster
Sample Type
Scaled β-values
Cluster
Cluster 1
Cluster 2
Cluster 3
Tumour
Matched Normal
3
2
1
0
-1
-2
-3
Scaled β-values

Figure 5C

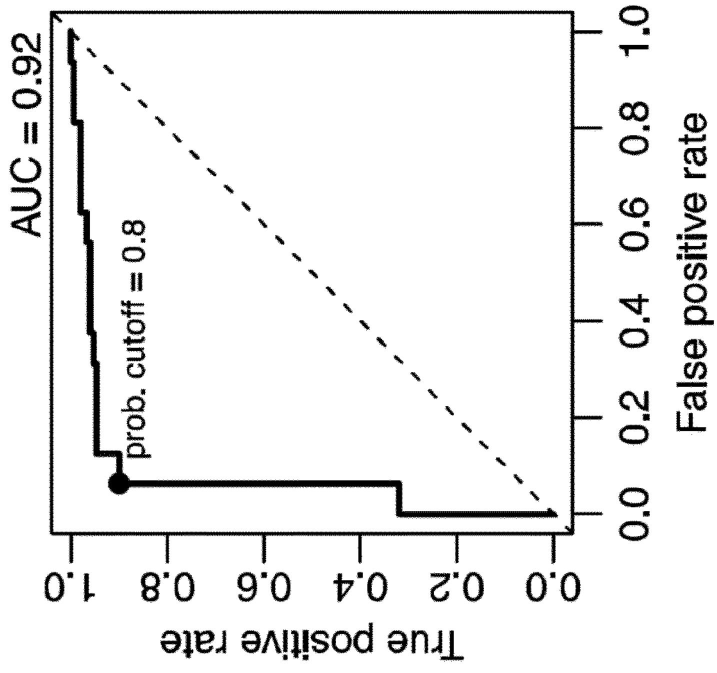


Figure 5E

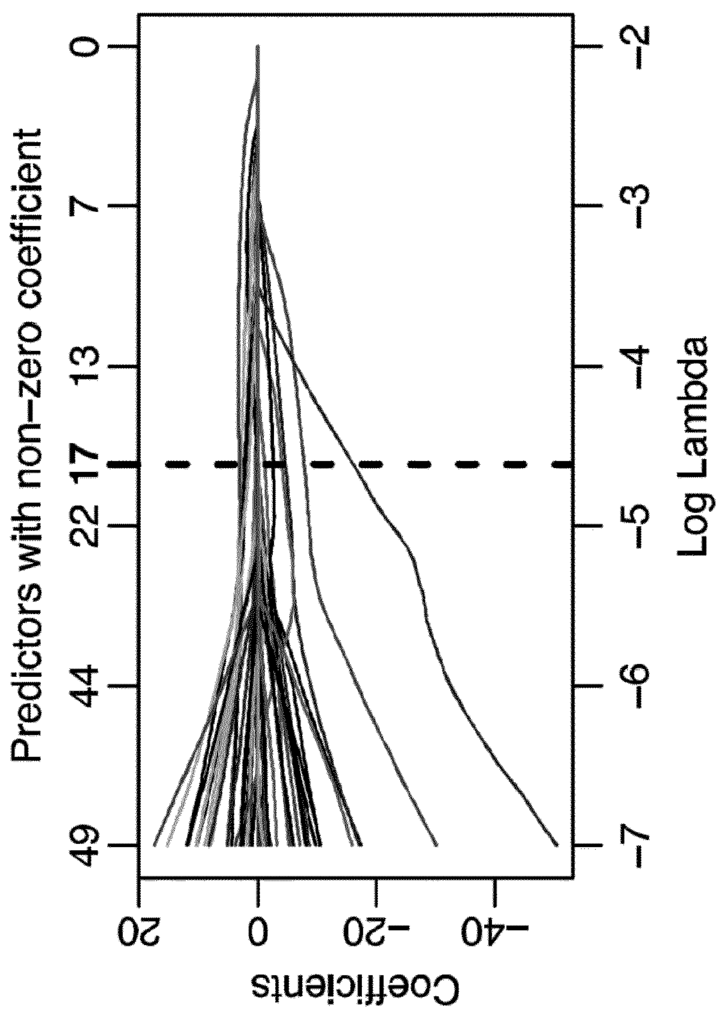


Figure 5D

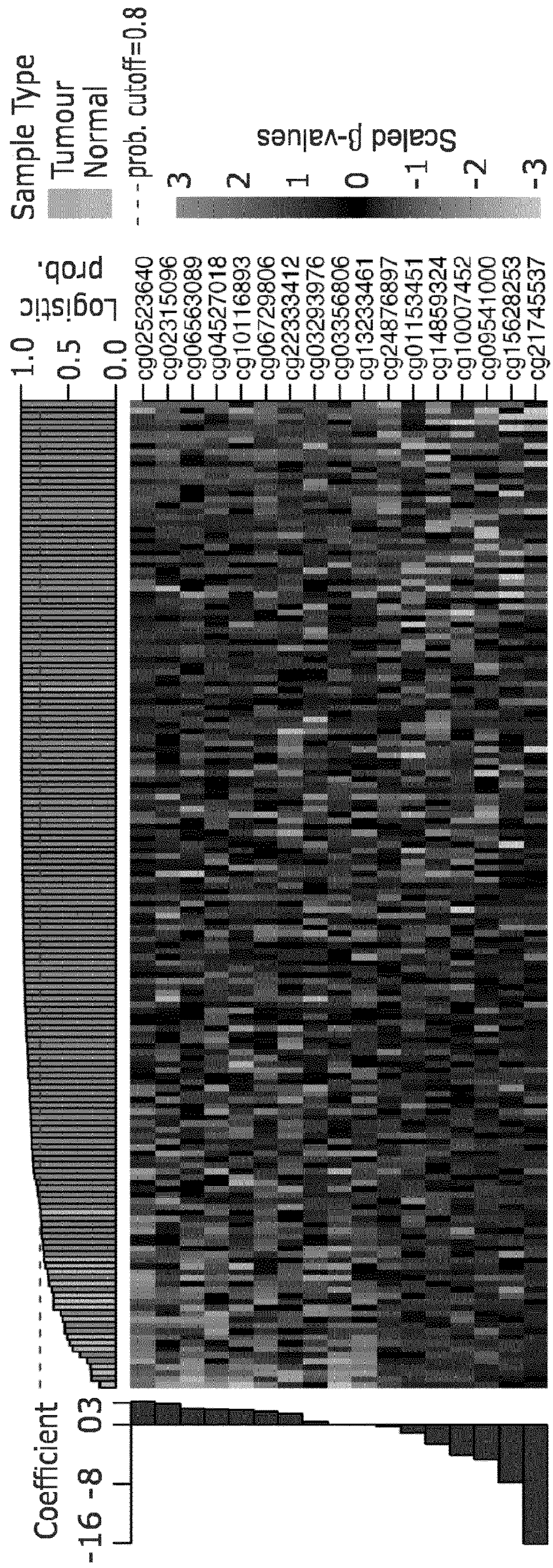

Sample Type
Tumour
Normal
prob. cutoff=0.8
Scaled β-values
3
2
1
0
-1
-2
-3
Logistic prob.
1.0
0.5
0.0
cg02523640
cg02315096
cg06563089
cg04527018
cg10116893
cg06729806
cg22333412
cg03293976
cg03356806
cg13233461
cg24876897
cg01153451
cg14859324
cg10007452
cg09541000
cg15628253
cg21745537
Coefficient
-16
-8
0
3


Figure 5F

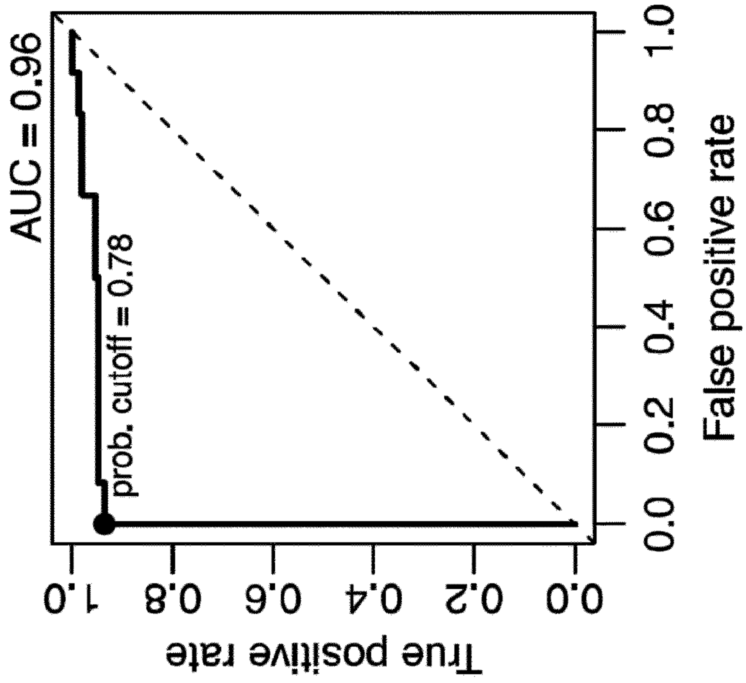


Figure 6B

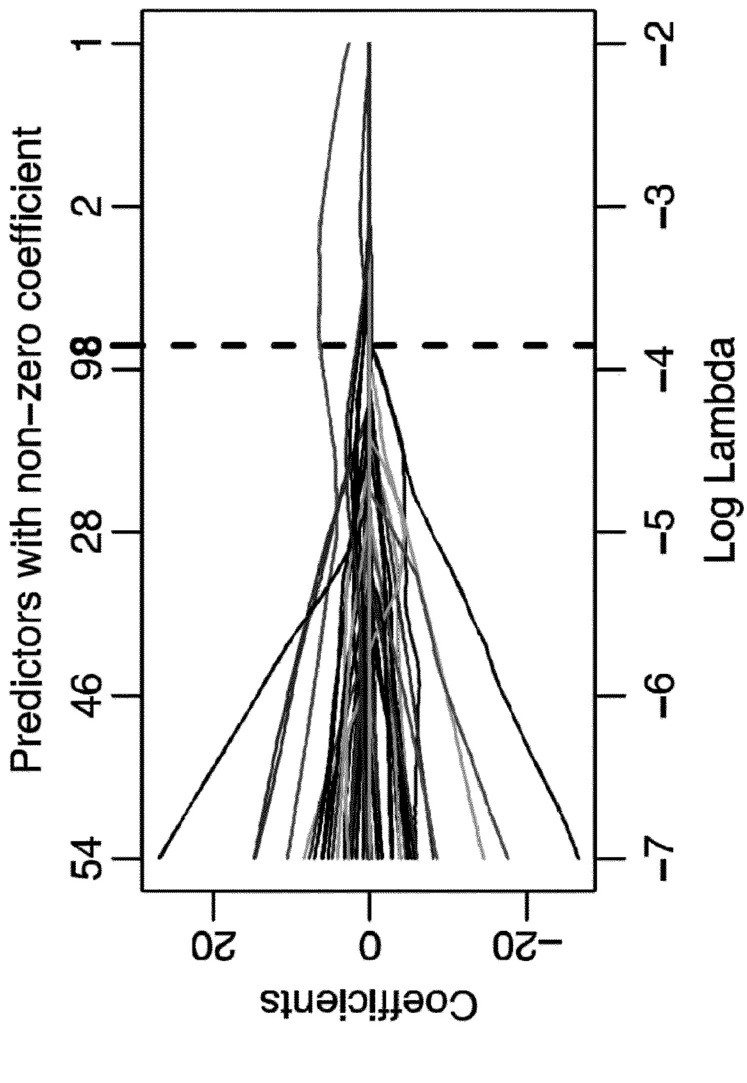


Figure 6A

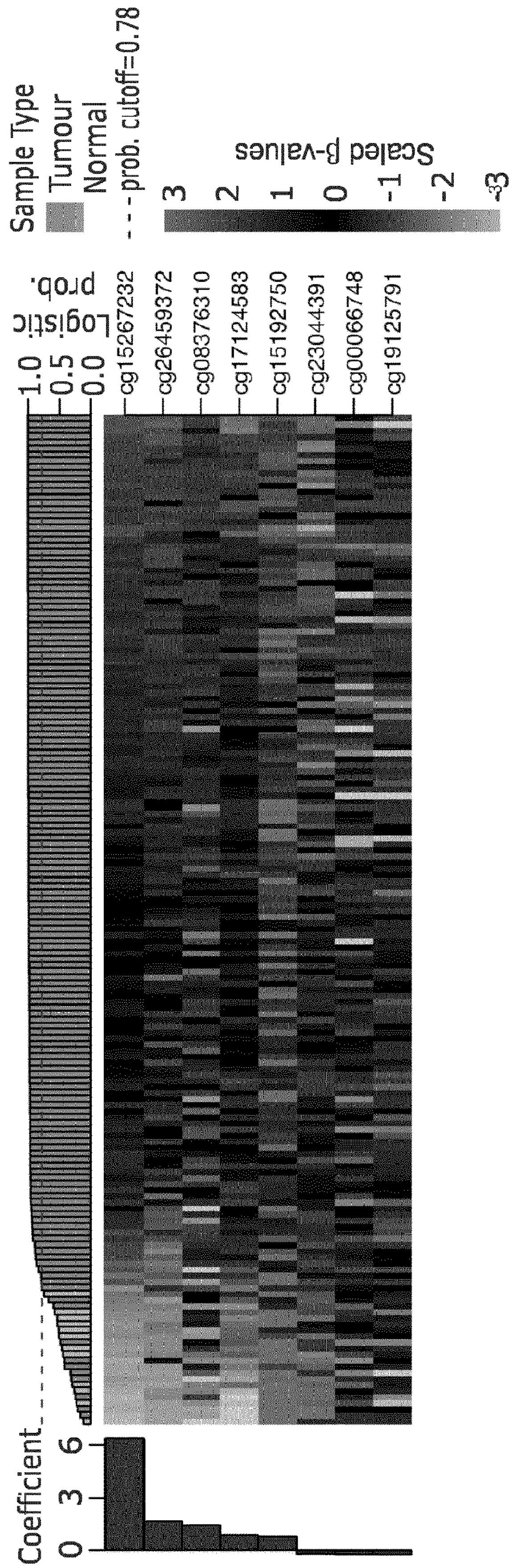

Sample Type
Tumour
Normal
prob. cutoff=0.78
Scaled β-values
3
2
1
0
-1
-2
-3
Logistic prob.
1.0
0.5
0.0
cg15267232
cg26459372
cg08376310
cg17124583
cg15192750
cg23044391
cg00066748
cg19125791
Coefficient
6
3
0


Figure 6C

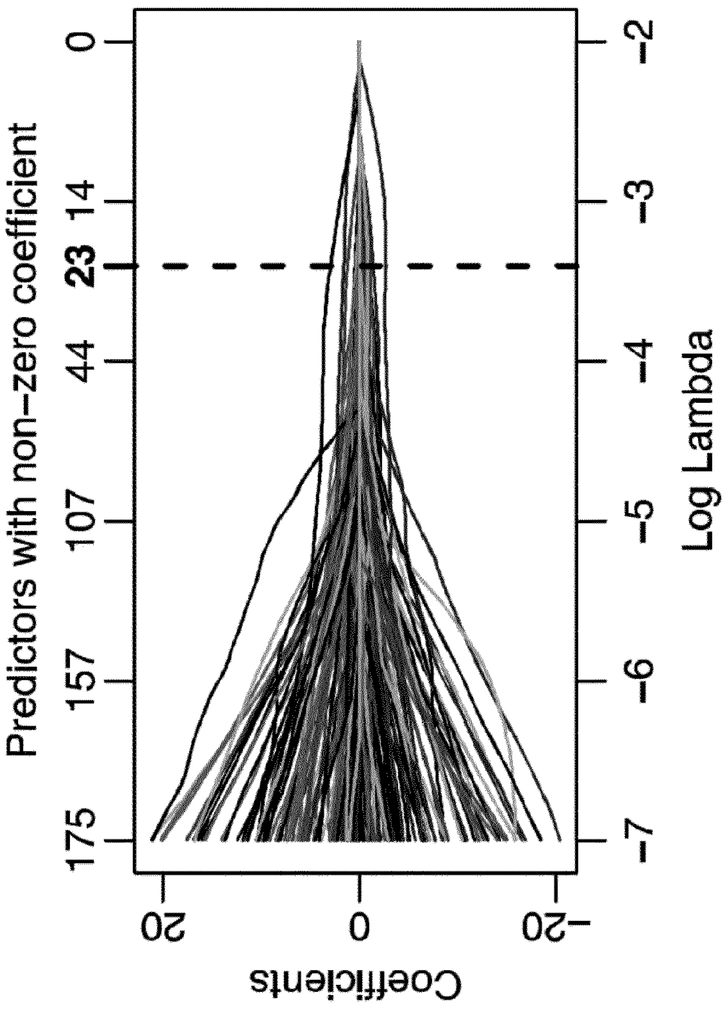


Figure 6D

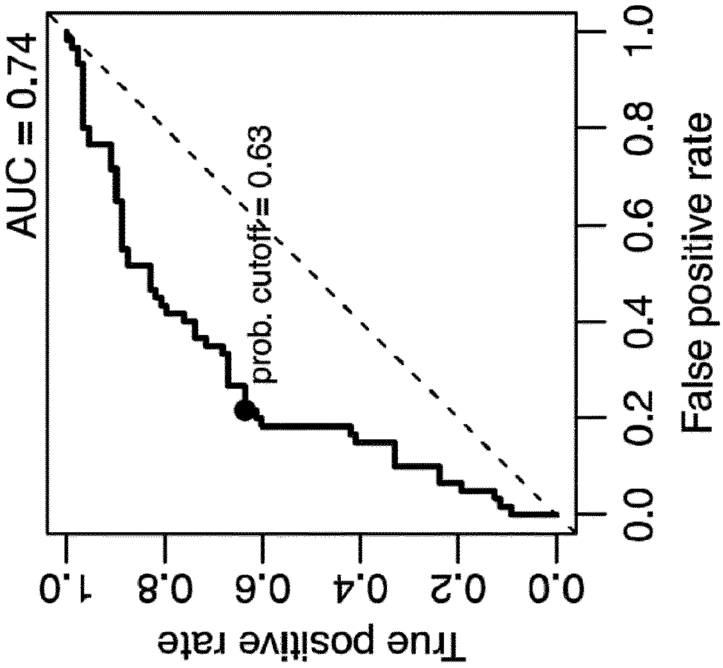


Figure 6E

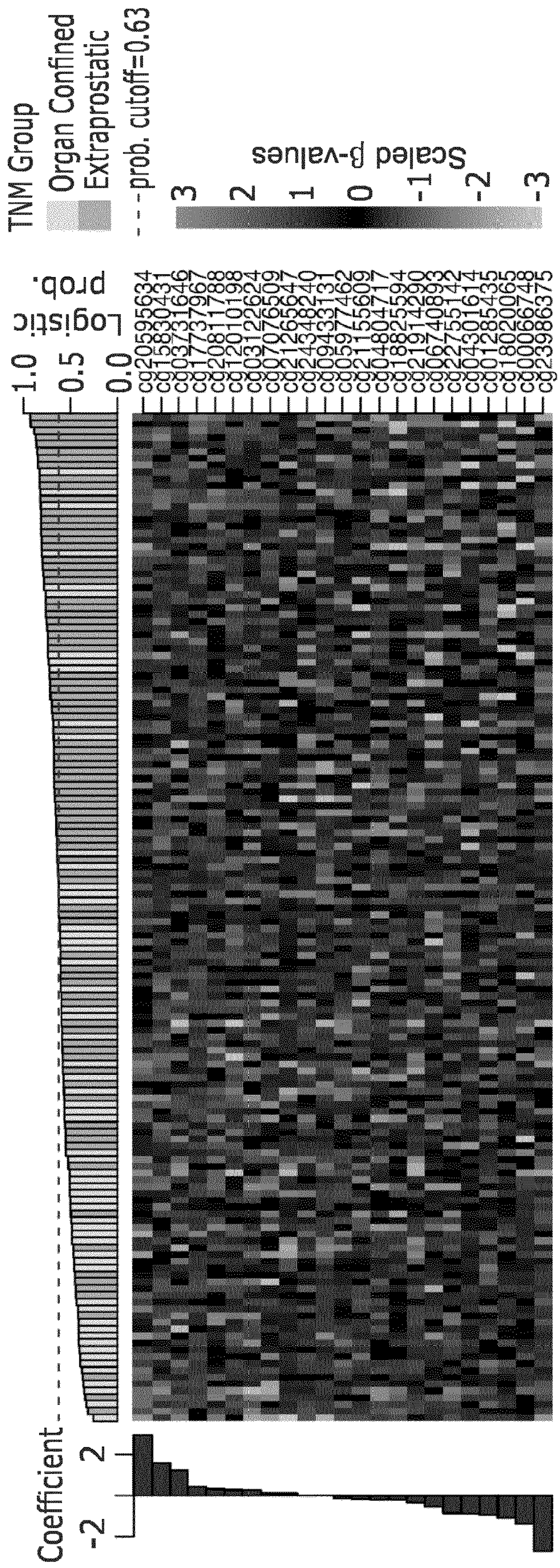
TNM Group
Organ Confined
Extraprostatic
prob. cutoff=0.63
Scaled β-values
3
2
1
0
-1
-2
-3
Logistic prob.
1.0
0.5
0.0
Coefficient
2
-2
cg20595634
cg15830431
cg03731646
cg17737967
cg20811788
cg12010198
cg03122624
cg07076509
cg21265647
cg24348240
cg09433131
cg05977462
cg21155609
cg04804717
cg18825594
cg21914290
cg06740893
cg22755142
cg04301614
cg01285435
cg18020065
cg00066748
cg23986375

Figure 6F

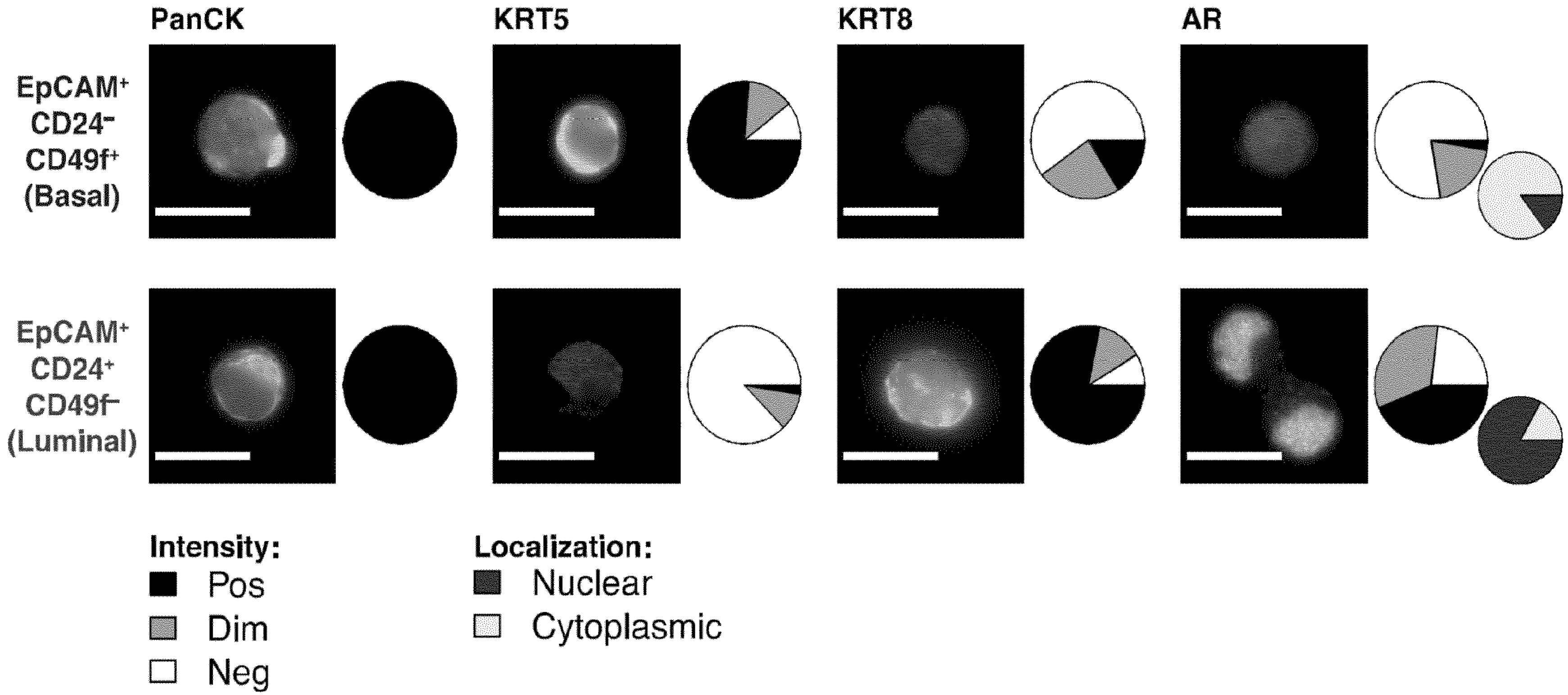


Supplementary Figure 1A

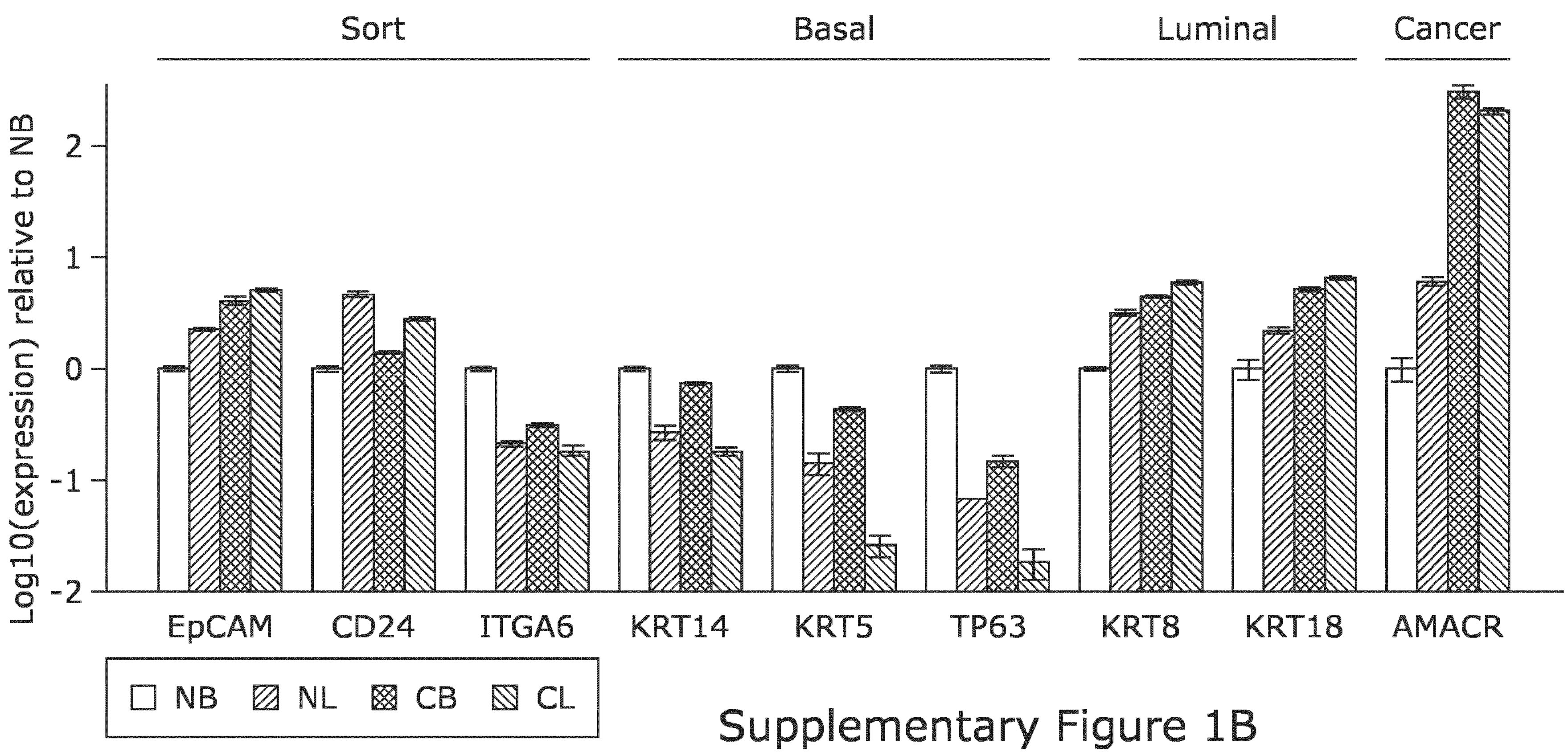


Supplementary Figure 1B

| Donor | Age | Gleason | PSA | Cancer Positive Biopsy Cores | Palpable Tumour |
|---|---|---|---|---|---|
| H329 | 53 | 3+4 | 51 | Right 0/5 - Left 3/5 | Left base |
| H309 | NA | 3+4 | 9.1 | Right 0/5 - Left 2/5 | Left apex |
| H323 | 64 | 3+4 | 20.4 | Right 6/7 - Left 7/8 | Left side and right apex |
| H324 | 62 | 3+4 | 3.9 | Right 4/5 - Left 0/5 | Right side |

Supplementary Figure 1C

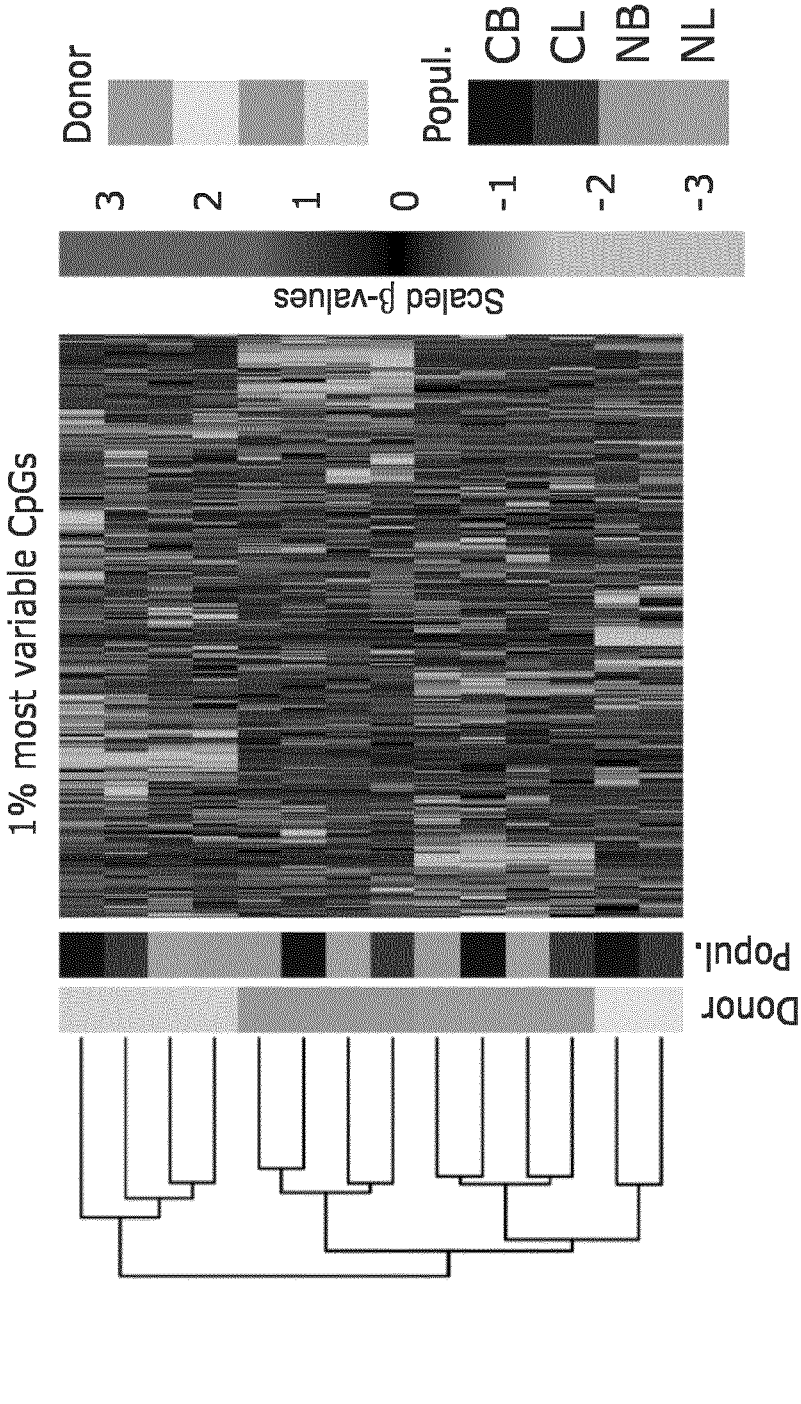


Supplementary Figure 2A

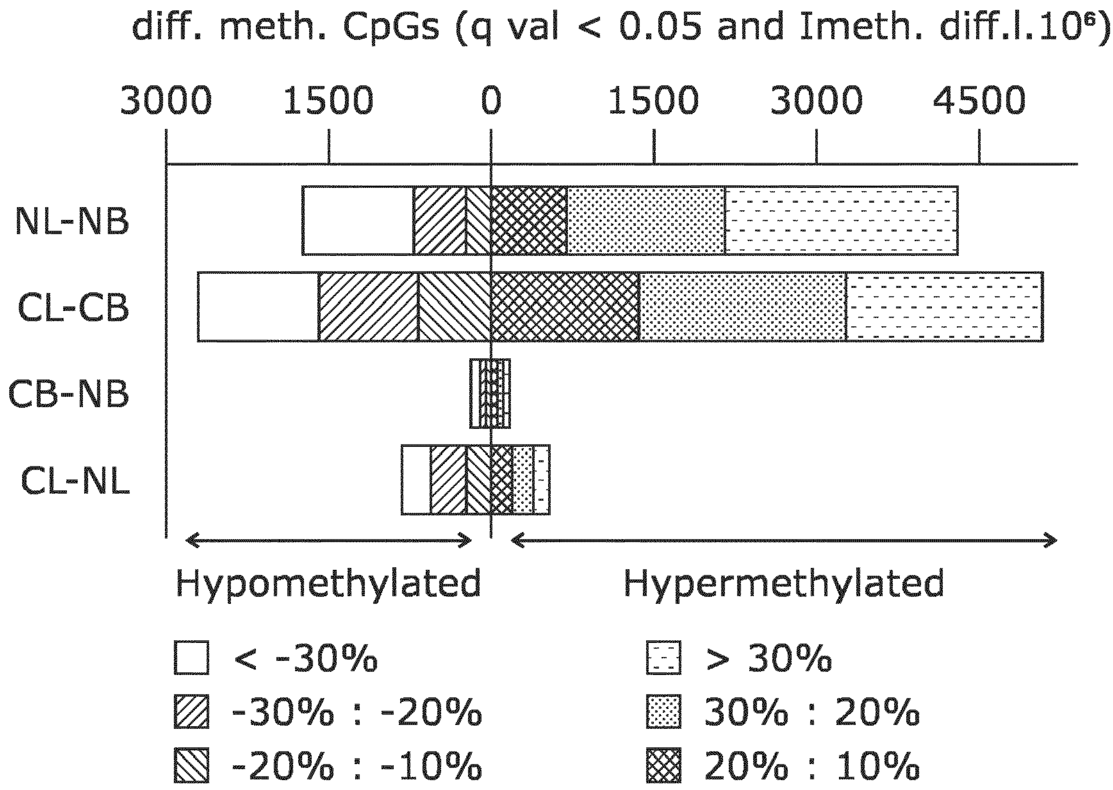


Supplementary Figure 2B

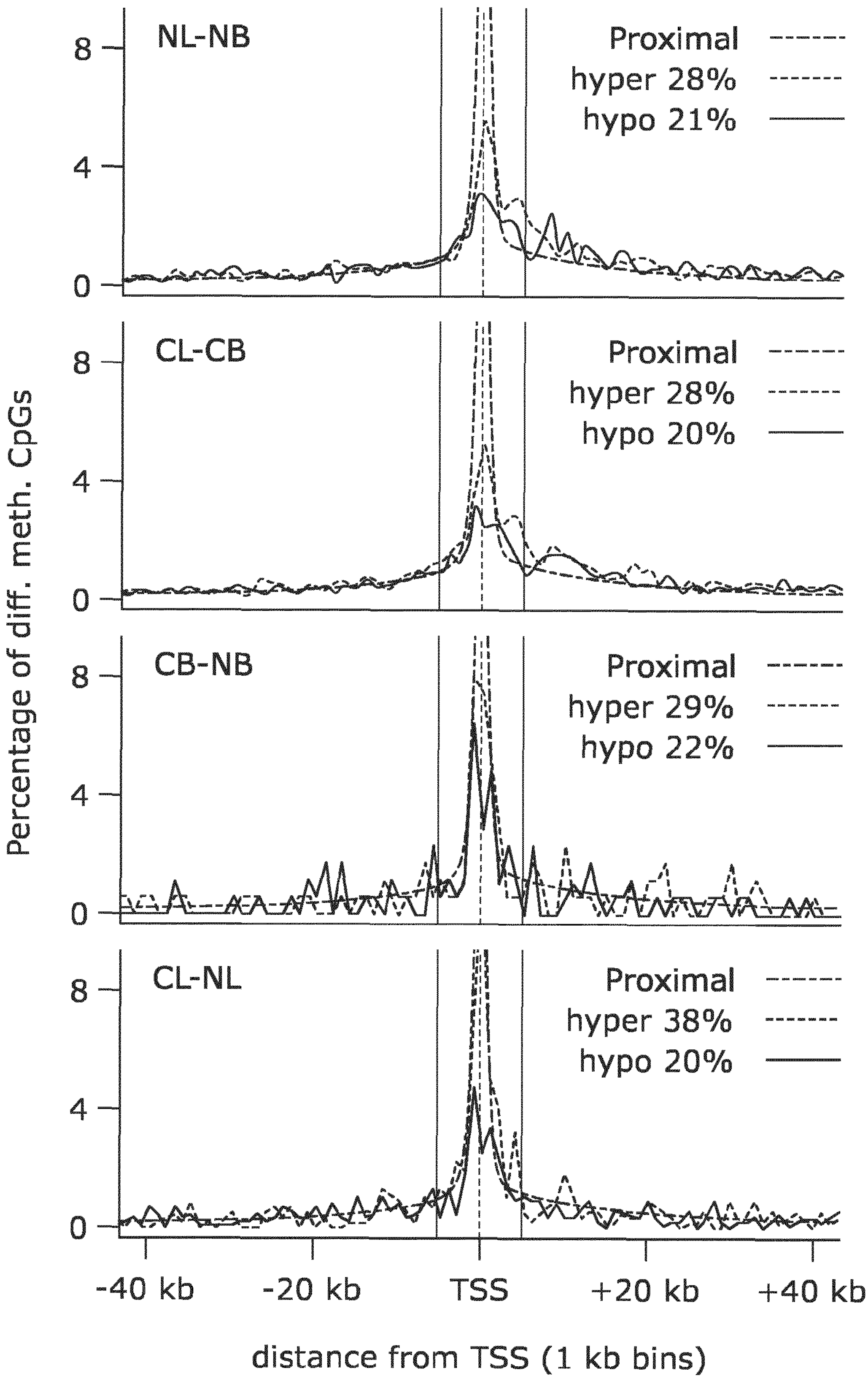


Supplementary Figure 2C

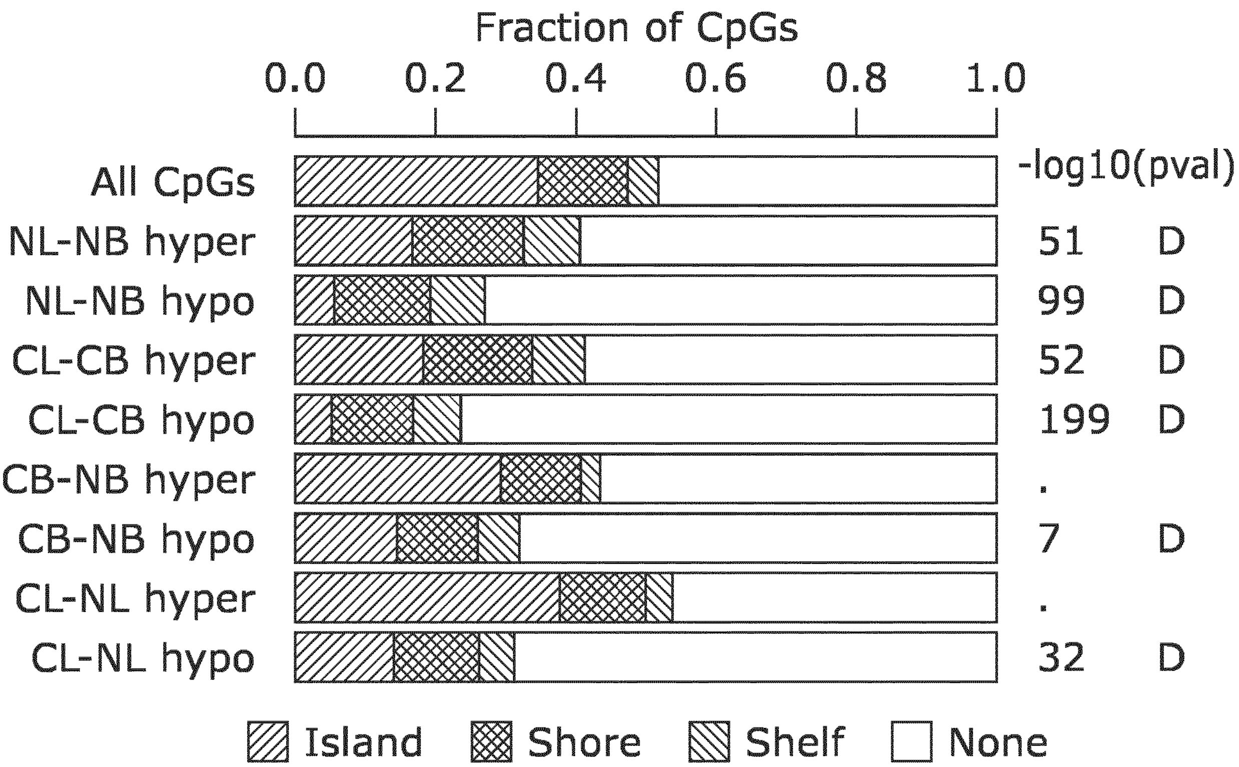


Supplementary Figure 2D

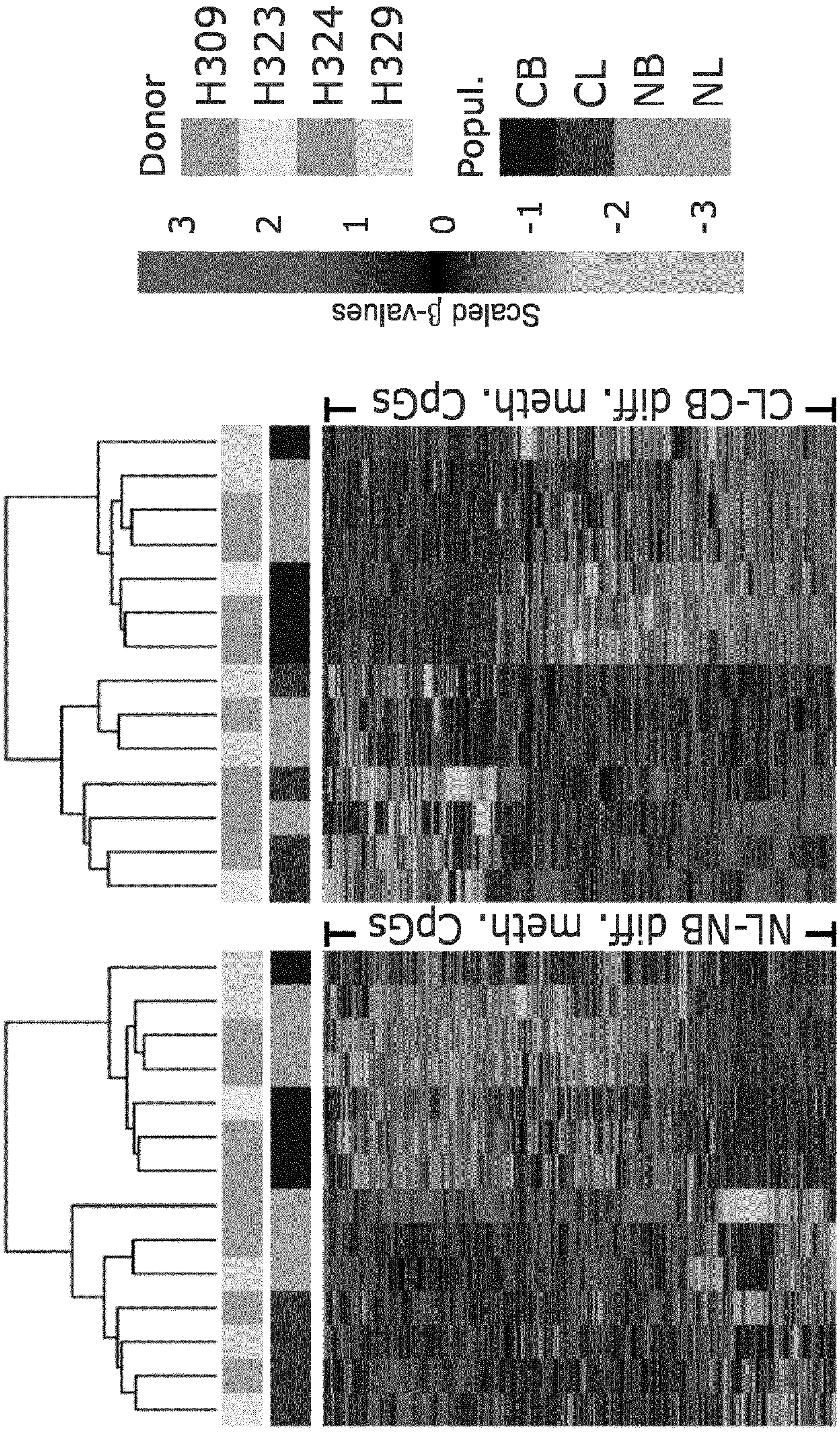


Supplementary Figure 2E

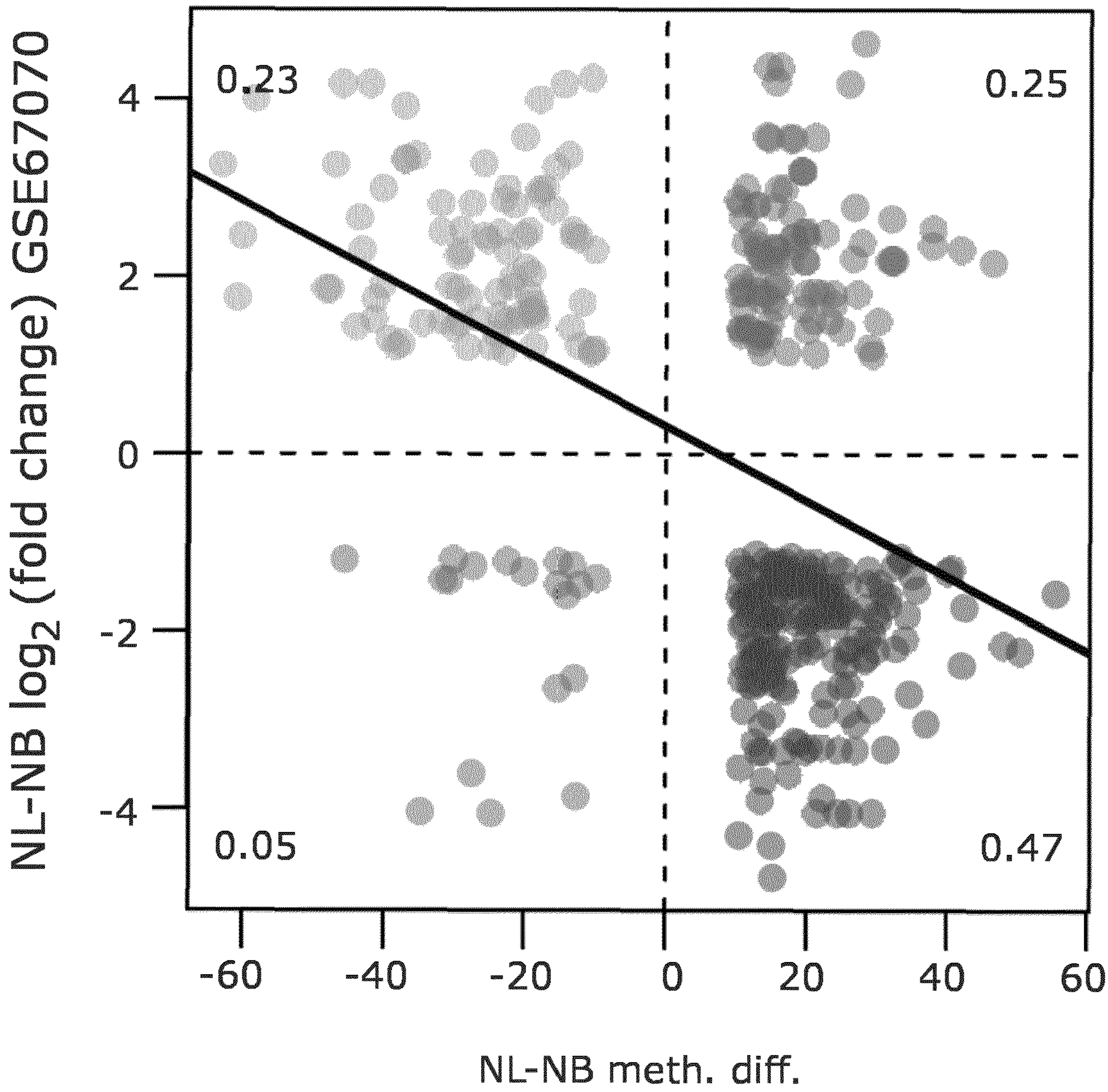


Supplementary Figure 3A

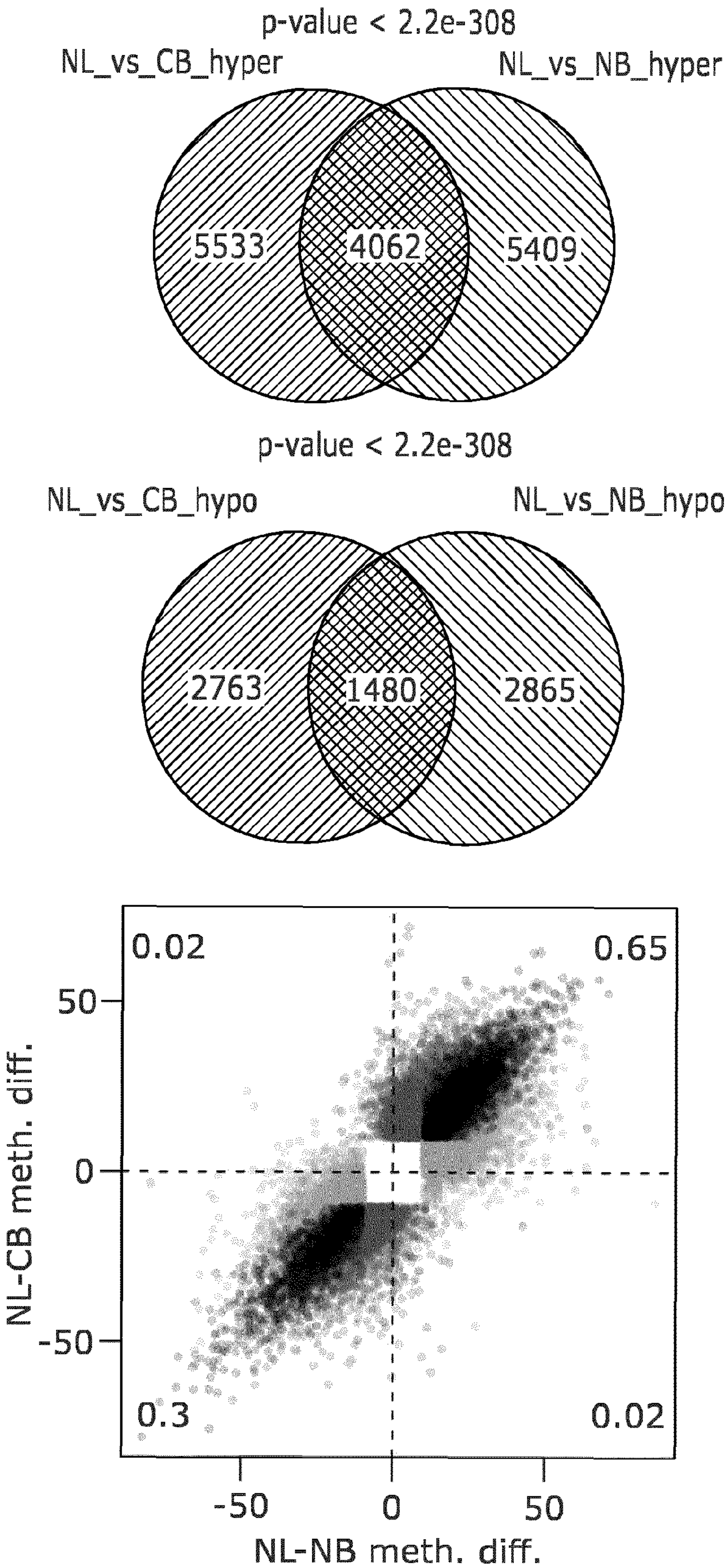


Supplementary Figure 3B

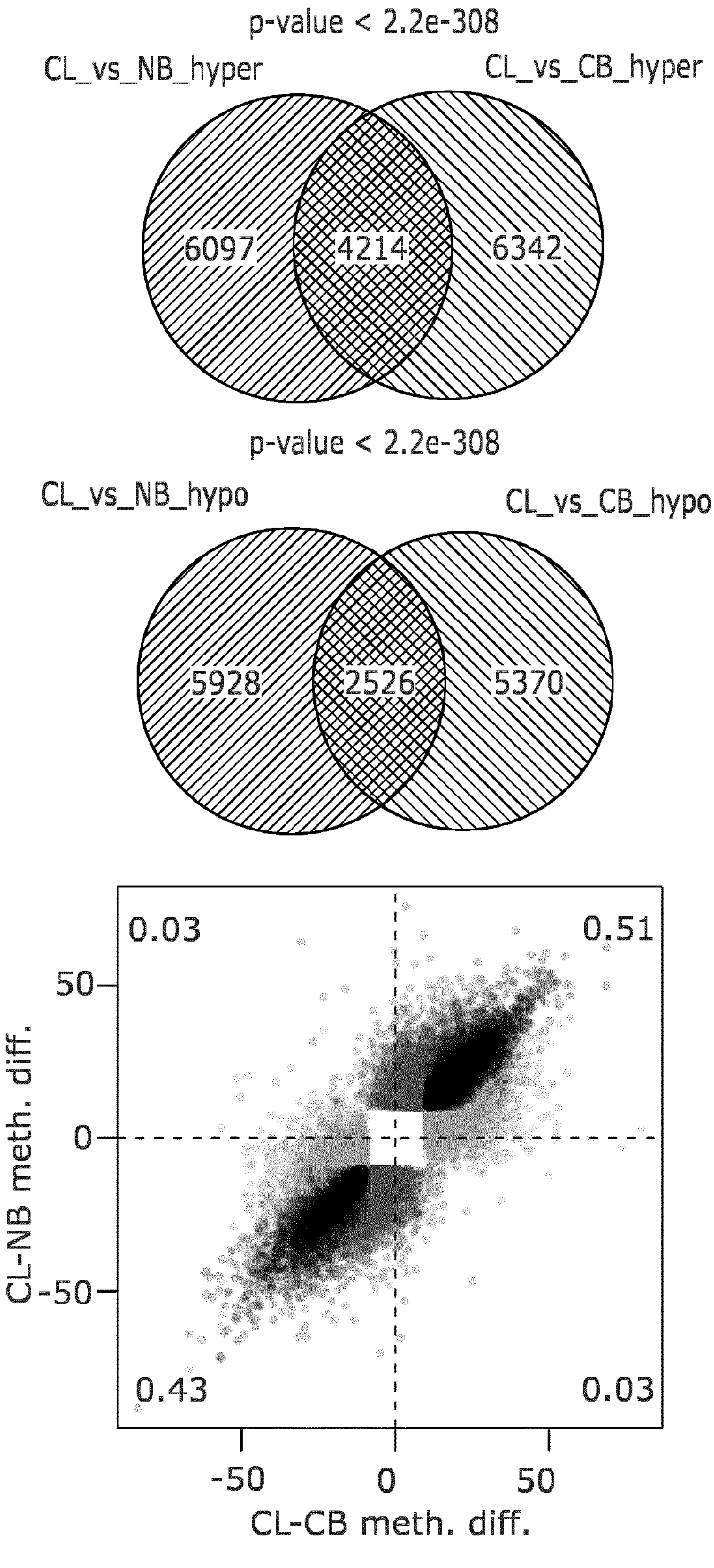


Supplementary Figure 3C

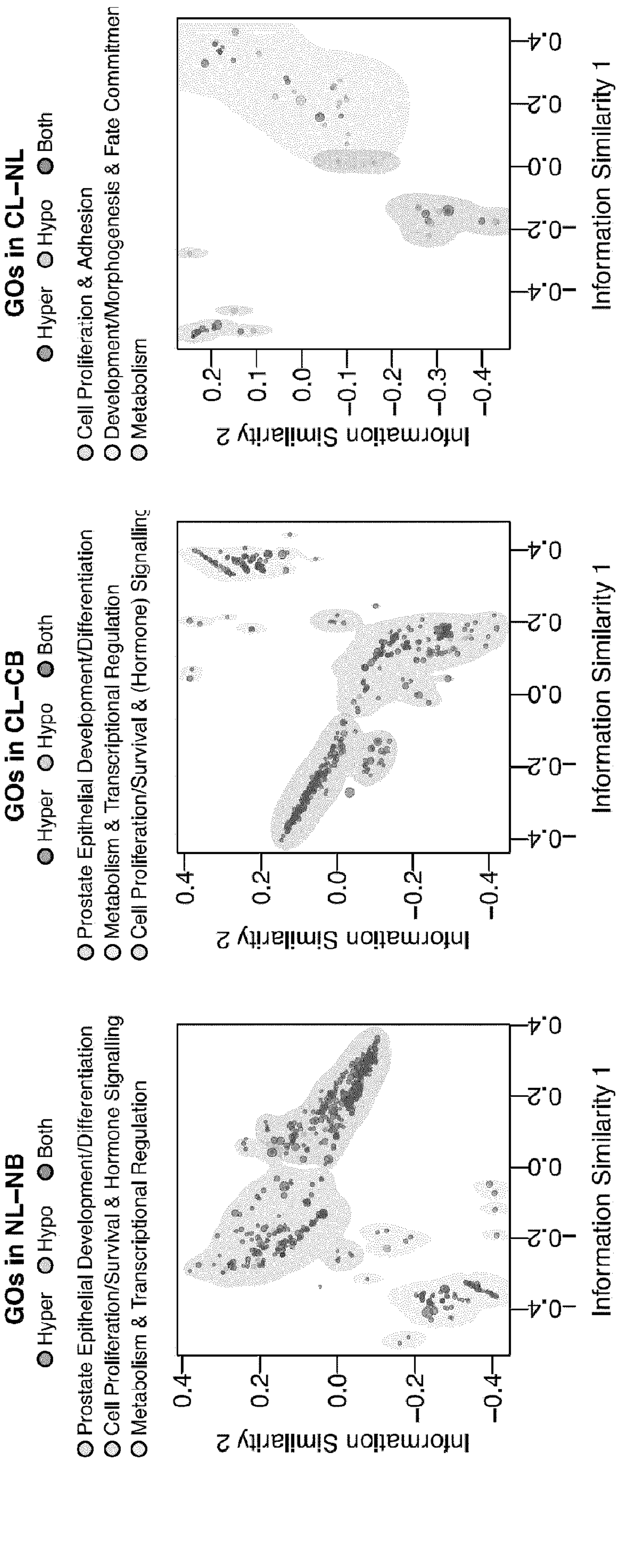


Supplementary Figure 4

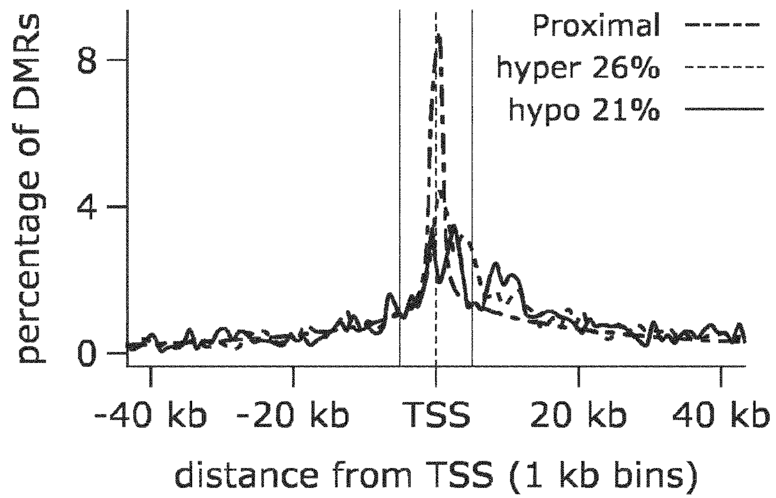


Supplementary Figure 5A

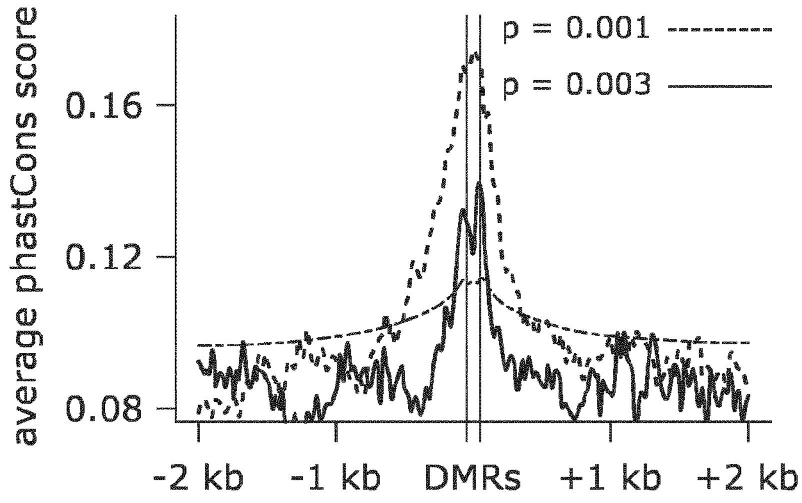


Supplementary Figure 5B

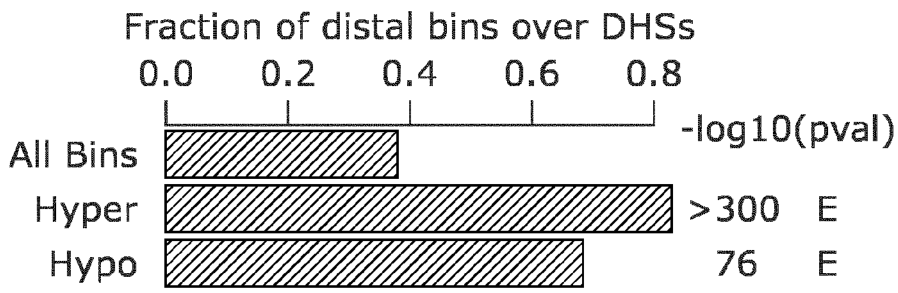


Supplementary Figure 5C

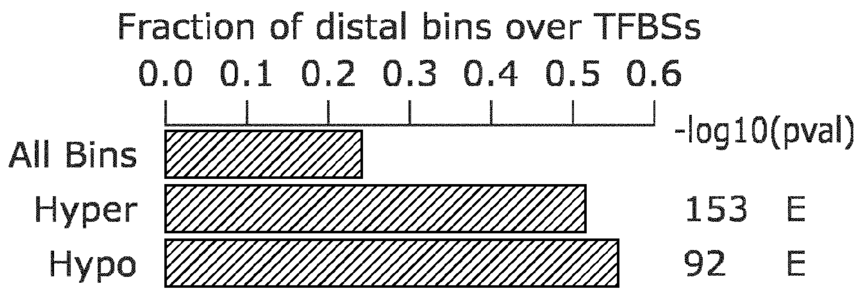


Supplementary Figure 5D

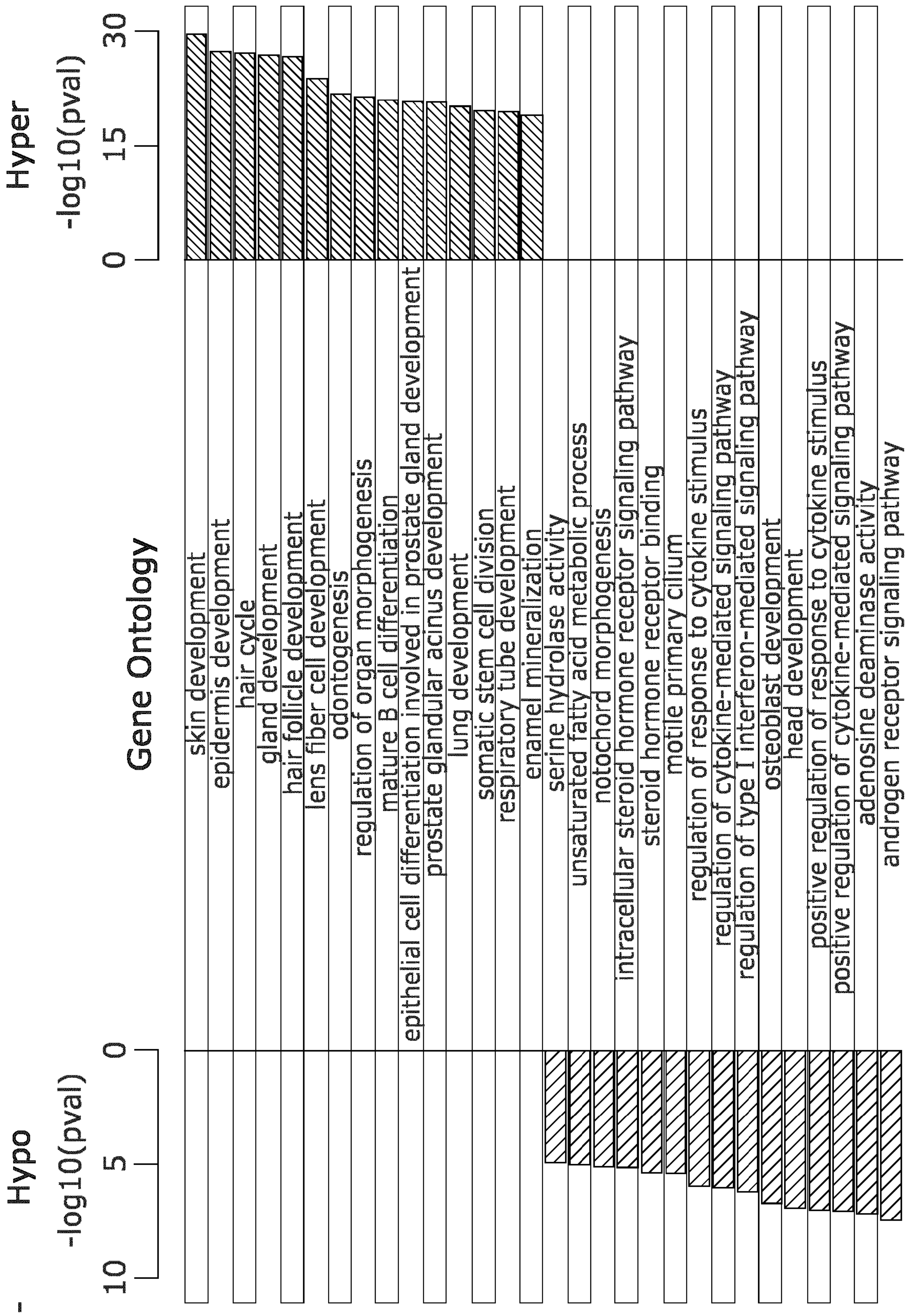


Supplementary Figure 5E

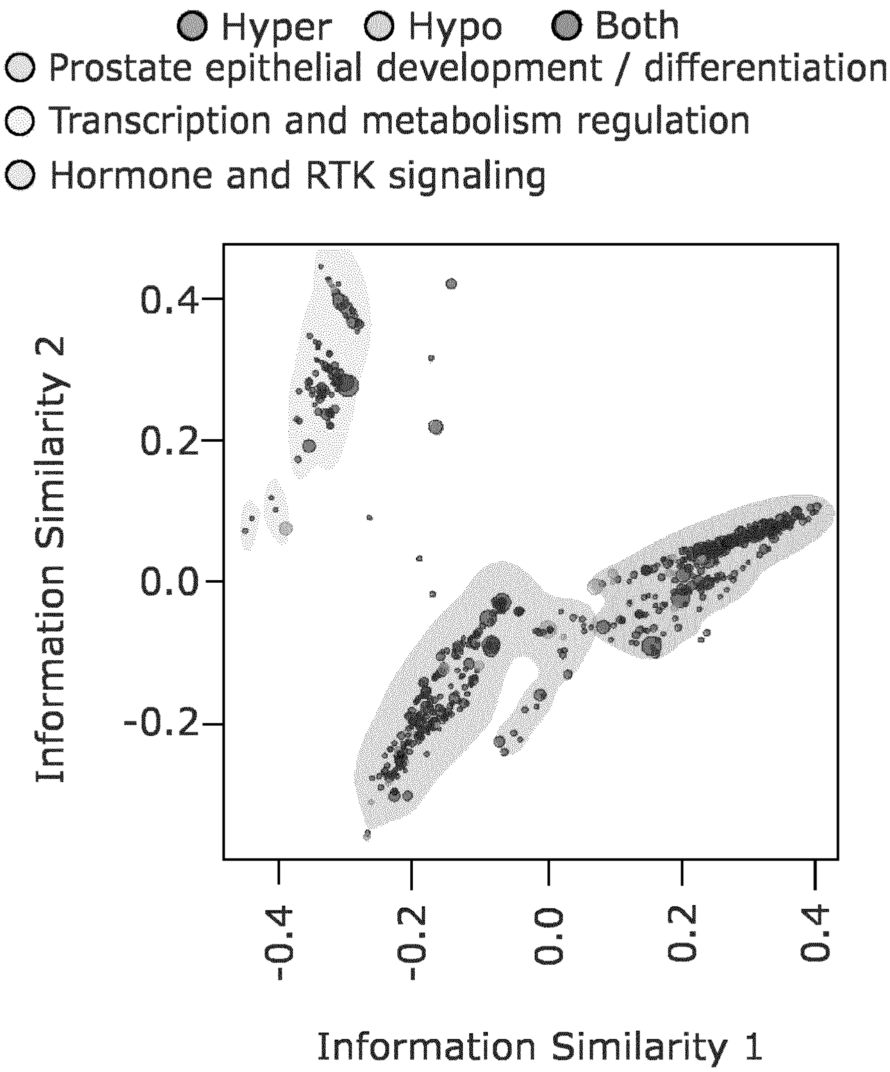


Supplementary Figure 5F

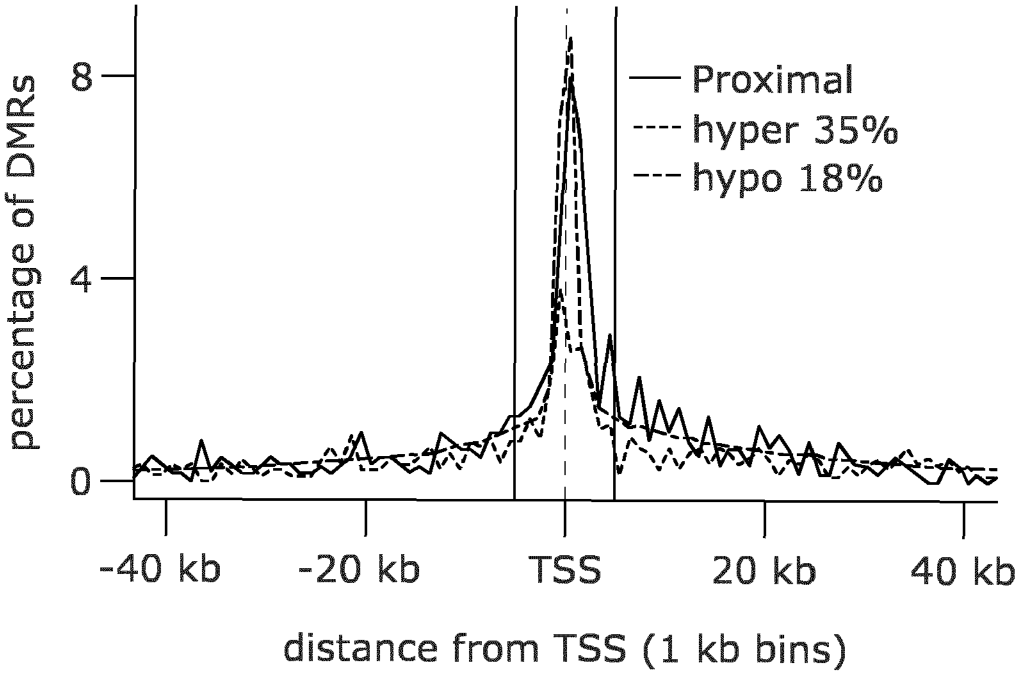


Supplementary Figure 6A

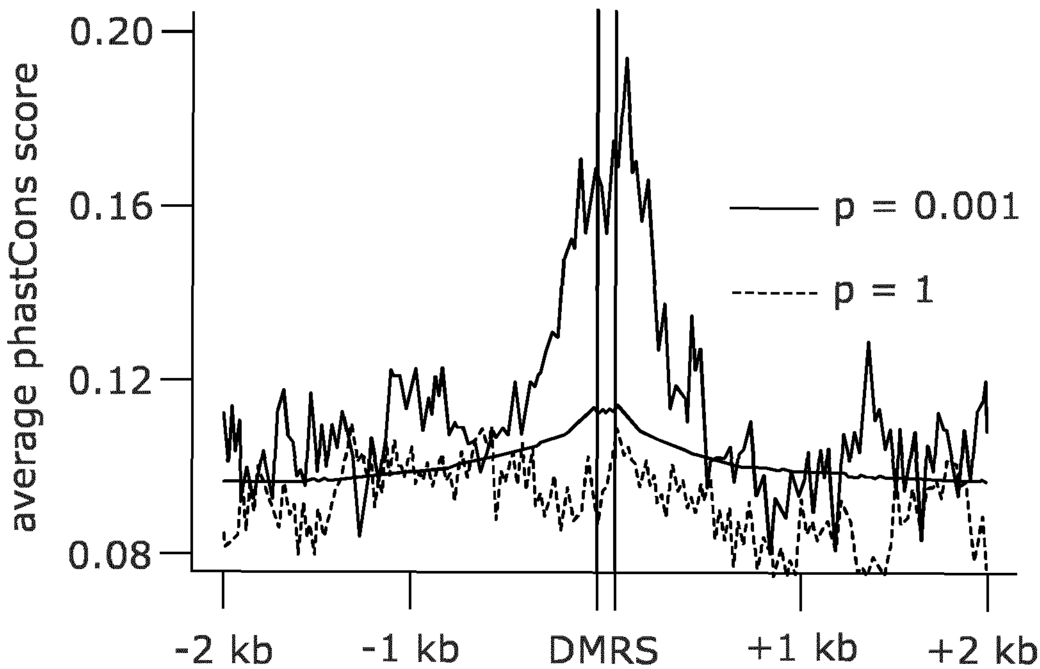


Supplementary Figure 6B

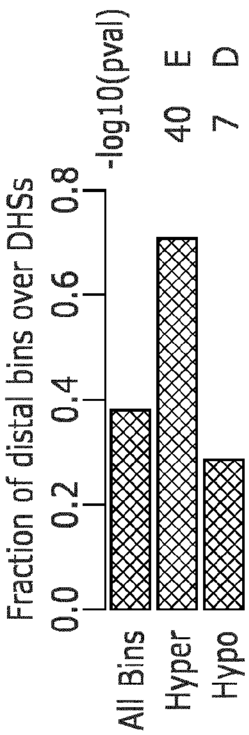


Supplementary Figure 6C

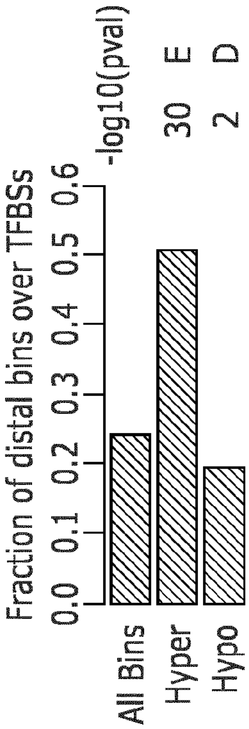


Supplementary Figure 6D

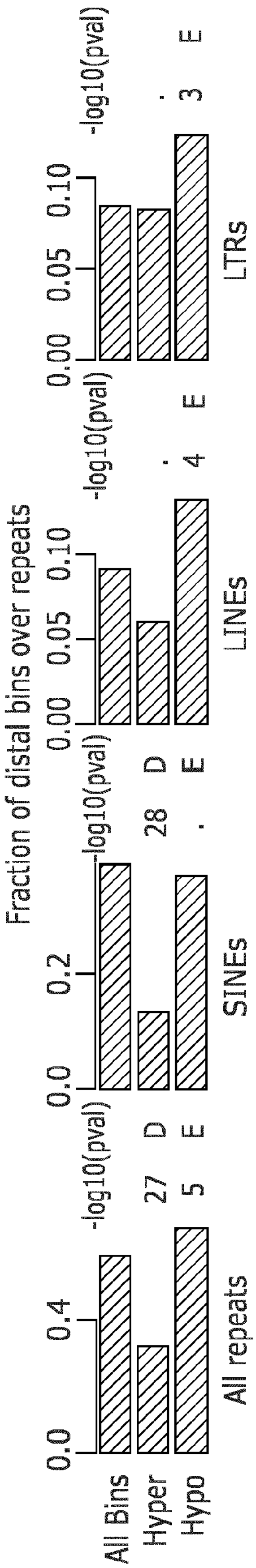


Supplementary Figure 6E

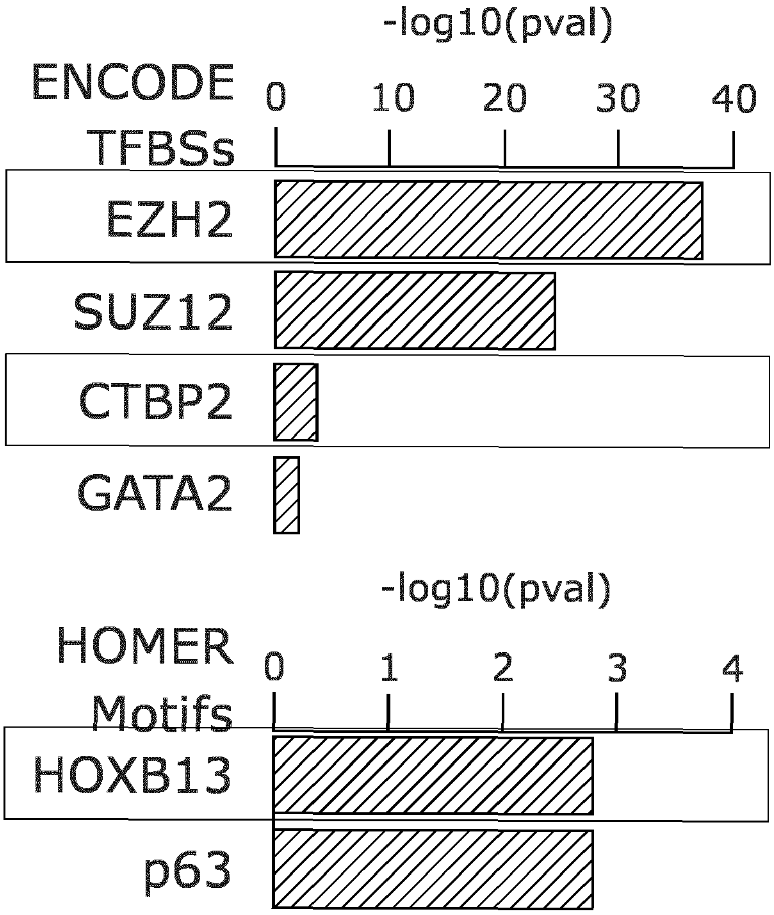


Supplementary Figure 6F

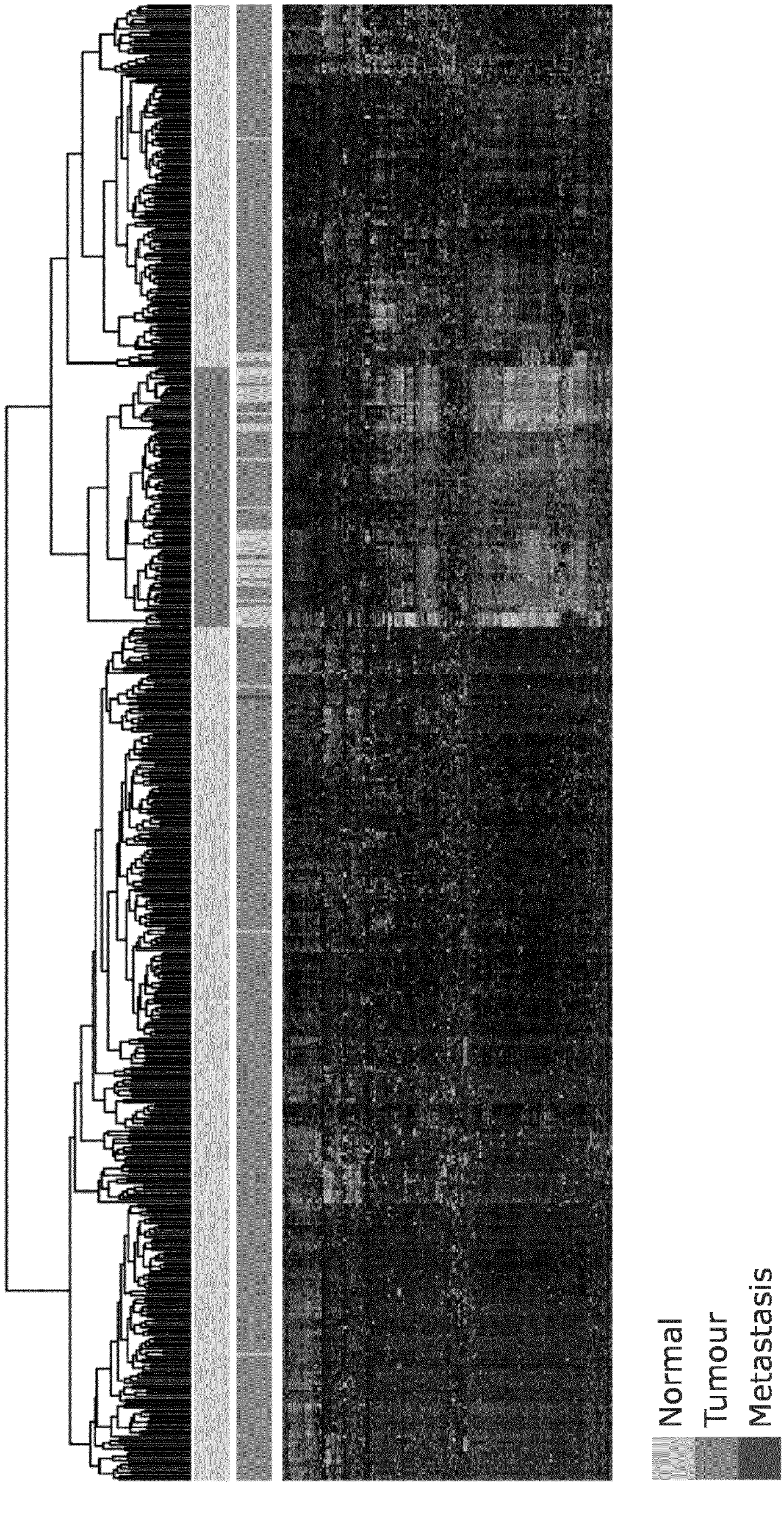


Supplementary Figure 6G

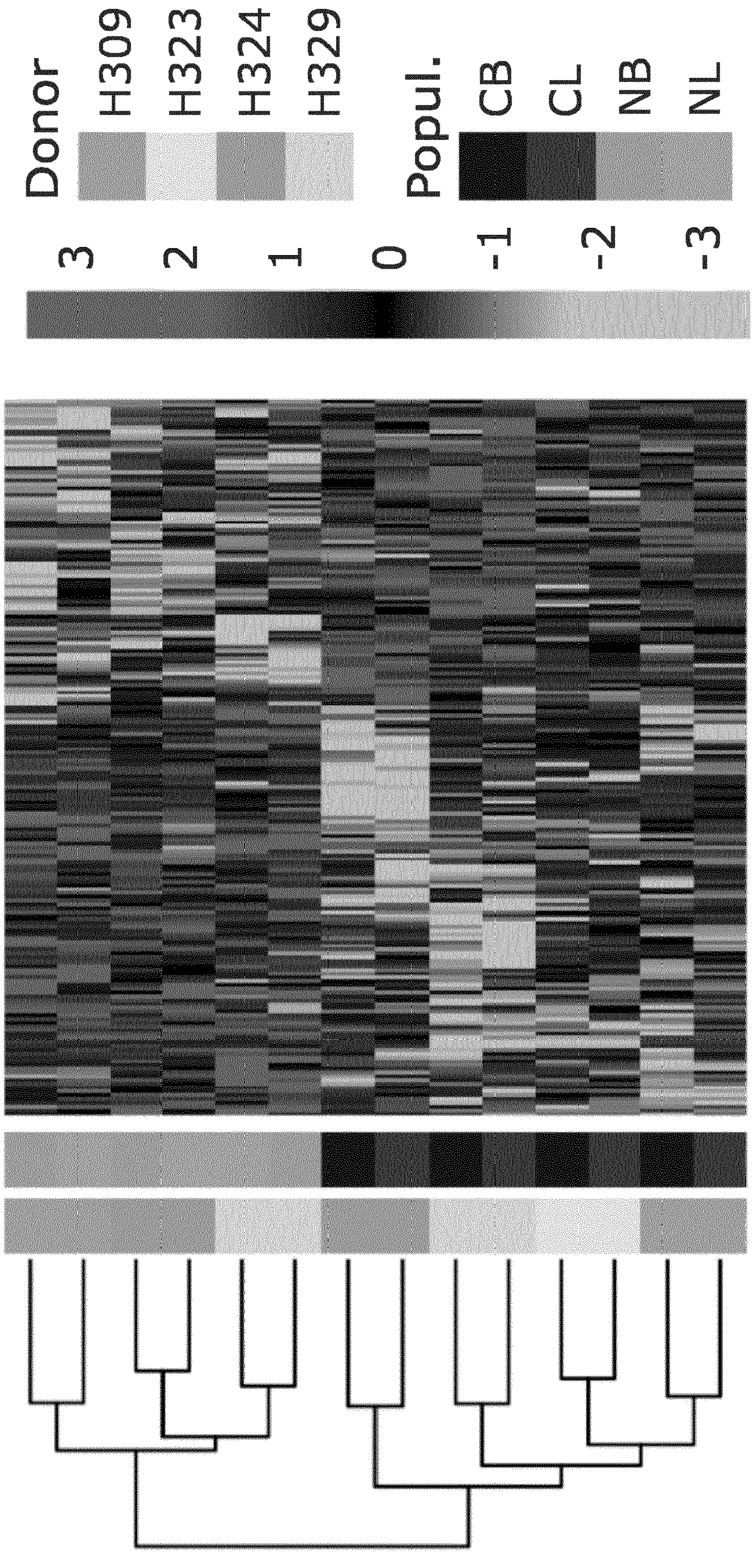


Supplementary Figure 7A

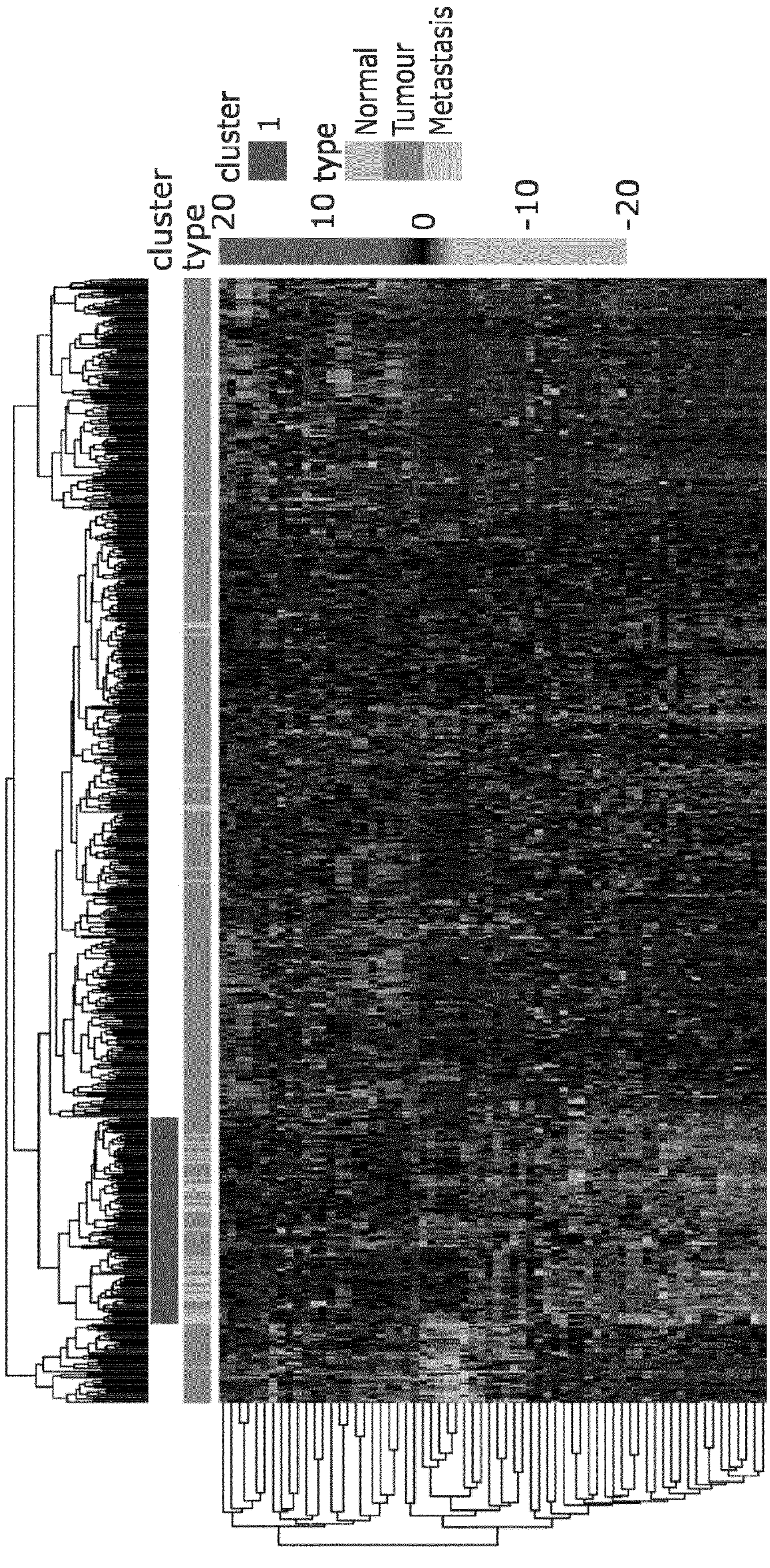


Supplementary Figure 7B

Europäisches Patentamt
European Patent Office
Office européen des brevets

# EUROPEAN SEARCH REPORT

Application Number
EP 19 16 2642

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2016/102674 A1 (UNIV DUBLIN [IE]) 30 June 2016 (2016-06-30) * the whole document * ----- | 1 | INV. C12Q1/6886 |
| A,D | WO 2013/185779 A2 (UNIV AARHUS [DK]; REGION MIDTJYLLAND [DK]) 19 December 2013 (2013-12-19) * the whole document * ----- | 1-3,9,15 | |
| X,D | WO 2017/143296 A2 (GILL INDERBIR SINGH [US]; JONES PETER [US] ET AL.) 24 August 2017 (2017-08-24) * the whole document * ----- | 1,9,15 | |

TECHNICAL FIELDS SEARCHED (IPC)
C12Q

~~The present search report has been drawn up for all claims~~

1

| Place of search | Date of completion of the search | Examiner |
|---|---|---|
| The Hague | 27 November 2019 | Cornelis, Karen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

Application Number
EP 19 16 2642

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-3, 9(completely); 15(partially)

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 19 16 2642

The Search Division considers that the present European patent application does not comply with the requirements of unity of invention and relates to several inventions or groups of inventions, namely:

1. claims: 1-3, 9(completely); 15(partially)

   A diagnostic method to determine the methylation of one or more of SEQ ID 26-48 a a measure of the aggressiveness of prostate cancer.

   ---

2. claims: 4-6, 10(completely); 15(partially)

   A diagnostic method to determine the methylation of one or more of SEQ ID 18-25 a a measure of the likelihood of prostate cancer.

   ---

3. claims: 7, 8, 11(completely); 15(partially)

   A diagnostic method to determine the methylation of one or more of SEQ ID 1-17 a a measure of the likelihood of prostate cancer.

   ---

4-6. claims: 12-14(completely); 15(partially)

   Diagnostic method to characterise a sample with or without prostate cancer by evaluating the expression level of the respective genes of claims 12-14.

   ---

# ANNEX TO THE EUROPEAN SEARCH REPORT ON EUROPEAN PATENT APPLICATION NO. EP 19 16 2642

This annex lists the patent family members relating to the patent documents cited in the above-mentioned European search report.
The members are as contained in the European Patent Office EDP file on
The European Patent Office is in no way liable for these particulars which are merely given for the purpose of information.

27-11-2019

| Patent document cited in search report | Publication date | Patent family member(s) | Publication date |
|---|---|---|---|
| WO 2016102674 A1 | 30-06-2016 | EP 3037545 A1 | 29-06-2016 |
| | | EP 3237640 A1 | 01-11-2017 |
| | | US 2017349952 A1 | 07-12-2017 |
| | | WO 2016102674 A1 | 30-06-2016 |
| WO 2013185779 A2 | 19-12-2013 | AU 2013275761 A1 | 22-01-2015 |
| | | CA 2876252 A1 | 19-12-2013 |
| | | EP 2861759 A2 | 22-04-2015 |
| | | JP 6242388 B2 | 06-12-2017 |
| | | JP 2015527875 A | 24-09-2015 |
| | | US 2015152507 A1 | 04-06-2015 |
| | | WO 2013185779 A2 | 19-12-2013 |
| WO 2017143296 A2 | 24-08-2017 | US 2019382845 A1 | 19-12-2019 |
| | | WO 2017143296 A2 | 24-08-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2016102674 A
- WO 2014160829 A
- WO 2013185779 A
- WO 2017143296 A
- WO 2012138609 A
- WO 2013140161 A
- WO 2008143896 A

### Non-patent literature cited in the description


- **MASSIE CE ; MILLS IG ; LYNCH AG.** The importance of DNA methylation in prostate cancer development. *J Steroid Biochem Mol Biol.,* February 2017, vol. 166, 1-15
- **ZHANG D ; PARK D ; ZHONG Y ; LU Y ; RYCAJ K ; GONG S et al.** Stem cell and neurogenic gene-expression profiles link prostate basal cells to aggressive prostate cancer. *Nat Commun,* 29 February 2016, vol. 7, 10798
- **PELLACANI D ; KESTORAS D ; DROOP A ; FRAME FM ; BERRY PA ; LAWRENCE MG et al.** DNA hypermethylation in prostate cancer is a consequence of aberrant epithelial differentiation and hyperproliferation. *Cell Death Differ.,* May 2014, vol. 21 (5), 761-73
- **FRAME FM ; PELLACANI D ; COLLINS AT ; MAITLAND NJ.** Harvesting Human Prostate Tissue Material and Culturing Primary Prostate Epithelial Cells. *Methods Mol Biol.,* 2016, vol. 1443, 181-201.4
- **AKALIN A ; KORMAKSSON M ; LI S ; GARRETT-BAKELMAN FE ; FIGUEROA ME ; MELNICK A et al.** methylKit: a comprehensive R package for the analysis of genome-wide DNA methylation profiles. *Genome Biol.,* 03 October 2012, vol. 13 (10), R87
- **POHL A ; BEATO M.** bwtool: a tool for bigWig files. *Bioinformatics,* 01 June 2014, vol. 30 (11), 1618-9
- **MCLEAN CY ; BRISTOR D ; HILLER M ; CLARKE SL ; SCHAAR BT ; LOWE CB et al.** GREAT improves functional interpretation of cis-regulatory regions. *Nat Biotechnol.,* May 2010, vol. 28 (5), 495-501
- Cancer Genome Atlas Research Network. *The Molecular Taxonomy of Primary Prostate Cancer,* 05 November 2015, vol. 163 (4), 1011-25
- **YU YP ; DING Y ; CHEN R ; LIAO SG ; REN B-G ; MICHALOPOULOS A et al.** Whole-genome methylation sequencing reveals distinct impact of differential methylations on gene transcription in prostate cancer. *Am J Pathol.,* December 2013, vol. 183 (6), 1960-70
- **HEINTZMAN ND ; HON GC ; HAWKINS RD ; KHERADPOUR P ; STARK A ; HARP LF et al.** Histone modifications at human enhancers reflect global cell-type-specific gene expression. *Nature,* 07 May 2009, vol. 459 (7243), 108-12
- **HEINZ S ; ROMANOSKI CE ; BENNER C ; GLASS CK.** The selection and function of cell type-specific enhancers. *Nat Rev Mol Cell Biol,* March 2015, vol. 16 (3), 144-54
- **MUNDBJERG K ; CHOPRA S ; ALEMOZAFFAR M ; DUYMICH C ; LAKSHMINARASIMHAN R ; NICHOLS PW et al.** Identifying aggressive prostate cancer foci using a DNA methylation classifier. *Genome Biol. BioMed Central,* 12 January 2017, vol. 18 (1), 3
- **TANG Y ; JIANG S ; GU Y ; LI W ; MO Z ; HUANG Y et al.** Promoter DNA methylation analysis reveals a combined diagnosis of CpG-based biomarker for prostate cancer. *Oncotarget. Impact Journals,* 29 August 2017, vol. 8 (35), 58199-209
- **FRAME FM ; PELLACANI D ; COLLINS AT ; MAITLAND NJ.** Harvesting Human Prostate Tissue Material and Culturing Primary Prostate Epithelial Cells. *Methods Mol Biol.,* 2016, vol. 1443, 181-20
- **XI Y ; LI W.** BSMAP: whole genome bisulfite sequence MAPping program. *BMC Bioinformatics,* 2009, vol. 10, 232
- **LI H ; HANDSAKER B ; WYSOKER A ; FENNELL T ; RUAN J ; HOMER N et al.** The Sequence Alignment/Map format and SAMtools. *Bioinformatics,* 15 August 2009, vol. 25 (16), 2078-9

- **WANG H-Q ; TUOMINEN LK ; TSAI C-J.** SLIM: a sliding linear model for estimating the proportion of true null hypotheses in datasets with dependence structures. *Bioinformatics,* 15 January 2011, vol. 27 (2), 225-31
- **QUINLAN AR ; HALL IM.** BEDTools: a flexible suite of utilities for comparing genomic features. *Bioinformatics,* 15 March 2010, vol. 26 (6), 841-2
- **THURMAN RE ; RYNES E ; HUMBERT R ; VIERSTRA J ; MAURANO MT, HAUGEN E et al.** The accessible chromatin landscape of the human genome. *Nature,* 29 August 2012, vol. 489 (7414), 75-82
- Epigenome-Wide Tumor DNA Methylation Profiling Identifies Novel Prognostic Biomarkers of Metastatic-Lethal Progression in Men Diagnosed with Clinically Localized Prostate Cancer. **ZHAO S ; GEYBELS MS ; LEONARDSON A ; RUBICZ R ; KOLB S ; YAN Q et al.** Clin Cancer Res. American Association for Cancer Research, 01 January 2017, vol. 23, 311-9
- **GEYBELS MS ; ZHAO S ; WONG C-J ; BIBIKOVA M ; KLOTZLE B ; WU M et al.** Epigenomic profiling of DNA methylation in paired prostate cancer versus adjacent benign tissue. *Prostate,* December 2015, vol. 75 (16), 1941-50
- **EYBELS MS ; WRIGHT JL ; BIBIKOVA M ; KLOTZLE B ; FAN J-B ; ZHAO S et al.** Epigenetic signature of Gleason score and prostate cancer recurrence after radical prostatectomy. Clin Epigenetics. *BioMed Central,* 2016, vol. 8 (1), 97